(19) Europäisches Patentamt
European Patent Office
Office européen des brevets

(11) EP 3 939 975 A1

(12) EUROPEAN PATENT APPLICATION
published in accordance with Art. 153(4) EPC

(43) Date of publication:
19.01.2022 Bulletin 2022/03

(21) Application number: 20773358.5

(22) Date of filing: 12.03.2020

(51) International Patent Classification (IPC):
*C07D 413/12* (2006.01) *C07D 413/14* (2006.01)
*C07D 417/12* (2006.01) *C07D 417/14* (2006.01)
*A01N 47/18* (2006.01) *A01P 3/00* (2006.01)
*A01N 43/78* (2006.01) *A01N 43/824* (2006.01)

(52) Cooperative Patent Classification (CPC):
A01N 43/78; A01N 47/18; C07D 413/12;
C07D 413/14; C07D 417/12; C07D 417/14

(86) International application number:
PCT/JP2020/010905

(87) International publication number:
WO 2020/189525 (24.09.2020 Gazette 2020/39)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 15.03.2019 JP 2019048196

(71) Applicant: Nippon Soda Co., Ltd.
Tokyo 100-8165 (JP)

(72) Inventors:
- KOUBORI Shinya
  Odawara-shi, Kanagawa 250-0280 (JP)
- ITO Syuichi
  Odawara-shi, Kanagawa 250-0280 (JP)
- TAMAI Tetsuo
  Joetsu-shi, Niigata 949-2392 (JP)
- KOYAMA Hiroaki
  Odawara-shi, Kanagawa 250-0280 (JP)
- KAWASAKI Tatsuhiro
  Odawara-shi, Kanagawa 250-0280 (JP)
- FUJII Takayuki
  Yama-gun, Fukushima 969-3302 (JP)
- NISHINO Shigeki
  Makinohara-shi, Shizuoka 421-0412 (JP)
- WATANABE Shinya
  Odawara-shi, Kanagawa 250-0280 (JP)
- KATO Hideki
  Odawara-shi, Kanagawa 250-0280 (JP)
- KUNISHIMA Mikiko
  Odawara-shi, Kanagawa 250-0280 (JP)
- UESUSUKI Shinya
  Odawara-shi, Kanagawa 250-0280 (JP)
- NOMURA Juri
  Odawara-shi, Kanagawa 250-0280 (JP)
- SANO Hiroshi
  Odawara-shi, Kanagawa 250-0280 (JP)
- SAIGA Tomoyuki
  Odawara-shi, Kanagawa 250-0280 (JP)

(74) Representative: Wibbelmann, Jobst
Wuesthoff & Wuesthoff
Patentanwälte PartG mbB
Schweigerstrasse 2
81541 München (DE)

# (54) HETEROCYCLIC COMPOUND AND AGRICULTURAL OR HORTICULTURAL BACTERICIDE

(57) A compound of formula (I) (in the formula (I), $R^1$ represents a substituted or unsubstituted C1-6 alkyl group or the like, $R^2$ represents a substituted or unsubstituted 5- or 6-membered heteroaryl group, $R^3$ represents a substituted or unsubstituted C1-6 alkyl group, a substituted or unsubstituted C1-6 alkoxycarbonyl group or the like, Y represents an oxygen atom or NH, $A^1$ represents a sulfur atom, an oxygen atom, or $NR^4$, $R^4$ represents a C1-6 alkyl group, $A^2$ is a carbon atom or a nitrogen atom, X represents a substituent, n represents the chemically acceptable number of X, and is an integer of 0 to 3, and, when n is 2 or more, X may be identical to or different from each other), or a salt thereof is provided.

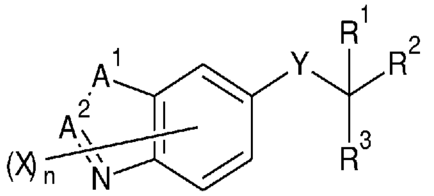

(I)

## Description

TECHNICAL FIELD

The present invention relates to a heterocyclic compound and an agricultural or horticultural fungicide. Furthermore, the present invention specifically relates to a heterocyclic compound that has excellent fungicidal activity and safety and that can be synthesized industrially advantageously, and to an agricultural or horticultural fungicide containing the same as an active ingredient thereof.

The present invention claims priority on the basis of Japanese Patent Application No. 2019-048196 filed in Japan on March 15, 2019, the contents of which are incorporated herein by reference.

BACKGROUND OF THE INVENTION

A large number of control agents have been proposed to control crop diseases in cultivation of agricultural or horticultural crops. However, most of the proposed control agents are not sufficiently satisfactory due to inadequate control efficacy, restriction on the use thereof owing to the appearance of drug-resistant pathogenic fungi, phytotoxicity or contamination to plants, strong toxicity in humans, animals, or fish, or detrimental effects on the environment. Accordingly, there is a strong demand for a control agent which has fewer defects and can be used safely.

Patent Document 1 discloses a compound of formula (A).

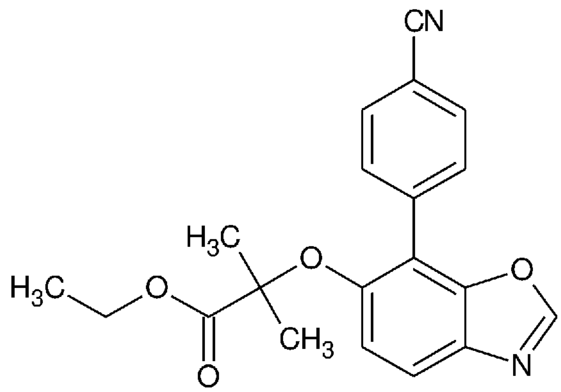

(A)

Patent Document 2 discloses a compound of formula (B).

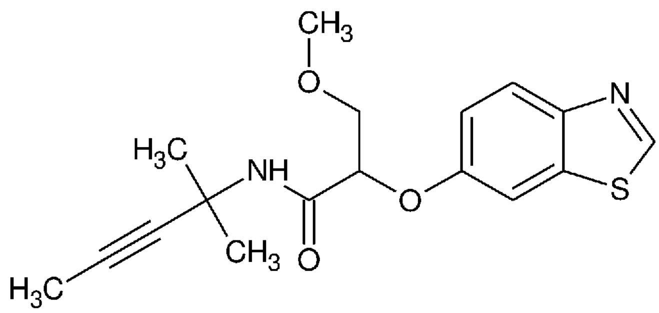

(B)

Non-patent Document 1 discloses a compound of formula (C).

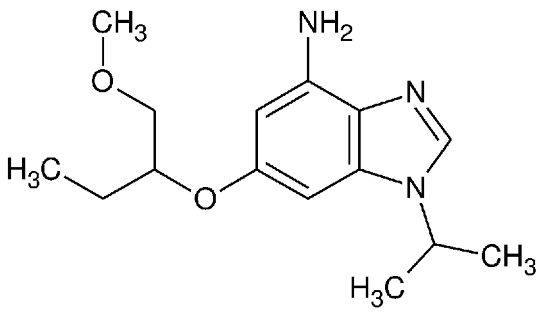

(C)

DOCUMENTS OF RELATED ART

Patent Documents

Patent Document 1: WO 2016/108282 A
Patent Document 2: WO 2004/108964 A

Non-patent Documents

Non-patent Document 1: Bioorganic & Medicinal Chemistry, Volume: 19, Issue: 1, Pages: 359-373.

SUMMARY OF THE INVENTION

PROBLEMS TO BE SOLVED BY THE INVENTION

The present invention aims to provide a heterocyclic compound that has excellent fungicidal activity and safety and that can be synthesized industrially advantageously, and an agricultural or horticultural fungicide containing the same as an active ingredient thereof.

MEANS TO SOLVE THE PROBLEMS

The present invention encompassing the following aspects has been completed as a result of studying to solve the above-described problems.
(1) A compound of formula (I) or a salt thereof.

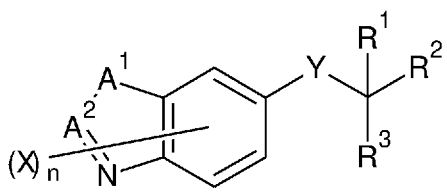

(I)

(In formula (I),

$R^1$ represents a hydrogen atom, a substituted or unsubstituted C1-6 alkyl group, or a halogeno group,
$R^2$ represents a substituted or unsubstituted 5- or 6-membered heteroaryl group,
$R^3$ represents a substituted or unsubstituted C1-6 alkyl group, a substituted or unsubstituted C1-6 alkoxycarbonyl group, a substituted or unsubstituted C1-6 alkylthio group, a substituted or unsubstituted C6-10 aryl group, a substituted or unsubstituted 5-or 6-membered heteroaryl group, a group of formula: "-C($R^i$)=NOR$^j$", or a group of formula: "-C(=O)NHR$^s$",
$R^1$ represents a hydrogen atom or a C1-6 alkyl group,

$R^j$ represents a substituted or unsubstituted C1-6 alkyl group,
$R^s$ represents a substituted or unsubstituted C1-6 alkyl group,
Y represents an oxygen atom or NH,
$A^1$ represents a sulfur atom, an oxygen atom, or $NR^4$,
$R^4$ represents a C1-6 alkyl group,
$A^2$ represents a carbon atom or a nitrogen atom,
X represents a substituent,
n represents the chemically acceptable number of X, and is an integer of 0 to 3, and
when n is 2 or more, X may be identical to or different from each other.)

(2) The compound or the salt thereof according to (1), wherein, in formula (I), X represents a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a substituted or unsubstituted C1-6 alkylthio group, a substituted or unsubstituted C3-8 cycloalkyl group, a substituted or unsubstituted C6-10 aryl group, a halogeno group, a cyano group, a group of formula: "$-NR^a R^b$" (wherein $R^a$ and $R^b$ each independently represents a hydrogen atom, a C1-6 alkyl group, or a C1-6 alkoxycarbonyl group), or a group of formula: "$-C(R^e)=NOR^f$" (wherein $R^e$ and $R^f$ each independently represents a hydrogen atom or a C1-6 alkyl group).
(3) The compound or the salt thereof according to (1), wherein formula (I) is formula (II).

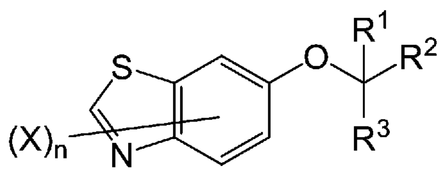
(II)

In formula (II), $R^1$, $R^2$, $R^3$, X and n mean the same as defined in formula (I).
(4) The compound or the salt thereof according to (1), wherein formula (I) is formula (III).

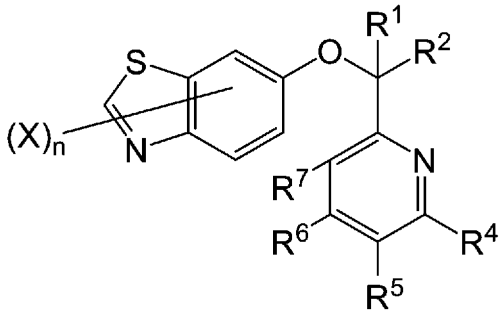
(III)

In formula (III),

$R^1$, $R^2$, X and n mean the same as defined in formula (I),
$R^4$, $R^5$, $R^6$ and $R^7$ each independently represents a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C6-10 aryl group, a substituted or unsubstituted 5- or 6-membered heterocyclyl group, a halogeno group, a cyano group, a nitro group, a group of formula "$-C(R^g)=NOR^h$" (wherein $R^g$ represents a hydrogen atom, a C1-6 alkyl group or an amino group, and $R^h$ represents a hydrogen atom, a substituted or unsubstituted C1-6 alkyl group, or a C2-6 alkynyl group), a group of formula: "$-O-N=CR^cR^d$" (wherein $R^c$ and $R^d$ each independently represents a hydrogen atom or a C1-6 alkyl group), or a group of formula: "$-C(=O)NR^pR^q$" (wherein $R^p$ and $R^q$ each independently represents a hydrogen atom or a C1-6 alkyl group).

(5) An agricultural or horticultural fungicide containing, as an active ingredient thereof, at least one selected from

compounds and salts thereof of (1) to (4).

EFFECTS OF THE INVENTION

The heterocyclic compound and the salt thereof according to the present invention have secured fungicidal activity and excellent safety and can be synthesized industrially advantageously. The agricultural or horticultural fungicide according to the present invention has excellent control effects, causes no phytotoxicity to plant bodies, and provides less toxicity in humans, animals, or fish, and fewer detrimental effects on the environment.

EMBODIMENTS FOR CARRYING OUT THE INVENTION

(Heterocyclic compound)

A heterocyclic compound according to the present invention is a compound of formula (I) (hereinafter, may be indicated as compound (I)), or a salt of compound (I).

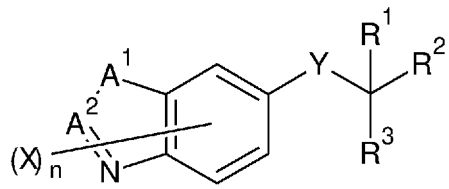
(I)

In formula (I),

$R^1$ represents a hydrogen atom, a substituted or unsubstituted C1-6 alkyl group, or a halogeno group,
$R^2$ represents a substituted or unsubstituted 5- or 6-membered heteroaryl group,
$R^3$ represents a substituted or unsubstituted C1-6 alkyl group, a substituted or unsubstituted C1-6 alkoxycarbonyl group, a substituted or unsubstituted C1-6 alkylthio group, a substituted or unsubstituted C6-10 aryl group, a substituted or unsubstituted 5-or 6-membered heteroaryl group, a group of formula: "-C($R^i$)=NO$R^j$", or a group of formula: "-C(=O)NH$R^s$",
$R^i$ represents a hydrogen atom or a C1-6 alkyl group,
$R^j$ represents a substituted or unsubstituted C1-6 alkyl group,
$R^s$ represents a substituted or unsubstituted C1-6 alkyl group,
Y represents an oxygen atom or NH,
$A^1$ represents a sulfur atom, an oxygen atom, or $NR^4$,
$R^4$ represents a C1-6 alkyl group,
$A^2$ represents a carbon atom or a nitrogen atom,
X represents a substituent,
n represents the chemically acceptable number of X, and is an integer of 0 to 3, and
when n is 2 or more, X may be identical to or different from each other.

In the present invention, the term "unsubstituted" refers to a group consisting of a mother nucleus. In the case where only the name of a group serving as a mother nucleus is provided without accompanying the term "substituted", this refers to "unsubstituted" unless specifically indicated otherwise.
On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.
The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.
There are no particular limitations on "substituents" provided that they are chemically available and achieve the effects of the present invention. Specific examples of groups that can be "substituents" include the following groups:

C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;

C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-8 cycloalkyl groups such as a cyclopropyl group, a cyclobutyl group, a cyclopentyl group, a cyclohexyl group, and a cubanyl group;
C6-10 aryl groups such as a phenyl group and a naphthyl group;
C6-10 aryl C1-6 alkyl groups such as a benzyl group and a phenethyl group;
3- to 6-membered heterocyclyl groups;
3- to 6-membered heterocyclyl C1-6 alkyl groups;
a hydroxyl group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
C6-10 aryloxy groups such as a phenoxy group, and a naphthoxy group;
C6-10 aryl C1-6 alkoxy groups such as a benzyloxy group, and a phenethyloxy group;
5- or 6-membered heteroaryloxy groups such as a thiazolyloxy group, and a pyridyloxy group;
5- or 6-membered heteroaryl C1-6 alkyloxy groups such as a thiazolylmethyloxy group, and a pyridylmethyloxy group;
C1-6 alkoxy C2-6 alkynyl groups such as a 3-methoxy-1-propynyl group, and a 3-ethoxy-1-propynyl group;
a formyl group;
C1-6 alkylcarbonyl groups such as an acetyl group, and a propionyl group;
a formyloxy group;
C1-6 alkylcarbonyloxy groups such as an acetyloxy group, and a propionyloxy group;
C6-10 arylcarbonyl groups such as a benzoyl group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
C1-6 alkoxycarbonyloxy groups such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxyl group;
halogeno groups such as a fluoro group, a chloro group, a bromo group, and an iodo group;
C1-6 haloalkyl groups such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a difluoromethoxy group, a trifluoromethoxy group, a 2-chloro-n-propoxy group, and a 2,3-dichlorobutoxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, a diethylamino group, and a t-butylamino group;
C6-10 arylamino groups such as an anilino group, and a naphthylamino group;
C6-10 aryl C1-6 alkylamino groups such as a benzylamino group, and a phenethylamino group;
a formylamino group;
C1-6 alkylcarbonylamino groups such as an acetylamino group, a diacetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, an i-propoxycarbonylamino group, and a t-butoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;

hydroxyimino C1-6 alkyl groups such as a hydroxyiminomethyl group, a 1-(hydroxyimino)ethyl group, and a 2-(hydroxyimino)ethyl group;
CI-6 alkoxyimino CI-6 alkyl groups such as a methoxyiminomethyl group, a 1-(methoxyimino)ethyl group, a 2-(methoxyimino)ethyl group, an ethoxyiminomethyl group, a 1-(ethoxyimino)ethyl group, a 2-(ethoxyimino)ethyl group, a n-propoxyiminomethyl group, a 1-(n-propoxyimino)ethyl group, a 2-(n-propoxyimino)ethyl group, an i-propoxyiminomethyl group, a 1-(i-propoxyimino)ethyl group, and a 2-(i-propoxyimino)ethyl group;
C1-6 haloalkoxyimino C1-6 alkyl groups such as a trifluoromethoxyiminomethyl group, a 1-(trifluoromethoxyimino)ethyl group, and a 2 -(trifluoromethoxyimino)ethyl group;
an aminocarbonyloxy group;
C1-6 alkyl-substituted aminocarbonyloxy groups such as an ethylaminocarbonyloxy group, and a dimethylaminocarbonyloxy group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
C6-10 arylthio groups such as a phenylthio group, and a naphthylthio group;
C6-10 aryl C1-6 alkylthio groups such as a benzylthio group;
5- or 6-membered heteroarylthio groups such as a thiazolylthio group, and a pyridylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
C6-10 arylsulfinyl groups such as a phenylsulfinyl group;
5- or 6-membered heteroarylsulfinyl groups such as a thiazolylsulfinyl group, and a pyridylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
C6-10 arylsulfonyl groups such as a phenylsulfonyl group;
5- or 6-membered heteroarylsulfonyl groups such as a thiazolylsulfonyl group, and a pyridylsulfonyl group;
C1-6 alkylsulfonyloxy groups such as a methylsulfonyl oxy group, an ethylsulfonyloxy group, and a t-butylsulfonyloxy group;
C1-6 haloalkylsulfonyloxy groups such as a trifluoromethylsulfonyl oxy group, and a 2,2,2-trifluoroethylsulfonyloxy group;
tri C1-6 alkyl-substituted silyl groups such as a trimethylsilyl group, a triethylsilyl group, and a t-butyldimethylsilyl group;
tri C6-10 aryl-substituted silyl groups such as a triphenylsilyl group;
a cyano group; a nitro group;
hydroxy C1-6 alkyl groups such as a hydroxymethyl group, and a 2-hydroxyethyl group;
tri C1-6 alkyl-substituted silyloxy C1-6 alkyl groups such as a tert-butyldimethylsilyloxymethyl group, and a 2-tert-butyldimethylsilyloxyethyl group;
C1-6 alkoxy C1-6 alkyl groups such as a methoxymethyl group, an ethoxymethyl group, and a dimethoxymethyl group;
C1-6 alkylcarbonyloxy C1-6 alkyl groups such as an acetyloxymethyl group;
C1-6 alkoxy C1-6 alkoxy C1-6 alkyl groups such as a (methoxymethoxy)methyl group, a (2-methoxyethoxy)methyl group, a (1-ethoxyethoxy)methyl group, a (1-isopropoxyethoxy)methyl group, and a ((2 -methoxypropan-2-yl)oxy)methyl group;
C1-6 alkoxy C1-6 alkoxy C1-6 alkoxy C1-6 alkyl groups such as a ((2-methoxyethoxy)methoxy)methyl group;
C1-6 alkoxycarbonyloxy C1-6 alkyl groups such as a ((methoxycarbonyl)oxy)methyl group;
C1-6 alkoxy C1-6 alkylcarbonyl C1-6 alkyl groups such as a (2-methoxyacetoxy)methyl group, and a ((3-methoxypropanoyl)oxy)methyl group;
CI-6 alkylthio C1-6 alkoxy C1-6 alkyl groups such as a ((methylthio)methoxy)methyl group;
C1-6 alkyl-substituted aminocarbonyloxy C1-6 alkyl groups such as a ((methylcarbamoyl)oxy)methyl group, and a ((dimethylcarbamoyl)oxy)methyl group;
cyano-substituted C1-6 alkyl groups such as a cyanomethyl group, a 2-cyanoethyl group, and a 3-cyanopropyl group;
5- or 6-membered saturated heterocyclyl group-substituted C1-6 alkyl groups such as a (1,3-dioxolan-2-yl)methyl group, a 2-(1,3-dioxolan-2-yl)ethyl group, a (1,3-dioxan-2-yl)methyl group, and a 2-(1,3-dioxan-2-yl)ethyl group;
C1-6 alkoxy C2-6 alkynyl groups such as a 3-methoxy-1-propynyl group; and
C1-6 alkoxy C1-6 alkoxy groups such as a methoxymethoxy group, and a 2-methoxyethoxy group.

Any hydrogen atom of the "substituent" may be substituted with a group having a different structure. Examples of such a substituent include C1-6 alkyl groups, C1-6 haloalkyl groups, C1-6 alkoxy groups, C1-6 haloalkoxy groups, a hydroxy group, C1-6 alkoxy groups, C1-6 alkylthio groups, C1-6 alkylsulfinyl groups, C1-6 alkylsulfonyl groups, tri C1-6

alkyl-substituted silyl groups, halogeno groups, a cyano group, and a nitro group.

The "3- to 6-membered heterocyclyl group" contains, as a ring member atom, 1 to 4 hetero atoms selected from the group consisting of a nitrogen atom, an oxygen atom and a sulfur atom. The heterocyclyl group may be monocyclic or polycyclic. If at least one ring of a polycyclic heterocyclyl group is a hetero ring, remaining rings thereof may be any of saturated alicyclic rings, unsaturated alicyclic rings and aromatic rings. Examples of the "3- to 6-membered heterocyclyl group" include 3- to 6-membered saturated heterocyclyl groups, 5- or 6-membered heteroaryl groups, and 5- or 6-membered partially unsaturated heterocyclyl groups.

Examples of the 3- to 6-membered saturated heterocyclyl groups include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, a dioxanyl group, and a dithiolanyl group.

Examples of the 5-membered heteroaryl groups include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

Examples of the 5- or 6-membered partially unsaturated heterocyclyl groups include a dihydropyranyl group, and a 4,5-dihydroisooxazolyl group.

($R^1$)

In formula (I), $R^1$ represents a hydrogen atom, a substituted or unsubstituted C1-6 alkyl group, or a halogeno group.

The "C1-6 alkyl group" as $R^1$ may be linear or branched. Examples of the alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Specific examples of the "substituted C1-6 alkyl group" include: C1-6 haloalkyl groups such as a fluoromethyl group, a chloromethyl group, a bromomethyl group, a difluoromethyl group, a dichloromethyl group, a dibromomethyl group, a trifluoromethyl group, a trichloromethyl group, a tribromomethyl group, a 1-chloroethyl group, a 2,2,2-trifluoroethyl group, a 2,2,2-trichloroethyl group, a pentafluoroethyl group, a 4-fluorobutyl group, a 2,2,3,3,4,4,4-heptafluorobutyl group, a 4-chlorobutyl group, a 3,3,3-trifluoropropyl group, a 2,2,3,3,3-pentafluoropropyl group, a 1,1,1,3,3,3-hexafluoropropan-2-yl group, a perfluoropropan-2-yl group, a perfluorohexyl group, a perchlorohexyl group, and a 2,4,6-trichlorohexyl group;

hydroxy C1-6 alkyl groups such as a hydroxymethyl group, and a hydroxy ethyl group;

C1-6 alkoxy C1-6 alkyl groups such as a methoxymethyl group, an ethoxymethyl group, a methoxyethyl group, an ethoxyethyl group, a methoxy-n-propyl group, a n-propoxymethyl group, an i-propoxyethyl group, a s-butoxymethyl group, and a t-butoxyethyl group;

C6-10 aryl C1-6 alkyl groups such as a benzyl group, and a phenethyl group; and

C3-8 cycloalkyl C1-6 alkyl groups such as a cyclopropylmethyl group, a 2-cyclopropylethyl group, a cyclopentylmethyl group, a 2-cyclohexylethyl group, and 2-cyclooctylethyl group.

Preferable examples of a substituent on the "C1-6 alkyl group" as $R^1$ include: halogeno groups such as a fluoro group, a chloro group, a bromo group, and an iodo group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; and a cyano group.

Examples of the "halogeno group" as $R^i$ include a fluoro group, a chloro group, a bromo group, and an iodo group.

$R^1$ preferably represents a hydrogen atom.

($R^2$)

In formula (I), $R^2$ represents a substituted or unsubstituted 5- or 6-membered heteroaryl group.

Examples of the "5- or 6-membered heteroaryl group" as $R^2$ include: 5-membered heteroaryl groups, such as a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, and a tetrazolyl group; and 6-membered heteroaryl groups such as a pyridyl group, a pyrazinyl group, a pyrimidinyl group, a pyridazinyl group, and a triazinyl group.

Preferable examples of a substituent on the "5- or 6-membered heteroaryl group" as $R^2$ include: C1-6 alkyl groups such as a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group; and halogeno groups such as a fluoro group, a chloro group, a bromo group, and an iodo group.

$R^2$ is preferably a 5-membered heteroaryl group, more preferably an oxadiazolyl group, and even more preferably a 1,3, 4 -oxadizaol-2-yl group.

($R^3$)

In formula (I), $R^3$ represents a substituted or unsubstituted C1-6 alkyl group, a substituted or unsubstituted C1-6 alkoxycarbonyl group, a substituted or unsubstituted C1-6 alkylthio group, a substituted or unsubstituted C6-10 aryl group, a substituted or unsubstituted 5- or 6-membered heteroaryl group, a group of formula: "-C($R^i$)=NOR$^j$" , or a group of formula: "-C(=O)NHR$^s$".

Specific examples of the "C1-6 alkyl group" as $R^3$ include the same groups as those mentioned as $R^1$.

Specific examples of the "5- or 6-membered heteroaryl group" as $R^3$ include the same groups as those mentioned as $R^2$. Among these, a pyridyl group, a pyrimidinyl group, a thiazolyl group, and a pyrazinyl group are preferable.

Examples of the "C1-6 alkoxycarbonyl group" as $R^3$ include a methoxycarbonyl group, an ethoxycarbonyl group, a n-propoxycarbonyl group, an i-propoxycarbonyl group, a n-butoxycarbonyl group, and a t-butoxycarbonyl group.

Examples of the "C1-6 alkylthio group" as $R^3$ include a methylthio group, an ethylthio group, a n-propylthio group, a n-butylthio group, a n-pentylthio group, a n-hexylthio group, and an i-propylthio group.

Preferable examples of a substituent on the "C1-6 alkyl group", "C1-6 alkoxycarbonyl group", or "C1-6 alkylthio group" as $R^3$ include halogeno groups such as a fluoro group, a chloro group, a bromo group, and an iodo group.

The "C6-10 aryl group" as $R^3$ is a group formed by removing one hydrogen atom on a monocyclic or polycyclic aromatic hydrocarbon ring. Examples of the "C6-10 aryl group" include a phenyl group and a naphthyl group, and a phenyl group is preferable.

Examples of a substituent on the "C6-10 aryl group" or "5- or 6-membered heteroaryl group" as $R^3$ (hereinafter, may be indicated as $G^1$) include substituted or unsubstituted C1-6 alkyl groups, substituted or unsubstituted C2-6 alkenyl groups, substituted or unsubstituted C2-6 alkynyl groups, substituted or unsubstituted C1-6 alkoxy groups, substituted or unsubstituted C1-6 alkoxycarbonyl groups, substituted or unsubstituted C1-6 alkylthio groups, substituted or unsubstituted C1-6 alkylsulfinyl groups, substituted or unsubstituted C1-6 alkylsulfonyl groups, substituted or unsubstituted C6-10 aryl groups, substituted or unsubstituted 5- or 6-membered heterocyclyl groups, halogeno groups, a cyano group, a nitro group, groups of formula "-C($R^g$)=NOR$^h$", groups of formula: "-O-N=CR$^c$R$^d$", and groups of formula: "-C(=O)NR$^p$R$^q$".

Specific examples of the "C1-6 alkyl group" and "halogeno group" as $G^1$ include the same groups as those mentioned as $R^1$.

Examples of the "substituted C1-6 alkyl group" as $G^1$ include: hydroxy C1-6 alkyl groups, such as a hydroxymethyl group, and a 2-hydroxyethyl group; tri C1-6 alkyl-substituted silyloxy C1-6 alkyl groups, such as a tert-butyl dimethylsilyloxymethyl group, and a 2-tert-butyl dimethylsilyloxyethyl group; C1-6 alkoxy C1-6 alkyl groups, such as a methoxymethyl group, an ethoxymethyl group, and a dimethoxymethyl group; C1-6 alkylthio C1-6 alkyl groups such as a methylthiomethyl group; C1-6 alkylcarbonyloxy C1-6 alkyl groups such as an acetyloxymethyl group; C1-6 alkoxy C1-6 alkoxy C1-6 alkyl groups such as a (methoxymethoxy)methyl group, a (2-methoxyethoxy)methyl group, a (1-ethoxyethoxy)methyl group, a (1-isopropoxyethoxy)methyl group, and a ((2-methoxypropan-2-yl)oxy)methyl group; C1-6 alkoxy C1-6 alkoxy C1-6 alkoxy C1-6 alkyl groups such as a ((2-methoxyethoxy)methoxy)methyl group; C1-6 alkoxycarbonyloxy C1-6 alkyl groups such as a ((methoxycarbonyl)oxy)methyl group; C1-6 alkoxy C1-6 alkylcarbonyloxy C1-6 alkyl groups such as a (2-methoxyacetoxy)methyl group, and a ((3-methoxypropanoyl)oxy)methyl group; C1-6 alkylthio C1-6 alkoxy C1-6 alkyl groups such as a ((methylthio)methoxy)methyl group; C1-6 alkyl-substituted aminocarbonyloxy C1-6 alkyl groups such as a ((methylcarbamoyl)oxy)methyl group, and a ((dimethylcarbamoyl)oxy)methyl group; cyano-substituted C1-6 alkyl groups such as a cyanomethyl group, a 2-cyanoethyl group, and a 3-cyanopropyl group; C6-10 aryl C1-6 alkyl groups such as a benzyl group, and a phenethyl group; and 5- or 6-membered saturated heterocyclyl group-substituted C1-6 alkyl groups, such as a (1,3-dioxolan-2-yl)methyl group, a 2-(1,3-dioxolan-2-yl)ethyl group, a (1,3-dioxan-2-yl)methyl group, and a 2-(1,3-dioxan-2-yl)ethyl group.

Preferable examples of a substituent on the "C1-6 alkyl group" as $G^1$ include a hydroxy group; tri C1-6 alkyl-substituted silyloxy groups such as a tert-butyl dimethylsilyloxy group, and a 2-tert-butyl dimethylsilyloxy group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 alkylthio groups such as a methylthio group, an ethylthio group, a n-propylthio group, an i-propylthio group, a n-butylthio group, an i-butylthio group, a s-butylthio group, and a t-butylthio group; C1-6 alkylcarbonyloxy groups such as an acetyloxy group, and a propionyloxy group; C1-6 alkoxy C1-6

alkoxy groups such as a methoxymethoxy group, a 1-methoxyethoxy group, a 2-methoxyethoxy group, a 1-ethoxyethoxy group, a 2-ethoxyethoxy group, a 1-isopropoxyethoxy group, a 2-isopropoxyethoxy group, and a (2-methoxypropan-2-yl)oxy group; C1-6 alkoxy C1-6 alkoxy C1-6 alkoxy groups such as a (2-methoxyethoxy)methoxy group; C1-6 alkoxycarbonyloxy groups such as a methoxycarbonyloxy group, and an ethoxycarbonyloxy group; C1-6 alkoxy C1-6 alkylcarbonyloxy groups such as a 2-methoxyacetoxy group, and a (3-methoxypropanoyl)oxy group; C1-6 alkylthio C1-6 alkoxy groups such as a methylthiomethoxy group; C1-6 alkyl-substituted aminocarbonyloxy groups such as a (methylcarbamoyl)oxy group, and a (dimethylcarbamoyl)oxy group; a cyano group; C6-10 aryl groups such as a phenyl group and a naphthyl group; 5- or 6-membered saturated heterocyclyl groups such as a pyrrolidinyl group, a tetrahydrofuranyl group, a thiazolidinyl group, a piperidyl group, a piperazinyl group, a morpholinyl group, a dioxolanyl group, a dioxanyl group, and a dithiolanyl group.

Examples of the "C2-6 alkenyl group" as $G^1$ include a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group.

Examples of the "C2-6 alkynyl group" as $G^1$ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexenyl group, and a 1,1-dimethyl-2-butynyl group.

Examples of the "C1-6 alkoxy group" as $G^1$ include a methoxy group, an ethoxy group, a n-propoxy group, an i-propoxy group, a n-butoxy group, a s-butoxy group, an i-butoxy group, and a t-butoxy group.

Specific examples of the "C1-6 alkoxycarbonyl group" and "C1-6 alkylthio group" as $G^1$ include the same groups as those mentioned as $R^3$.

Examples of the "C1-6 alkylsulfinyl group" as $G^1$ include a methylsulfinyl group, an ethylsulfinyl group, and a t-butylsulfinyl group.

Examples of the "C1-6 alkylsulfonyl group" as $G^1$ include a methyl sulfonyl group, an ethylsulfonyl group, and a t-butylsulfonyl group.

Examples of a substituent on the "C2-6 alkenyl group", "C2-6 alkynyl group", "C1-6 alkoxy group", "C1-6 alkoxycarbonyl group", "C1-6 alkylthio group", "C1-6 alkylsulfinyl group" or "C1-6 alkylsulfonyl group" as $G^1$ include C1-6 alkoxy groups such as a methoxy group, an ethoxy group, a n-propoxy group, an i-propoxy group, a n-butoxy group, a s-butoxy group, an i-butoxy group, and a t-butoxy group.

Specific examples of the "C6-10 aryl group" as $G^1$ include the same groups as those mentioned as $R^3$.

Examples of the "5- or 6-membered heterocyclyl group" as $G^1$ include: 5- or 6-membered saturated heterocyclyl groups, such as a pyrrolidinyl group, a tetrahydrofuranyl group, a thiazolidinyl group, a piperidyl group, a piperazinyl group, a morpholinyl group, a dioxolanyl group, a dioxanyl group, and a dithiolanyl group; 5- or 6-membered partially unsaturated heterocyclyl groups, such as a dihydropyranyl group, and a 4,5-dihydroisooxazolyl group; 5-membered heteroaryl groups, such as a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, and a tetrazolyl group; and 6-membered heteroaryl groups such as a pyridyl group, a pyrazinyl group, a pyrimidinyl group, a pyridazinyl group, and a triazinyl group. Among these, a dioxolanyl group, a dioxanyl group, a dithiolanyl group, a furyl group, a thienyl group, a pyrazolyl group, an isooxazolyl group, a pyridyl group, a pyrimidinyl group, and a dihydropyranyl group are preferable.

Examples of a substituent on the "C6-10 aryl group" or the "5- or 6-membered heterocyclyl group" as $G^1$ include: halogeno groups, such as a fluoro group, a chloro group, a bromo group, and an iodo group; C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group; a hydroxy group; and C1-6 alkoxy groups such as a methoxy group, an ethoxy group, a n-propoxy group, an i-propoxy group, a n-butoxy group, a s-butoxy group, an i-butoxy group, and a t-butoxy group.

In the formula "$-C(R^g)=NOR^h$" as $G^1$, $R^g$ represents a hydrogen atom, a C1-6 alkyl group or an amino group, and preferably represents a hydrogen atom.

Specific examples of the "C1-6 alkyl group" as $R^g$ include the same groups as mentioned as $R^1$.

In the formula "$-C(R^g)=NOR^h$" as $G^1$, $R^h$ represents a hydrogen atom, a substituted or unsubstituted C1-6 alkyl group, or a C2-6 alkynyl group.

Specific examples of the "C1-6 alkyl group" as $R^h$ include the same groups as mentioned as $R^1$.

Examples of the "substituted C1-6 alkyl group" as $R^h$ include: C1-6 haloalkyl groups, such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl

group, a 4-chlorobutyl group, a perchlorohexyl group, and a 2,4,6-trichlorohexyl group; C1-6 alkoxy C1-6 alkyl groups, such as a methoxymethyl group, an ethoxymethyl group, a 2-methoxyethyl group, and 2-ethoxyethyl group; C3-8 cycloalkyl C1-6 alkyl groups, such as a cyclopropylmethyl group; C6-10 aryl C1-6 alkyl groups such as a benzyl group, and a phenethyl group; and 5- or 6-membered heteroaryl C1-6 alkyl groups such as a (1,3,4-oxadizaol-2-yl)methyl group, and a (pyridin-2-yl)methyl group. Among these, C1-6 alkoxy C1-6 alkyl groups, C3-8 cycloalkyl C1-6 alkyl groups, and 5- or 6-membered heteroaryl C1-6 alkyl groups are preferable.

Specific examples of the "C2-6 alkynyl group" as $R^h$ include the same groups as those mentioned as $G^1$.

In the formula: "-O-N=$CR^cR^d$" as $G^1$, $R^c$ and $R^d$ each independently represents a hydrogen atom or a C1-6 alkyl group.

Specific examples of the "C1-6 alkyl group" as $R^c$ and $R^d$ include the same groups as those defined as $R^1$.

In the formula: "-C(=O)$NR^pR^q$" as $G^1$, $R^p$ and $R^q$ each independently represents a hydrogen atom or a C1-6 alkyl group.

Specific examples of the "C1-6 alkyl group" as $R^p$ and $R^q$ include the same groups as those defined as $R^1$.

$G^1$ preferably represents a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C6-10 aryl group, a substituted or unsubstituted 5- or 6-membered heterocyclyl group, a halogeno group, a cyano group, a group of formula "-C($R^g$)=$NOR^h$", or a group of formula: "-O-N=$CR^cR^d$", and more preferably a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylthio group, a substituted or unsubstituted C1-6 alkylsulfinyl group, a substituted or unsubstituted C6-10 aryl group, a substituted or unsubstituted 5- or 6-membered heterocyclyl group, a halogeno group, a cyano group, a group of formula "-C($R^g$)=$NOR^h$", or a group of formula: "-O-N=$CR^cR^d$".

In the formula: "-C($R^i$)=$NOR^j$", $R^i$ represents a hydrogen atom, or a C1-6 alkyl group, and preferably represents a hydrogen atom.

In the formula: "-C($R^i$ )=$NOR^j$" , $R^j$ represents a substituted or unsubstituted C1-6 alkyl group.

Specific examples of the "C1-6 alkyl group" as $R^1$ and $R^j$ include the same groups as those mentioned as $R^1$.

Examples of the "substituted C1-6 alkyl group" as $R^j$ include: C6-10 aryl C1-6 alkyl groups such as a benzyl group, and a phenethyl group; and C1-6 alkoxy C1-6 alkyl groups such as a methoxymethyl group, an ethoxymethyl group, a 2-methoxyethyl group, and a 2-ethoxyethyl group, and preferably represents a benzyl group.

In the formula: "-C(=O)$NHR^s$", $R^s$ represents a substituted or unsubstituted C1-6 alkyl group.

Specific examples of the "C1-6 alkyl group" as $R^s$ include the same groups as those mentioned as $R^1$.

Examples of the "substituted C1-6 alkyl group" as $R^s$ include C1-6 alkoxyimino group-substituted C1-6 alkyl groups such as a methoxyiminomethyl group, an ethoxyiminomethyl group, a 2-methoxyiminoethyl group, a 2-ethoxyiminoethyl group, a 1-(methoxyimino)propan-2-yl group, a 1-(ethoxyimino)propan-2-yl group, a 1-(methoxyimino)-2-methylpropan-2-yl group, and a 1-(ethoxyimino)-2-methylpropan-2-yl group.

$R^3$ preferably represents a C1-6 alkyl group, a substituted or unsubstituted C6-10 aryl group, or a substituted or unsubstituted 5- or 6-membered heteroaryl group, more preferably a substituted or unsubstituted C6-10 aryl group, or a substituted or unsubstituted 5- or 6-membered heteroaryl group, even more preferably a substituted or unsubstituted phenyl group, or a substituted or unsubstituted 5- or 6-membered heteroaryl group, and even more preferably a substituted or unsubstituted 6-membered heteroaryl group.

(Y) In formula (I), Y represents an oxygen atom or NH.

Y preferably represents an oxygen atom.

(X)

In formula (I), X represents a substituent. Examples of the substituent X include the same substituents as mentioned above.

Preferable examples of the substituent X (hereinafter, may be indicated as $G^2$) include substituted or unsubstituted C1-6 alkyl groups, substituted or unsubstituted C2-6 alkenyl groups, substituted or unsubstituted C2-6 alkynyl groups, a hydroxy group, substituted or unsubstituted C1-6 alkoxy groups, substituted or unsubstituted C1-6 alkoxycarbonyl groups, substituted or unsubstituted C1-6 alkylthio groups, substituted or unsubstituted C3-8 cycloalkyl groups, substituted or unsubstituted C6-10 aryl groups, halogeno groups, a cyano group, groups of formula: "-$NR^aR^b$" (wherein $R^a$ and $R^b$ each independently represents a hydrogen atom, a C1-6 alkyl group, or a C1-6 alkoxycarbonyl group), and groups of formula: "-C($R^e$)=$NOR^f$" (wherein $R^e$ and $R^f$ each independently represents a hydrogen atom or a C1-6 alkyl group).

Specific examples of the "C1-6 alkyl group" and "halogeno group" as $G^2$ include the same groups as those mentioned as $R^1$.

Examples of the "substituted C1-6 alkyl group" as $G^2$ include: C1-6 haloalkyl groups such as a fluoromethyl

group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group; hydroxy C1-6 alkyl groups such as a hydroxymethyl group, and a 2-hydroxyethyl group; C1-6 alkoxy C1-6 alkyl groups such as a methoxymethyl group, an ethoxymethyl group, a 2-methoxyethyl group, and a 2-ethoxyethyl group.

Preferable examples of the substituent on the "C1-6 alkyl group" as $G^2$ include: halogeno groups such as a fluoro group, a chloro group, a bromo group, and an iodo group; a hydroxy group; and C1-6 alkoxy groups such as a methoxy group, an ethoxy group, a n-propoxy group, an i-propoxy group, a n-butoxy group, a s-butoxy group, an i-butoxy group, and a t-butoxy group.

Examples of the "C3-8 cycloalkyl group" as $G^2$ include a cyclopropyl group, a cyclobutyl group, a cyclopentyl group, a cyclohexyl group, and a cubanyl group, and a cyclopropyl group is preferable.

Specific examples of the "C1-6 alkoxycarbonyl group" and the "C1-6 alkylthio group" as $G^2$ include the same groups as those mentioned as $R^3$.

Examples of the "C2-6 alkenyl group" as $G^2$ include a vinyl group, a 1-propenyl group, a 2-propenyl group (an allyl group), a 1-butenyl group, a 2-butenyl group, a 3-butenyl group, a 1-methyl-2-propenyl group, a 2-methyl-1-propenyl group and a 2-methyl-2-propenyl group.

Specific examples of the "C2-6 alkynyl group" and the "C1-6 alkoxy group" as $G^2$ include the same groups as those mentioned as $G^1$.

Preferable examples of a substituent on the "C2-6 alkenyl group", "C2-6 alkynyl group", "C1-6 alkoxy group", "C1-6 alkoxycarbonyl group", "C1-6 alkylthio group", or "C3-8 cycloalkyl group" as $G^2$ include halogeno groups such as a fluoro group, a chloro group, a bromo group, and an iodo group.

Specific examples of the "C6-10 aryl group" as $G^2$ include the same groups as those mentioned as $R^3$, and a phenyl group is preferable.

Preferable examples of a substituent on the "C6-10 aryl group" as $G^2$ include: halogeno groups such as a fluoro group, a chloro group, a bromo group, and an iodo group; C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group; C1-6 haloalkyl groups such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a difluoromethoxy group, a trifluoromethoxy group, a 2-chloro-n-propoxy group, and a 2,3-dichlorobutoxy group; C1-6 alkylthio groups such as a methylthio group, an ethylthio group, a n-propylthio group, a n-butylthio group, a n-pentylthio group, a n-hexylthio group, and an i-propylthio group; C1-6 alkylsulfinyl groups such as a methylsulfinyl group, an ethylsulfinyl group, and a t-butylsulfinyl group; C1-6 alkylsulfonyl groups such as a methylsulfonyl group, an ethylsulfonyl group, and a t-butylsulfonyl group; and a cyano group, and more preferable examples thereof include C1-6 haloalkyl groups, C1-6 alkoxy groups, and C1-6 haloalkoxy groups.

In the formula: "$-NR^a R^b$", $R^a$ and $R^b$ each independently represents a hydrogen atom, a C1-6 alkyl group, or a C1-6 alkoxycarbonyl group.

Specific examples of the "C1-6 alkyl group" as $R^a$ and $R^b$ include the same groups as those mentioned as $R^1$.

Specific examples of the "C1-6 alkoxycarbonyl group" as $R^a$ and $R^b$ include the same groups as those mentioned as $R^3$.

$R^a$ preferably represents a hydrogen atom, and $R^b$ preferably represents a hydrogen atom or a C1-6 alkyl group.

In the formula: "$-C(R^e)=NOR^f$", $R^e$ and $R^f$ each independently represents a hydrogen atom or a C1-6 alkyl group.

Specific examples of the "C1-6 alkyl group" as $R^e$ and $R^f$ include the same groups as those mentioned as $R^1$.

$G^2$ preferably represents a substituted or unsubstituted C1-6 alkyl group, a C3-8 cycloalkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a substituted or unsubstituted C1-6 alkylthio group, a substituted or unsubstituted C6-10 aryl group, a halogeno group, a cyano group, or, a group of formula: "$-NR^aR^b$", and more preferably a substituted or unsubstituted C1-6 alkyl group, a C3-8 cycloalkyl group, a C2-6 alkenyl group, a C2-6 alkynyl group, a C1-6 alkoxy group, a C1-6 alkoxycarbonyl group, a substituted or unsubstituted C1-6 alkylthio group, a substituted or unsubstituted C6-10 aryl group, a halogeno group, a cyano group, or, a

group of formula: "-$NR^aR^b$".

(n)

In formula (I), n represents the chemically acceptable number of X, and is an integer of 0 to 3, more preferably an integer of 0 to 2, and even more preferably 0 or 1. When n is 2 or more, X may be identical to or different from each other.

(A1,A2)

In formula (I), $A^1$ represents a sulfur atom, an oxygen atom, or $NR^4$, and $R^4$ represents a C1-6 alkyl group.
In formula (I), $A^2$ is a carbon atom or a nitrogen atom.
Specific examples of the "C1-6 alkyl group" as $R^4$ include the same groups as those mentioned as $R^1$.
The compound of formula (I) is preferably any compound of formulae (1-1) to (I-4).

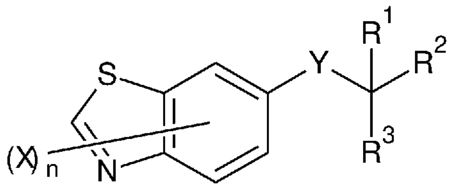

(I-1)

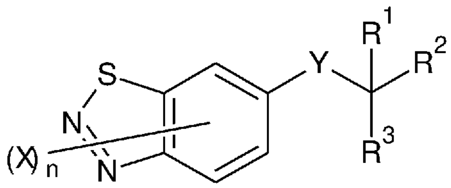

(I-2)

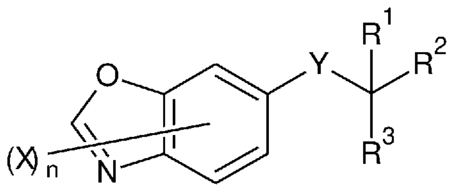

(I-3)

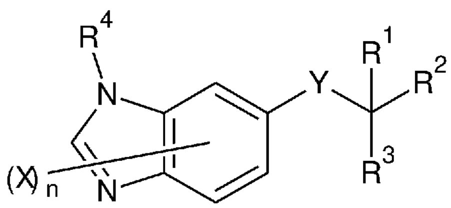

(I-4)

In formulae (I-1) to (I-4), $R^1$, $R^2$, $R^3$ , Y, X and n represent the same as defined in formula (I), and, in formula (1-4), $R^4$ represents the same as mentioned above.
The compound of formula (I) preferably represents the compound of formula (I-1).

(Compound of formula (II))

The compound of formula (I) is preferably a compound of formula (II).

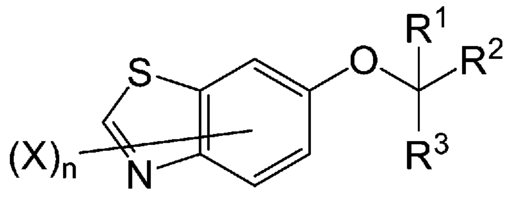

(II)

In formula (II), $R^1$, $R^2$, $R^3$, X and n represent the same as defined in formula (I).

The compound of formula (I) is preferably a compound of formula (III).

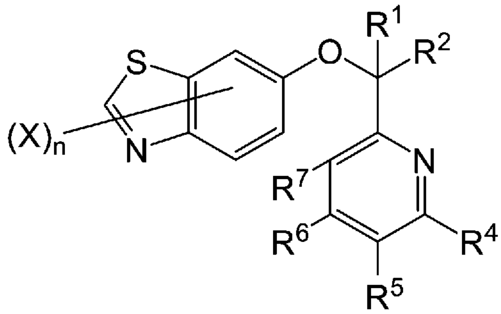

(III)

In formula (III),

$R^1$, $R^2$, X and n represent the same as defined in formula (I),
$R^4$, $R^5$, $R^6$ and $R^7$ each independently represents a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C6-10 aryl group, a substituted or unsubstituted 5- or 6-membered heterocyclyl group, a halogeno group, a cyano group, a nitro group, a group of formula "-C($R^g$)=NO$R^h$" (wherein $R^g$ represents a hydrogen atom, a C1-6 alkyl group or an amino group, and $R^h$ represents a hydrogen atom, a substituted or unsubstituted C1-6 alkyl group, or a C2-6 alkynyl group), a group of formula: "-O-N=C$R^cR^d$" (wherein, $R^c$ and $R^d$ each independently represents a hydrogen atom or a C1-6 alkyl group), or a group of formula: "-C(=O)N$R^pR^q$" (wherein $R^p$ and $R^q$ each independently represents a hydrogen atom or a C1-6 alkyl group).

Specific examples of the "substituted or unsubstituted C1-6 alkyl group", the "substituted or unsubstituted C2-6 alkenyl group", the "substituted or unsubstituted C2-6 alkynyl group", the "substituted or unsubstituted C1-6 alkoxy group", the "substituted or unsubstituted C1-6 alkoxycarbonyl group", the "substituted or unsubstituted C1-6 alkylthio group", the "substituted or unsubstituted C1-6 alkylsulfinyl group", the "substituted or unsubstituted C1-6 alkylsulfonyl group", the "substituted or unsubstituted C6-10 aryl group", the "substituted or unsubstituted 5- or 6-membered heterocyclyl group", the "halogeno group", the group of formula: "-C($R^g$)=NO$R^h$" (wherein $R^g$ represents a hydrogen atom, a C1-6 alkyl group or an amino group, and $R^h$ represents a hydrogen atom, a substituted or unsubstituted C1-6 alkyl group, or a C2-6 alkynyl group), a group of formula: "-O-N=C$R^cR^d$" (wherein, $R^c$ and $R^d$ each independently represents a hydrogen atom or a C1-6 alkyl group), and the group of formula: "-C(=O)N$R^pR^q$" (wherein $R^p$ and $R^q$ each independently represents a hydrogen atom or a C1-6 alkyl group) as $R^4$, $R^5$, $R^6$ and $R^7$ include the same groups as those mentioned as $G^1$.

It is preferable that $R^4$, $R^5$, $R^6$ and $R^7$ each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C6-10 aryl group, a substituted or unsubstituted 5- or 6-membered heterocyclyl group, a halogeno group, a cyano group, a group of formula: "-C($R^g$)=NO$R^h$", or a group of formula: "-O-N=C$R^cR^d$".

$R^4$ and $R^7$ more preferably represent a hydrogen atom, a substituted or unsubstituted C1-6 alkyl group, or a group of formula: "-C($R^g$)=NO$R^h$", and even more preferably a hydrogen atom.

$R^5$ and $R^6$ more preferably represent a hydrogen atom, a substituted or unsubstituted C1-6 alkyl group, a

substituted or unsubstituted C2-6 alkenyl group, a substituted or unsubstituted C2-6 alkynyl group, a substituted or unsubstituted C1-6 alkoxy group, a substituted or unsubstituted C1-6 alkylthio group, a substituted or unsubstituted C1-6 alkylsulfinyl group, a substituted or unsubstituted C6-10 aryl group, a substituted or unsubstituted 5- or 6-membered heterocyclyl group, a halogeno group, a cyano group, a group of formula: "-C($R^g$)=NOR$^h$", or a group of formula: "-O-N=CR$^c$R$^d$".

Salts of the compound (I) are not particularly limited in the case of agriculturally or horticulturally acceptable salts. Examples thereof include salts of inorganic acids such as a hydrochloric acid or a sulfuric acid; salts of organic acids such as an acetic acid or a lactic acid; salts of alkali metals such as lithium, sodium or potassium; salts of alkaline earth metals such as calcium or magnesium; salts of transition metals such as iron or copper; and salts of organic bases such as ammonia, triethylamine, tributylamine, pyridine, or hydrazine.

The compound (I) or the salts of the compound (I) are not particularly limited by the preparation process thereof. Salts of the compound (I) may be obtained from the compound (I) by known methods. For example, the compound (I) or the salt of the compound (I) according to the present invention may be obtained by the process described in Examples, or the like.

For example, the compound according to the present invention may be produced by the following methods.

(Synthesis method 1)

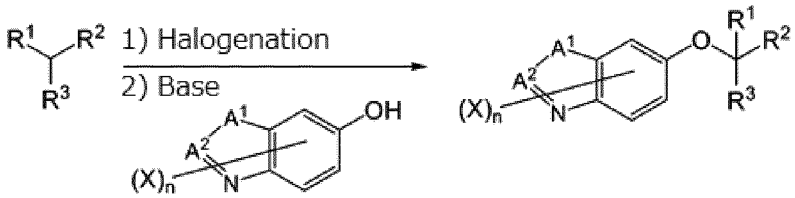


(In the scheme, $R^1$, $R^2$, $R^3$, $A^1$, $A^2$, X and n represent the same as defined above.)

(Synthesis method 2)

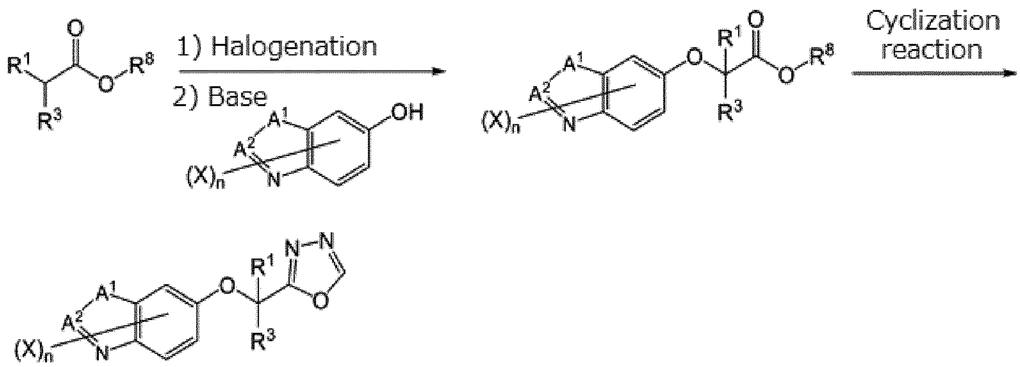


(In the scheme, $R^1$, $R^3$, $A^1$, $A^2$, X and n represent the same as defined above, and $R^8$ represents a C1-6 alkyl group such as a methyl group or an ethyl group.)

(Agricultural or horticultural fungicide)

An agricultural or horticultural fungicide according to the present invention contains, as an active ingredient thereof, at least one selected from the group consisting of compounds (I) and salts thereof. The compound (I) or the salt thereof contained in the agricultural or horticultural fungicide according to the present invention is not particularly limited provided that fungicidal effects are exhibited.

The agricultural or horticultural fungicide according to the present invention can be used to control plant diseases caused by a wide variety of filamentous fungi, such as fungi belonging to algae fungi (Oomycetes), sac fungi (Ascomycetes), imperfect fungi (Deuteromycetes), Basidiomycete fungi (Basidiomycetes) or zygomycete fungi (Zygomycetes).

Examples of plant diseases (pathogens) to be controlled include the following.

Sugar beet: brown spot disease (Cercospora beticola), black root disease (Aphanomyces cochllioides), root rot disease (Thanatephorus cucumeris), leaf rot disease (Thanatephorus cucumeris), rust disease (Uromyces betae), powdery mildew (Oidium sp.), spot blotch disease (Ramularia beticola), damping-off disease (Aphanomyces cochlioides, or Pythium ultimum), and the like.

Peanut: brown spot disease (Mycosphaerella arachidis), leaf mold (Ascochyta sp.), rust disease (Puccinia arachidis), damping-off disease (Pythium debaryanum), rust spot disease (Alternaria alternata), stem rot disease (Sclerotium rolfsii), black rust disease (Mycosphaerella berkeleyi), and the like.

Cucumber: powdery mildew (Sphaerotheca fuliginea), downy mildew (Pseudoperonospora cubensis), gummy stem blight (Mycosphaerella melonis), wilt disease (Fusarium oxysporum), sclerotinia rot (Sclerotinia sclerotiorum), gray mold (Botrytis cinerea), anthracnose (Colletotrichum orbiculare), scab (Cladosporium cucumerinum), brown spot disease (Corynespora cassicola), damping-off disease (Pythium debaryanam, Rhizoctonia solani Kuhn), Phomopsis root rot disease (Phomopsis sp.), Bacterial spot (Pseudomonas syringae pv.Lecrymans), and the like.

Tomato: gray mold disease (Botrytis cinerea), leaf mold disease (Cladosporium fulvum), late blight disease (Phytophthora infestans), verticillium wilt disease (Verticillium albo-atrum, Verticillium dahliae), powdery mildew disease (Oidium neolycopersici), early blight disease (Alternaria solani), leaf mold disease (Pseudocercospora fuligena), and the like.

Eggplant: gray mold disease (Botrytis cinerea), black rot disease (Corynespora melongenae), powdery mildew (Erysiphe cichoracearum), leaf mold disease (Mycovellosiella nattrassii), sclerotinia rot disease (Sclerotinia sclerotiorum), verticillium wilt disease (Verticillium dahliae), phomopsis blight (Phomopsis vexans), and the like.

Strawberry: gray mold disease (Botrytis cinerea), powdery mildew (Sphaerotheca humuli), anthracnose disease (Colletotrichum acutatum, Colletotrichum fragariae), phytophthora rot disease (Phytophthora cactorum), soft rot disease (Rhizopus stolonifer), fsarium wilt disease (Fusarium oxysporum), verticillium wilt disease (Verticillium dahliae), and the like.

Onion: neck rot disease (Botrytis allii), gray mold disease (Botrytis cinerea), leaf blight disease (Botrytis squamosa), downy mildew disease (Peronospora destructor), Phytophthora porri disease (Phytophthora porri), leaf blight disease (Ciobrinia allii), and the like.

Green onion: soft rot disease (Pectobacterium carotovorum), downy mildew (Peronospora destructor), leaf scorch disease (Pleospora allii), bulb black rot disease (Sclerotium cepivorum), rust disease (Puccinia allii), leaf blight disease (Botrytis squamosa), and the like.

Cabbage: clubroot disease (Plasmodiophora brassicae), soft rot disease (Erwinia carotovora), black rot disease (Xanthomonas campesrtis pv. campestris), bacterial black spot disease (Pseudomonas syringae pv. maculicala, Pseudomonas syringae pv. alisalensis), downy mildew disease (Peronospora parasitica), sclerotinia rot disease (Sclerotinia sclerotiorum), black spot disease (Alternaria brassicicola), gray mold disease (Botrytis cinerea), and the like.

Common bean: sclerotinia rot disease (Sclerotinia sclerotiorum), gray mold disease (Botrytis cinerea), anthracnose (Colletotrichum lindemuthianum), angular spot disease (Phaeoisariopsis griseola), and the like.

Apple: powdery mildew disease (Podosphaera leucotricha), scab disease (Venturia inaequalis), Monilinia disease (Monilinia mali), black spot disease (Mycosphaerella pomi), valsa canker disease (Valsa mali), alternaria blotch disease (Alternaria mali), rust disease (Gymnosporangium yamadae), ring rot disease (Botryosphaeria berengeriana), anthracnose disease (Glomerella cingulata, Colletotrichum acutatum), leaf srot disease (Diplocarpon mali), fly speck disease (Zygophiala jamaicensis), Sooty blotch (Gloeodes pomigena), violet root rot disease (Helicobasidium mompa), gray mold disease (Botrytis cinerea), fire blight disease(Erwinia amylovora), and the like.

Japanese apricot: scab disease (Cladosporium carpophilum), gray mold disease (Botrytis cinerea), brown rot disease (Monilinia mumecola), sooty blotch (Peltaster sp.), and the like.

Persimmon: powdery mildew disease (Phyllactinia kakicola), anthracnose disease (Gloeosporium kaki), angular leaf spot (Cercospora kaki), circular leaf spot (Mycosphaerella nawae), and the like.

Peach: brown rot disease (Monilinia fructicola), scab disease (Cladosporium carpophilum), phomopsis rot disease (Phomopsis sp.), bacterial shot hole disease (Xanthomonas campestris pv. pruni), leaf curl disease (Taphrina deformans), anthracnose disease (Colletotrichum gloeosporioides), and the like.

Almond: brown rot disease (Monilinia laxa), spot blotch disease (Stigmina carpophila), scab disease (Cladosporium carpophilum), red leaf spot disease (Polystigma rubrum), alternaria blotch disease (Alternaria alternata), anthracnose (Colletotrichum gloeospoides), and the like.

Yellow peach: brown rot disease (Monilinia fructicola), anthracnose disease (Colletotrichum acutatum), black spot disease (Alternaria sp.), Monilinia Kusanoi disease (Monilinia kusanoi), Mycosphaerella leaf spot disease (Mycosphaerella cerasella), and the like.

Grape: gray mold disease (Botrytis cinerea), powdery mildew disease (Uncinula necator), ripe rot disease (Glomerella cingulata, Colletotrichum acutatum), downy mildew disease (Plasmopara viticola), anthracnose disease

(Elsinoe ampelina), brown spot disease (Pseudocercospora vitis), black rot disease (Guignardia bidwellii), white rot disease (Coniella castaneicola), rust disease (Phakopsora ampelopsidis), Cottony bunch (the pathogen thereof has not been identified), and the like.

Pear: scab disease (Venturia nashicola), rust disease (Gymnosporangium asiaticum), black spot disease (Alternaria kikuchiana), ring rot disease (Botryosphaeria berengeriana), powdery mildew disease (Phyllactinia mali), cytospora canker disease (Phomopsis fukushii), brown spot blotch disease (Stemphylium vesicarium), anthracnose disease (Glomerella cingulata), and the like.

Tea: ring spot disease (Pestalotiopsis longiseta, P. theae), anthracnose disease (Colletotrichum theae-sinensis), net blister blight disease (Exobasidium reticulatum), and the like.

Citrus fruits: scab disease (Elsinoe fawcetti), blue mold disease (Penicillium italicum), common green mold disease (Penicillium digitatum), gray mold disease (Botrytis cinerea), melanose disease (Diaporthe citri), canker disease (Xanthomonas campestris pv.Citri), powdery mildew disease (Oidium sp.), brown rot disease (Phytophthora citrophthora), anthracnose disease (Colletotrichum fioriniae), and the like.

Wheat: powdery mildew (Blumeria graminis f.sp. tritici), red mold disease (Gibberella zeae), red rust disease (Puccinia recondita), yellow rust disease (Puccinia striiformis), brown snow mold disease (Pythium iwayamai), pink snow mold disease (Monographella nivalis), eye spot disease (Pseudocercosporella herpotrichoides), leaf scorch disease (Septoria tritici), glume blotch disease (Leptosphaeria nodorum), typhulasnow blight disease (Typhula incarnata), sclerotinia snow blight disease (Myriosclerotinia borealis), damping-off disease (Gaeumannomyces graminis), ergot disease (Claviceps purpurea), stinking smut disease (Tilletia caries), loose smut disease (Ustilago nuda), blast disease (Pyricularia grisea), and the like.

Barley: leaf spot disease (Pyrenophora graminea), net blotch disease (Pyrenophora teres), leaf blotch disease (Rhynchosporium secalis), loose smut disease (Ustilago tritici, U.nuda), and the like.

Rice: blast disease (Pyricularia oryzae), sheath blight disease (Rhizoctonia solani), bakanae disease (Gibberella fujikuroi), brown spot disease (Cochliobolus miyabeanus), damping-off disease (Pythium graminicolum), bacterial leaf blight (Xanthomonas oryzae), bacterial seedling blight disease (Burkholderia plantarii), brown stripe disease (Acidovorax avenae), bacterial grain rot disease (Burkholderia glumae), Cercospora leaf spot disease (Cercospora oryzae), false smut disease (Ustilaginoidea virens), rice brown spot disease (Alternaria alternata, Curvularia intermedia), kernel discoloration of rice (Alternaria padwickii), pink coloring of rice grains (Epicoccam purpurascenns), and the like.

Tobacco: sclerotinia rot disease (Sclerotinia sclerotiorum), powdery mildew disease (Erysiphe cichoracearum), phytophthora rot disease (Phytophthora nicotianae), and the like.

Tulip: gray mold disease (Botrytis cinerea), and the like.

Sunflower: downy mildew disease (Plasmopara halstedii), sclerotinia rot disease (Sclerotinia sclerotiorum), gray mold disease (Botrytis cinerea), and the like.

Bent grass: Sclerotinia snow blight (Sclerotinia borealis), large patch (Rhizoctonia solani), brown patch (Rhizoctonia solani), Dollar spot (Sclerotinia homoeocarpa), blast disease (Pyricularia sp.), Pythium red blight disease (Pythium aphanidermatum), anthracnose disease (Colletotrichum graminicola), and the like.

Orchard grass: powdery mildew disease (Erysiphe graminis), and the like.

Soybean: purple stain disease (Cercospora kikuchii), downy mildew disease (Peronospora manshurica), phytophthora rot disease (Phytophthora sojae), rust disease (Phakopsora pachyrhizi), sclerotinia rot disease (Sclerotinia sclerotiorum), anthracnose disease (Colletotrichum truncatum), gray mold disease (Botrytis cinerea), Sphaceloma scab (Elsinoe glycines), black spot disease (Diaporthe phaseolorum var. sojae), and the like.

Potato: Phytophthora rot disease (Phytophthora infestans), early blight disease (Aleternaria solani), scurf disease (Thanatephorus cucumeris), verticillium wilt disease (Verticillium albo-atrum, V. dahliae, V. nigrescens), blackleg disease (Pectobacterium atrosepticum), soft rot disease (Pectobacterium carotovorum), and the like.

Banana: Panama disease (Fusarium oxysporum), Sigatoka disease (Mycosphaerella fijiensis, M. musicola), and the like.

Mango: anthracnose (Colletotrichum aenigma)

Rape seed: sclerotinia rot disease (Sclerotinia sclerotiorum), root rot disease (Phoma lingam), black leaf spot disease (Alternaria brassicae), and the like.

Coffee: rust disease (Hemileia vastatrix), anthracnose (Colletotrichum coffeanum), brown eye spot disease (Cercospora coffeicola), and the like.

Sugarcane: brown rust disease (Puccinia melanocephala), and the like.

Corn: zonate spot disease (Gloecercospora sorghi), rust disease (Puccinia sorghi), southern rust disease (Puccinia polysora), smut disease (Ustilago maydis), brown spot disease (Cochliobolus heterostrophus), northern leaf blight (Setophaeria turcica), and the like.

Cotton: seedling blight disease (Pythium sp), rust disease (Phakopsora gossypii), sour rot disease (Mycosphaerella areola), anthracnose (Glomerella gossypii), and the like.

The agricultural or horticultural fungicide according to the present invention is preferably applied to cereals;

vegetables; root vegetables; potatoes; trees, such as fruitbearing trees, tea, coffee, or cacao; feed crops; lawn grasses; or plants such as cotton.

The agricultural or horticultural fungicide according to the present invention may be applied to any portions of plants, such as leaves, stems, stalks, flowers, buds, fruits, seeds, sprouts, roots, tubers, tuberous roots, shoots, or slips. The agricultural or horticultural fungicide according to the present invention may also be applied to varieties, improved varieties, cultivars, mutants, hybrid bodies, or gene recombinants (GMO) of the plants.

The agricultural or horticultural fungicide according to the present invention may be used to conduct seed treatment, foliage application, soil application, or submerged application so as to control various diseases generated in agricultural or horticultural crop plants including flowers, lawn grasses, and herbages.

The agricultural or horticultural fungicide according to the present invention may further contain additional components different from the heterocyclic compound according to the present invention. Examples of the additional components include conventional carriers available to conduct formulation. Examples of other additional components include conventional fungicides, insecticides, acaricides, nematicides, soil pest control agents, plant regulatory agents, synergists, fertilizers, soil improvement agents, and animal feeds. The presence of an additional component may cause synergistic effects.

Specific examples of the fungicides which may be mixed or used with the agricultural or horticultural fungicide according to the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, ofurace;
(b) Adenosine deaminase inhibitors: bupirimate, dimethirimol, ethirimol;
(c) DNA/RNA synthesis inhibitors: hymexazol, octhilinone;
(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, ethaboxam;
(b) Cell division inhibitors: pencycuron;
(c) Spectrin-like protein delocalization inhibitors: fluopicolide.

(3) Respiration inhibitors:

(a) Complex I NADH oxidation-reduction enzyme inhibitors: diflumetorimu, tolfenpyrad;
(b) Complex II succinate dehydrogenase inhibitors: benodanil, flutolanil, mepronil, isofetamide, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, pyraziflumid, pydiflumetofen, isoflucypram, inpyrfluxam;
(c) Complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, mandestrobin, metyltetraprole;
(d) Complex III ubiquinol reductase Qi inhibitors: cyazofamid, amisulbrom, fenpicoxamid;
(e) Oxidative phosphorylation uncoupling agents: binapacryl, meptyldinocap, dinocap, fluazinam, ferimzone;
(f) Oxidative phosphorylation inhibitors (ATP synthase inhibitors): fentin acetate, fentin chloride, fentin hydroxide;
(g) ATP production inhibitors: silthiofam;
(h) Complex III: Qx (unknown) inhibitor of cytochrome bc1 (ubiquinone reductase): ametoctradin.

(4) Amino acid and protein synthesis inhibitors

(a) Methionine biosynthesis inhibitors: andoprim, cyprodinil, mepanipyrim, pyrimethanil;
(b) Protein synthesis inhibitors: blasticidin-S, kasugamycin, kasugamycin hydrochloride, streptomycin, oxytetracycline.

(5) Signal transduction inhibitors:

(a) Signal transduction inhibitors: quinoxyfen, proquinazid;
(b) Inhibitors of MAP / histidine kinase in osmotic signal transduction: fenpiclonil, fludioxonil, chlozolinate, iprodione, procymidone, vinclozolin.

(6) Lipids and cell membrane synthesis inhibitors:

(a) Inhibitors of phospholipid biosynthesis, methyltransferase: edifenphos, iprobenfos, pyrazophos, isoprothiolane;
(b) Lipid peroxidation agent: biphenyl, chloroneb, dicloran, quintozene, tecnazene, tolclofos-methyl, etridiazole;
(c) Agents that act on the cell membrane: iodocarb, propamocarb, propamocarbhydrochloride, propamocarb-fosetylate, prothiocarb;
(d) Microorganisms that disrupt the cell membrane of pathogens: bacillus subtilis bacteria, bacillus subtilis strain QST713, bacillus subtilis strain FZB24, bacillus subtilis strain MBI600, bacillus subtilis strain D747, bacillus amyloliquefaciens;
(e) Agents that disrupt the cell membrane: melaleuca alternifolia (tea tree) extract.

(7) Inhibitors of sterol biosynthesis in the cell membrane:

(a) Inhibitors of demethylation at the C14 position in sterol biosynthesis: triforine, pyrifenox, pyrisoxazole, fenarimol, flurprimidol, nuarimol, imazalil, imazalil-sulphate, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, mefentrifluconazole;
(b) Inhibitors of $\Delta 14$ reductase and $\Delta 8 \rightarrow \Delta 7$-isomerase in sterol biosynthesis: aldimorph, dodemorph, dodemorph acetate, fenpropimorph, tridemorph, fenpropidin, piperalin, spiroxamine;
(c) Inhibitors of 3-keto reductase in C4 demethylation in sterol biosynthesis: fenhexamid, fenpyrazamine;
(d) Inhibitors of squalene epoxidase in sterol biosynthesis: pyributicarb, naftifine, terbinafine.

(8) Cell wall synthesis inhibitors

(a) Trehalase inhibitor: validamycin;
(b) Chitin synthesis inhibitors: polyoxin, polyoxorim;
(c) Cellulose synthase inhibitors: dimethomorph, flumorph, pyrimorph, benthiavalicarb-isopropyl, iprovalicarb, valifenalate, mandipropamid.

(9) Melanin biosynthesis inhibitors

(a) Melanin biosynthesis reductase inhibitors: fthalide, pyroquilon, tricyclazole;
(b) Melanin biosynthesis anhydrase inhibitors: carpropamid, diclocymet, fenoxanil.
(c) Melanin biosynthesis polyketide synthase inhibitors: tolprocarb.

(10) Host plant resistance inducer:

(a) Agents that act on the salicylic acid synthesis pathway: acibenzolar-S-methyl;
(b) Others: probenazole, tiadinil, isotianil, dichlobentiazox, laminarin, reynoutria sachalinensis extract.

(11) Agents, action of which is unclear: cymoxanil, fosetyl-aluminum, phosphoric acid (phosphate), tecloftalam, triazoxide, flusulfamide, diclomezine, methasulfocarb, cyflufenamid, metrafenone, pyriofenone, dodine, dodine free base, flutianil.
(12) Agents having multi-functionality: copper (copper salt), bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, iminoctadine triacetate, iminoctadine trialbesilate, anilazine, dithianon, quinomethionate, fluoroimide.
(13) Other agents: DBEDC, fluorofolpet, guazatine acetate, bis (8-quinolinolato) copper (II), propamidine, chloropicrin, cyprofuram, agrobacterium, bethoxazin, diphenylamine, methyl isothiocyanate (MITC), mildiomycin, capsaicin,

cufraneb, cyprosulfamide, dazomet, debacarb, dichlorophen, flumetover, fosetyl-calcium, fosetylsodium, irumamycin, natamycin, nitrothal isopropyl, oxamocarb, pyrrolnitrin, tebufloquin, tolnifanide, zarilamid, algophase, amicarthiazol, oxathiapiprolin, metiram zinc, benthiazole, trichlamide, uniconazole, oxyfenthiin, picarbutrazox, dichlobentiazox, quinofumelin, thiuram, ambam, agrobacterium radiobacter, coniothyrium minitans, pseudomonas fluorescens, pseudomonas rhodesiae, talaromyces flavus, trichoderma atroviride, erwinia carotovora subsp. carotovora, bacillus simplex, variovorax paradoxus, lactobacillus plantarum, florylpicoxamid, pyrapropoyne, fluindapyr, aminopyrifen, pyridachlometyl, ipflufenoquin.

Specific examples of the insecticides, acaricides, nematicides, soil insect pest control agents, and anthelmintic agents which may be mixed or used with the agricultural or horticultural fungicide according to the present invention are shown below.

(1) Acetylcholinesterase inhibitors:

(a) Carbamates: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, chloethocarb, metam-sodium, promecarb;
(b) Organophosphate-based: acephate, azamethiphos, azinphos-ethyl, azinphosmethyl, cadusafos, chloroethoxyfos, chlorfenvinphos, chlormephos, chlorpyrifos, chlorpyrifos-methyl, coumaphos, cyanophos, demeton-S-methyl, diazinon, dichlorvos / DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S methyl sulfone, dialifos, dichlofenthion, dioxabenzofos, etrimfos, fensulfothion, flupyrazofos, fonofos, formothion, fosmethilan, isazofos, jodfenphos, methacrifos, pirimiphos-ethyl, phosphocarb, propaphos, prothoate, sulprofos.

(2) GABAergic chloride ion channel antagonists: acetoprole, chlordane, endosulfan, ethiprole, fipronil, pyrafluoprole, pyriprole, camphechlor, heptachlor, dienochlor.
(3) Sodium channel modulators: acrinathrin, d-cis-trans allethrin, d-trans allethrin, bifenthrin, bioallethrin, bioallethrin s-cyclopentyl isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ζ-cypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, τ-fluvalinate, halfenprox, imiprothrin, kadethrin, permethrin, phenothrin [(1R)-trans isomer], prallethrin, pyrethrum, resmethrin, silafluofen, tefluthrin, tetramethrin [(1R)-isomer], tralomethrin, transfluthrin, allethrin, pyrethrins, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, terallethrin.
(4) Nicotinic acetylcholine receptor agonist: acetamiprid, clothianidin, dinotefuran, imidacloprid, nitenpyram, nithiazine, thiacloprid, thiamethoxam, sulfoxaflor, nicotine, flupyradifurone, flupyrimine.
(5) Nicotinic acetylcholine receptor allosteric modulators: spinetoram, spinosad.
(6) Chloride channel activators: abamectin, emamectin-benzoate, lepimectin, milbemectin, ivermectin, selamectin, doramectin, eprinomectin, moxidectin, milbemycin, milbemycin oxime, nemadectin.
(7) Juvenile hormone-like substances: hydroprene, kinoprene, methoprene, fenoxycarb, pyriproxyfen, diofenolan, epofenonane, triprene.
(8) Other non-specific inhibitors: methyl bromide, chloropicrin, sulfuryl fluoride, borax, tartar emetic.
(9) Homoptera selective feeding inhibitors: flonicamid, pymetrozine, pyrifluquinazon.
(10) Acarian growth inhibitors: clofentezine, diflovidazin, hexythiazox, etoxazole.
(11) Midgut inner membrane disrupting agent derived from microorganisms: bacillus thuringiensis subsp. Israelensis, bacillus sphaericus, bacillus thuringiensis subsp. aizawai, bacillus thuringiensis subsp. kurstaki, bacillus thuringiensis subsp. tenebrionis, Bt crop protein: Cry1Ab, Cry1Ac, Cry1Fa, Cry1A.105, Cry2Ab, Vip3A, mCry3A, Cry3Ab, Cry3Bb, Cry34Ab1 / Cry35Ab1.
(12) Mitochondrial ATP biosynthetic enzyme inhibitors: diafenthiuron, azocyclotin, cyhexatin, fenbutatin oxide, propargite, tetradifon.
(13) Oxidative phosphorylation uncouplers: chlorfenapyr, sulfluramid, DNOC, binapacryl, dinobuton, dinocap.

(14) Nicotinic acetylcholine receptor channel blockers: bensultap, cartap hydrochloride, nereistoxin, thiosultap-sodium, thiocyclam.
(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, fluazuron.
(16) Diptera molting disrupting agents: cyromazine.
(17) Molting hormone receptor agonists: chromafenozide, halofenozide, methoxyfenozide, tebufenozide.
(18) Octopamine receptor agonists: amitraz, demiditraz, chlordimeform.
(19) Mitochondrial electron transport system complex III inhibitors: acequinocyl, fluacrypyrim, hydramethylnon.
(20) Mitochondrial electron transport system complex I inhibitors: fenazaquin, fenpyroximate, pyrimidifen, pyridaben, tebufenpyrad, tolfenpyrad, rotenone.
(21) Voltage-dependent sodium channel blockers: indoxacarb, metaflumizone.
(22) Acetyl CoA carboxylase inhibitors: spirodiclofen, spiromesifen, spirotetramat.
(23) Mitochondrial electron transport system complex IV inhibitors: aluminum phosphide, calcium phosphide, phosphine, zinc phosphide, cyanide.
(24) Mitochondrial electron transport system complex II inhibitors: cyenopyrafen, cyflumetofen, pyflubumide.
(25) Ryanodine receptor modulators: chlorantraniliprole, cyantraniliprole, flubendiamide, cyclaniliprole, tetraniliprole.
(26) Mixed function oxidase inhibitor compounds: piperonyl butoxide.
(27) Latrophilin receptor agonists: depsipeptide, cyclodepsipeptide, 24 membered cyclodepsipeptide, emodepside.
(28) Other agents (mechanism of which have not been known): azadirachtin, benzoximate, bifenazate, bromopropylate, quinomethionate, cryolite, dicofol, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimine, fluphenazine, gossyplure, japonilure, metoxadiazone, oil, potassium oleate, tetrasul, triarathene, afidopyropen, flometoquin, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazole-3-yl]-2-(1H-1,2,4-triazole-1-yl) benzonitrile (CAS: 943137-49-3), broflanilide, other metahnediamedes, steinernema carpocapsae, steinernema glaseri, pasteuria penetrans, paecilomyces tenuipes, paecilomyces fumosoroseus, beauveria bassiana, beauveria brongniartii, metarhizium anisopliae, verticillium lecanii, acynonapyr.
(29) Anthelmintic agents:

(a) Benzimidazole-based: fenbendazole, albendazole, triclabendazole, oxibendazole, mebendazole, oxfendazole, parbendazole, flubendazole, febantel, netobimin, thiophanate, thiabendazole, cambendazole;
(b) Salicylanilide-based: closantel, oxyclozanide, rafoxanide, niclosamide;
(c) Substituted phenol-based: nitroxinil, nitroscanate;
(d) Pyrimidine-based: pyrantel, morantel;
(e) Imidazothiazole-based: levamisole, tetramisole;
(f) Tetrahydropyrimidine-based: praziquantel, epsiprantel;
(g) Other anthelmintic agents: cyclodiene, ryania, clorsulon, metronidazole, demiditraz, piperazine, diethylcarbamazine, dichlorophen, monepantel, tribendimidine, amidantel, thiacetarsamide, melarsomine, arsenamide.

Specific examples of plant regulatory agents which can be mixed or used with the agricultural or horticultural fungicide according to the present invention are shown below.
Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (another name: aviglycine), aminooxyacetate, silver nitrate, cobalt chloride, IAA, 4-CPA, cloprop, 2,4-D, MCPB, indole-3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyladenine, 5-amino levulinic acid, daminozide.

(Preparation formulation)

The agricultural or horticultural fungicide according to the present invention is not particularly limited by the dosage form thereof. Examples of the dosage form include wettable powder, emulsion, powder, granule, water-soluble agent, suspension, granular wettable powder, and tablet. The method for preparing formulation is not particularly limited, and any conventional preparation method may be adopted depending on the dosage form.
Some preparation examples are shown below. The following preparation formulations are shown as examples, and may be modified to the extent that the modification is consistent with the gist of the present invention, and the present invention is not intended to be limited to the following preparation examples. The term "part" means "part by mass"

unless particularly mentioned.

(Preparation 1: wettable powder)

40 parts of a heterocyclic compound according to the present invention, 53 parts of diatomaceous earth, 4 parts of higher alcohol sulfuric acid ester, and 3 parts of alkyl naphthalene sulfonate were mixed uniformly, and then finely pulverized to obtain wettable powders containing 40% by mass of the active ingredient.

(Preparation 2: emulsion)

30 parts of a heterocyclic compound according to the present invention, 33 parts of xylene, 30 parts of dimethylformamide, and 7 parts of polyoxyethylene alkyl allyl ether were mixed and dissolved to obtain an emulsion containing 30% by mass of the active ingredient.

(Preparation 3: granule)

5 parts of a heterocyclic compound according to the present invention, 40 parts of talc, 38 parts of clay, 10 parts of bentonite, and 7 parts of sodium alkyl sulfate were mixed uniformly, and then finely pulverized, followed by granulating to obtain a particle diameter of 0.5 to 1.0 mm, and thus granules containing 5% by mass of the active ingredient were obtained.

(Preparation 4: granule)

5 parts of a heterocyclic compound according to the present invention, 73 parts of clay, 20 parts of bentonite, 1 part of sodium dioctyl sulfosuccinate, and 1 part of potassium phosphate were mixed and then pulverized, followed by adding water thereto, and then kneading the mixture. Then, granulation and drying were conducted to obtain granules containing 5% by mass of the active ingredient.

(Preparation 5: suspension)

10 parts of a heterocyclic compound according to the present invention, 4 parts of polyoxyethylene alkyl allyl ether, 2 parts of sodium polycarboxylate, 10 parts of glycerin, 0.2 parts of xanthan gum, and 73.8 parts of water were mixed, and then wet-pulverized until the particle size became 3 $\mu$m or less to obtain a suspension containing 10% by mass of the active ingredient.

(Preparation 6: granular wettable powder)

40 parts of a heterocyclic compound according to the present invention, 36 parts of clay, 10 parts of potassium chloride, 1 part of sodium alkylbenzene sulfonate, 8 parts of sodium lignin sulfonate, and 5 parts of formaldehyde condensate of alkylbenzene sulfonate were mixed uniformly and then pulverized finely, and then an appropriate amount of water was added and kneaded thereinto until the resultant became clayey. The clayey resultant was granulated and then dried to obtain granular wettable powders containing 40% by mass of the active ingredient.

Then, compound examples are shown to explain the present invention further specifically. However, the present invention is not intended to be limited by the following compound examples.

(Example 1)

Synthesis of 2-((benzo[d]thiazol-6-yloxy)(5-fluoropyridin-2-yl)methyl)-1,3,4-oxadiazole (compound number a-8)

(Step 1) Synthesis of ethyl 2-(benzo[d]thiazol-6-yloxy)-2-(5-fluoropyridin-2-yl)acetate

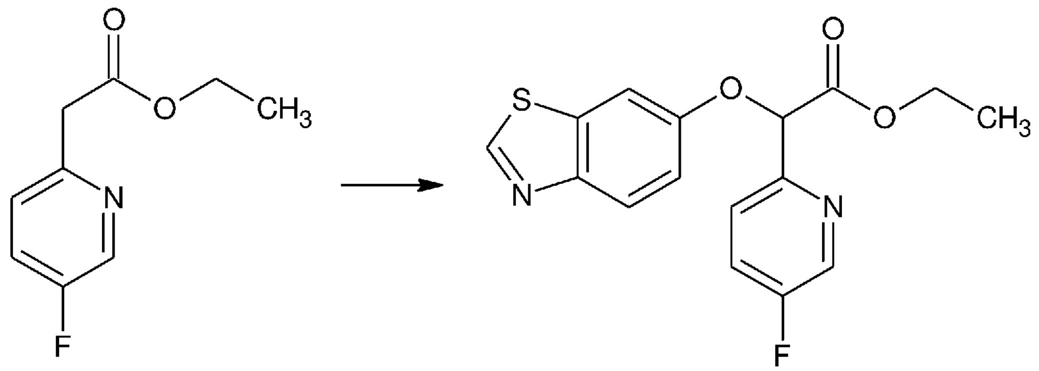

Ethyl 2-(5-fluoropyridin-2-yl)acetate (1.00 g, 5.46 mmol) was dissolved in carbon tetrachloride (10 mL), and then N-bromosuccinimide (1.02 g, 5.73 mmol) and benzoyl peroxide (0.09 g, 0.28 mmol) were added thereto, followed by stirring the mixture while heating the mixture to reflux for 6 hours. The resultant was left to cool, and then filtrated through CELITE, followed by concentrating the filtrate under reduced pressure. A N,N-dimethylformamide solution of the resultant residue (1 mL) was added to a N,N-dimethylformamide solution (5 mL) of 6-hydroxybenzothiazole (0.55 g, 3.62 mmol) and potassium carbonate (0.91 g, 6.58 mmol), and then stirring the mixture at room temperature overnight. The reaction liquid was poured into water, and then extracted with ethyl acetate. The resultant organic layer was washed with water and saturated saline, and then dried over anhydrous magnesium sulfate, followed by removing an inorganic product by filtration, and then concentrating the resultant under reduced pressure. The resultant residue was purified by silica gel chromatography to obtain 0.63 g of the target product (yield 63%).

The $^1$H-NMR result of the obtained target product is shown below.

$^1$H-NMR (400 MHz, $CDCl_3$): δ1.23 (dd, 3H), 4.19-4.32 (m, 2H), 5.85 (s, 1H), 7.21 (d, 1H), 7.42-7.51 (m, 2H), 7.69 (dd, 1H), 8.05 (d, 1H), 8.46 (d, 1H), 8.83 (s, 1H).

(Step 2) Synthesis of 2-((benzo[d]thiazol-6-yloxy)(5-fluoropyridin-2-yl)methyl)-1,3,4-oxadiazole)

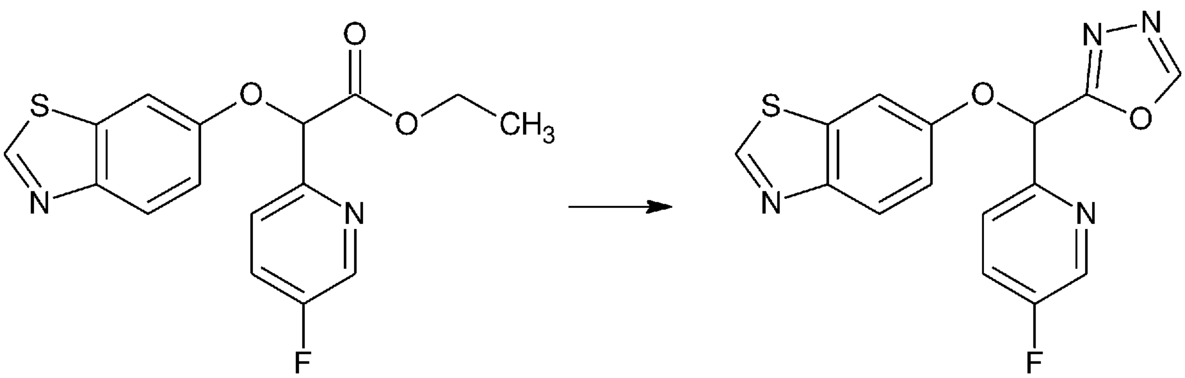

Ethyl 2-(benzo[d]thiazol-6-yloxy)-2-(5-fluoropyridin-2-yl) acetate (0.63 g, 1.90 mmol) was dissolved in tetrahydrofuran (10 mL) and ethanol (1 mL), and then hydrazine monohydrate (0.29 g, 5.79 mmol) was added thereto, followed by stirring the mixture while heating the mixture to reflux for 6 hours. The solvent was distilled off under reduced pressure, and the resultant residue was diluted with ethyl acetate, followed by washing the resultant organic layer with water and saturated saline. The resultant organic layer was dried over anhydrous magnesium sulfate, and an inorganic product was removed by filtration, followed by concentrating the resultant under reduced pressure. The resultant light yellow solid (0.49 g) was dissolved in acetic acid (5 mL), and then trimethyl orthoformate (0.49 g, 4.62 mmol) was added thereto, followed by stirring the mixture while heating the mixture to reflux for 10 minutes. The resultant was left to cool, and the solvent was distilled off under reduced pressure, followed by neutralizing the residue with saturated sodium bicarbonate water and then subjecting the resultant to extraction with ethyl acetate. The resultant organic layer was washed with water and saturated saline, and then dried over anhydrous magnesium sulfate, followed by removing an inorganic product by filtration and then concentrating the resultant under reduced pressure. The resultant residue was purified with column chromatography to obtain 0.22 g of a target product (yield 35%).

The $^1$H-NMR result of the resultant target product is shown below.

$^1$H-NMR (400 MHz, $CDCl_3$ ): δ6.76 (s, 1H), 7.27 (dd, 1H), 7.48-7.57 (m, 1H), 7.59 (d, 1H), 7.85 (dd, 1H), 8.03 (d, 1H), 8.45 (d, 2H), 8.89 (s, 1H).

(Example 2)

Synthesis of 2-(1-(benzo[d]thiazol-6-yloxy)pentyl)-1,3,4-oxadiazole (compound number a-1)

(Step 1) Synthesis of ethyl 2-(benzo[d]thiazol-6-yloxy)hexanoate

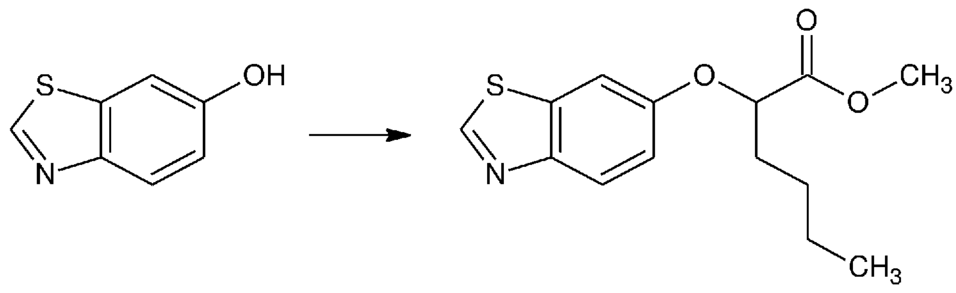

6-Hydroxybenzothiazole (0.25 g, 1.65 mmol) was dissolved in N,N-dimethylformamide (3 mL), and then potassium carbonate (0.27 g, 1.95 mmol) and methyl 2-bromohexanate (0.38 g, 1.81 mmol) were added thereto, followed by stirring the mixture at room temperature overnight. Water was added to the reaction liquid, and the mixture was subjected to extraction with ethyl acetate, followed by washing the resultant organic layer with saturated saline. The organic layer was dried over anhydrous magnesium sulfate, and then an inorganic product was filtered off, followed by concentrating the resultant under reduced pressure. The resultant residue was purified by silica gel chromatography to obtain 0.45 g of the target product (yield 97%).

(Step 2) Synthesis of 2-(1-(benzo[d]thiazol-6-yloxy)pentyl)-1,3,4-oxadiazole

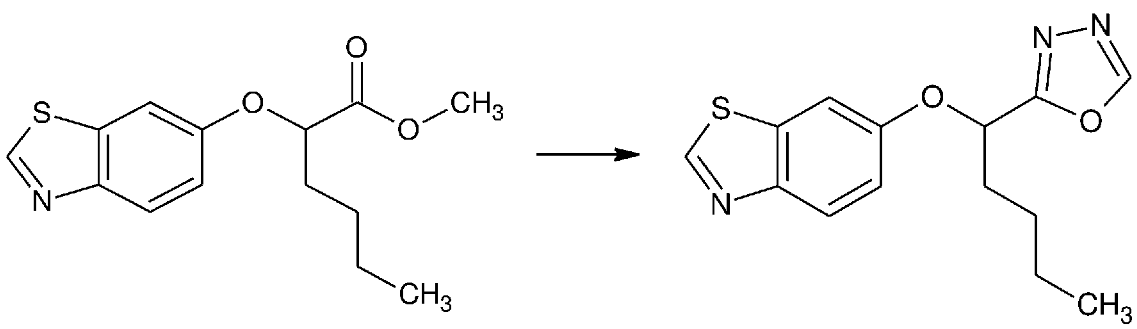

Ethyl 2-(benzo[d]thiazol-6-yloxy)hexanoate (0.45 g, 1.61 mmol) was dissolved in methanol (6 mL), and then hydrazine monohydrate (0.40 g, 7.99 mmol) was added thereto, followed by stirring the mixture at room temperature for 2 days. Water was added to the reaction liquid, and the mixture was subjected to extraction with ethyl acetate, followed by washing the resultant organic layer with saturated saline. The resultant organic layer was dried over anhydrous magnesium sulfate, and then an inorganic product was filtered off, followed by concentrating the resultant under reduced pressure. The resultant light yellow solid (0.42 g) was dissolved in acetic acid (4 mL), and then trimethyl orthoformate (0.80 g, 7.54 mmol) was added thereto, followed by stirring the mixture while heating the mixture to reflux for 15 minutes. The resultant was left to cool, and then the solvent was distilled off under reduced pressure, followed by neutralizing the resultant residue with saturated sodium bicarbonate water, and then extracting the resultant with ethyl acetate. The resultant organic layer was washed with saturated saline and then dried over anhydrous magnesium sulfate, and an inorganic product was filtered off, followed by concentrating the resultant under reduced pressure. The resultant residue was purified with column chromatography to obtain 0.29 g of the target product (yield 67%).

The $^1$H-NMR result of the resultant target product is shown below.
$^1$H-NMR (400 MHz, $CDCl_3$ ): δ0.92 (dd, 3H), 1.31-1.46 (m, 3H), 1.50-1.61 (m, 1H), 2.01-2.13 (m, 1H) , 2.20-2.31 (m, 1H) , 5.55 (dd, 1H), 7.18 (dd, 1H), 7.52 (s, 1H), 7.99 (d, 1H), 8.38 (s, 1H), 8.84 (s, 1H).

(Example 3)

Synthesis of 2-((benzo[d]thiazol-6-yloxy) (4-(1-propynyl)pyridin-2-yl)methyl)-1,3,4- oxadiazole (2-((benzo[d]thiazol-6-yloxy)(4-(prop-1-yn-1-yl)pyridin-2-yl)methyl)-1,3,4-oxadiazole) (compound number a-45)

(Step 1) Synthesis of ethyl 4-((1-propynyl)pyridin-2-yl) acetate (ethyl 2-(4-(prop-1-yn-1-yl)pyridin-2-yl)acetate)

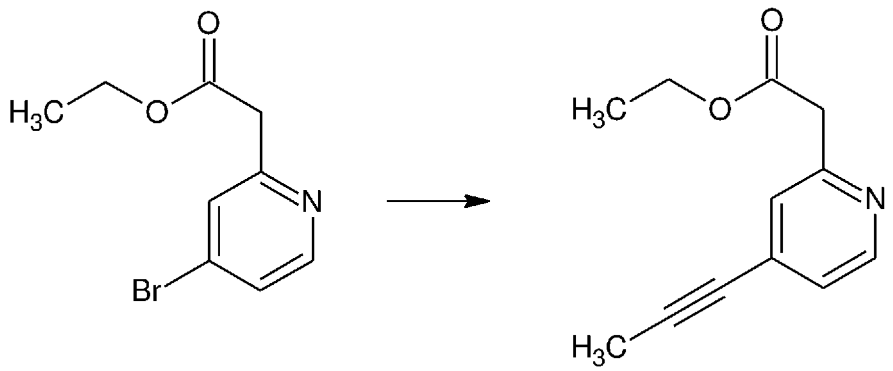

Ethyl 2-(4-bromopyridin-2-yl)acetate (0.65 g, 2.66 mmol) was dissolved in triethylamine (10 mL), and then 1-(trimethylsilyl)-1-propyne (0.33 g, 2.93 mmol), copper iodide (I) (51 mg, 0.26 mmol), bis(triphenylphosphine)palladium(II) dichloride (0.19 g, 0.27 mmol), and tetra-n-butylammonium fluoride (1M tetrahydrofuran solution) (3 mL) were added thereto, followed by replacing air in the reactor with nitrogen, and then stirring the mixture at 70°C overnight. The resultant was left to cool, and then the reaction liquid was diluted with ethyl acetate, followed by adding a saturated ammonium chloride aqueous solution thereto, and then conducting extraction and washing with water. The resultant organic layer was dried over anhydrous magnesium sulfate, and an inorganic product was filtered off, followed by concentrating the resultant under reduced pressure. The resultant residue was purified with column chromatography to obtain 0.38 g of the target product (yield 70%).
The $^1$H-NMR result of the resultant target product is shown below.
$^1$H-NMR (400 MHz, $CDCl_3$ ): δ1.26 (t, 3H), 2.07 (s, 3H), 3.79 (s, 2H), 4.18 (q, 2H), 7.14 (dd, 1H), 7.26 (s, 1H), 8.46 (d, 1H).

(Step 2) Synthesis of ethyl 2-(benzo[d]thiazol-6-yloxy)-2-(4-(1-propynyl)pyridin-2-yl) acetate (ethyl 2-(benzo[d]thiazol-6-yloxy)-2-(4-(prop-1-yn-1-yl)pyridin-2-yl)acetate)

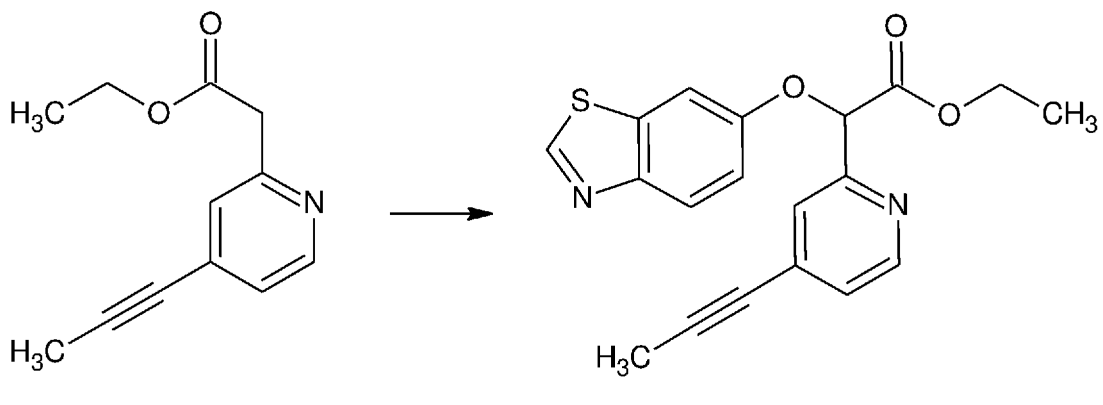

Ethyl 4-((1-propynyl)pyridin-2-yl)acetate (0.38 g, 1.87 mmol) was dissolved in carbon tetrachloride (6 mL), and then N-bromosuccinimide (0.33 g, 1.87 mmol) and benzoyl peroxide (60 mg, 0.18 mmol) were added thereto, followed

by stirring the mixture while heating the mixture to reflux for 2 hours. The resultant was left to cool, and the reaction liquid was filtrated through CELITE, followed by concentrating the filtrate under reduced pressure. An acetone solution of the resultant residue was added to an acetone solution (4 mL) containing 6-hydroxybenzothiazole (0.14 g, 0.93 mmol) and potassium carbonate (0.39 g, 2.82 mmol), followed by stirring the mixture at room temperature overnight. The reaction liquid was poured into water, and extracted with ethyl acetate. The resultant organic layer was washed with water, and saturated saline, and then dried over anhydrous magnesium sulfate, and an inorganic product was filtered off, followed by concentrating the resultant under reduced pressure. The resultant residue was purified by aminosilica gel chromatography to obtain 0.17 g of the target product (yield 51%).

(Step 3) Synthesis of 2-((benzo[d]thiazol-6-yloxy)(4-(1-propynyl)pyridin-2-yl)methyl)-1,3,4-oxadiazole (2-((benzo[d]thiazol-6-yloxy)(4-(prop-1-yn-1-yl)pyridin-2-yl)methyl)-1,3,4-oxadiazole)

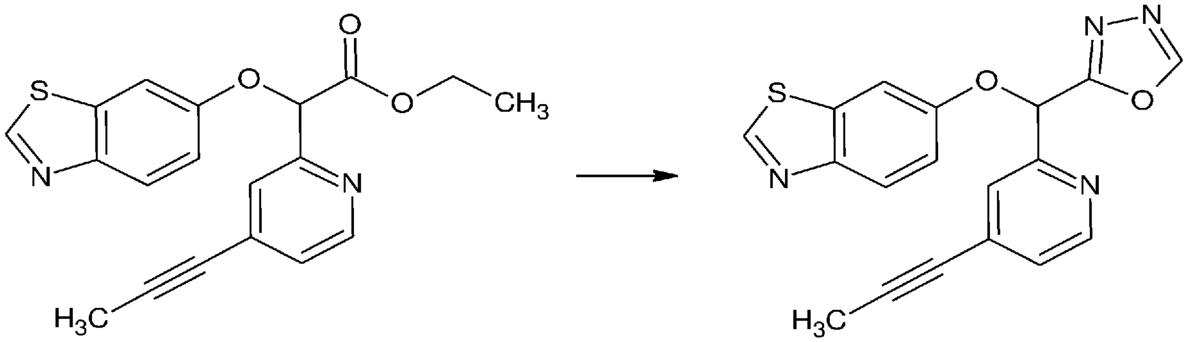

Ethyl 2-(benzo[d]thiazol-6-yloxy)-2-(4-(1-propynyl)pyridin-2-yl) acetate (0.17 g, 0.48 mmol) was dissolved in tetrahydrofuran (3 mL), and then hydrazine monohydrate (96 mg, 1.91 mmol) and one drop of 1,8-diazabicyclo[5.4.0]-7-undecen were added thereto, followed by stirring the mixture at room temperature overnight. Water was added to the reaction liquid, and the mixture was subjected to extraction with ethyl acetate, followed by washing the resultant organic layer with saturated saline. The resultant organic layer was dried over anhydrous magnesium sulfate, and an inorganic product was filtered off, followed by concentrating the resultant under reduced pressure. The resultant residue was dissolved in acetic acid (4 mL), and then trimethyl orthoformate (0.20 g, 1.88 mmol) was added thereto, followed by stirring the mixture while heating the mixture to reflux for 30 minutes. The resultant reaction liquid was concentrated under reduced pressure, ethyl acetate was added thereto, and then the mixture was washed with saturated sodium bicarbonate water, followed by purifying the resultant with column chromatography to obtain 0.07 g of the target product (yield 41%).

The $^{1}$H-NMR result of the target product is shown below.

$^{1}$H-NMR (400 MHz, $CDCl_3$): δ2.08 (s, 3H), 6.72 (s, 1H), 7.23-7.32 (m, 2H), 7.60 (d, 1H), 7.77 (s, 1H), 8.03 (d, 1H), 8.42 (s, 1H), 8.51 (d, 1H), 8.87 (s, 1H).

(Example 4)

Synthesis of 2-((4-bromobenzo[d]thiazol-6-yloxy)(5-fluoropyridin-2-yl)methyl)-1,3,4-oxadiazole) (compound number a-20)

(Step 1) Synthesis of 2-((5-fluoropyridin-2-yl)methyl)-1,3,4-oxadiazole

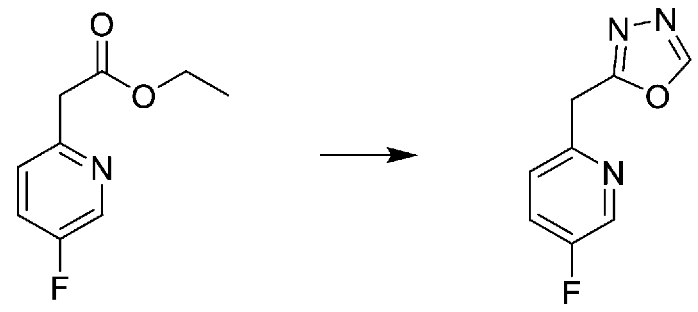

Ethyl 2-(5-fluoropyridin-2-yl) acetate (30.0 g, 164 mmol) was dissolved in tetrahydrofuran (540 mL), and then hydrazine monohydrate (24.6 g, 5491 mmol) and 1,8-diazabicyclo[5.4.0]-7-undecen (5.0 g, 32.8 mmol) were added thereto, followed by stirring the mixture while heating the mixture to reflux overnight. The solvent was distilled off under reduced pressure, and the resultant residue was washed with ether to collect light yellow solid. The collected light yellow solid (34.1 g) was dissolved in acetic acid (540 mL), and then trimethyl orthoformate (86.9 g, 819 mmol) was added thereto, followed by stirring the mixture while heating the mixture to reflux for 30 minutes. The resultant was left to cool, and then the solvent was distilled off under reduced pressure, followed by neutralizing the resultant residue with saturated sodium bicarbonate water, and then subjecting the resultant to extraction with ethyl acetate. The resultant organic layer was washed with water and saturated saline and then dried over anhydrous magnesium sulfate, and an inorganic product was filtered off, followed by concentrating the resultant under reduced pressure. The resultant residue was purified by column chromatography to obtain 23.9 g of the target product (yield 81%).

The $^1$H-NMR result of the resultant target product is shown below.

$^1$H-NMR (400 MHz, $CDCl_3$ ): δ4.42 (s, 2H), 7.25-7.39 (m, 2H), 8.36-8.39 (m, 2H).

(Step 2) Synthesis of 2-((4-bromobenzo[d]thiazol-6-yloxy)(5-fluoropyridin-2-yl)methyl)-1,3,4-oxadiazole)

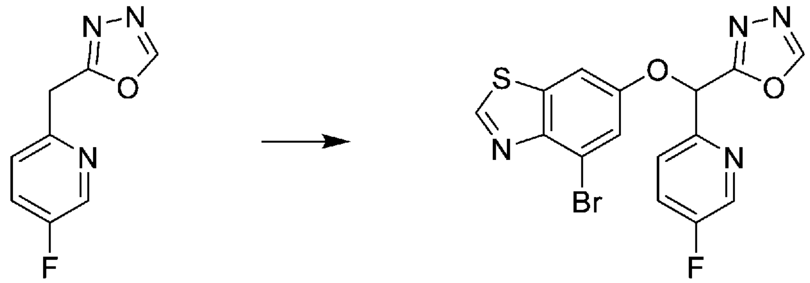

2-((5-Fluoropyridin-2-yl)methyl)-1,3,4-oxadiazole (1.50 g, 8.37 mmol) was dissolved in carbon tetrachloride (20 mL), and then N-bromosuccinimide (1.49 g, 8.37 mmol) and benzoyl peroxide (0.10 g, 0.41 mmol) were added thereto, followed by stirring the mixture while heating the mixture to reflux for 2 hours. The resultant was left to cool, and the reaction liquid was subjected to filtration with CELITE, and the resultant filtrate was concentrated under reduced pressure. An acetone solution (30 mL) of the resultant residue was added to 4-bromo-6-hydroxybenzothiazole (1.50 g, 6.52 mmol) and potassium carbonate (1.80 g, 13.02 mmol), followed by stirring the mixture at room temperature overnight. The reaction liquid was filtrated through CELITE, and the resultant filtrate was concentratesd under reduced pressure. The resultant residue was purified by silica gel chromatography to obtain 0.64 g of the target product (yield 24%).

The $^1$H-NMR result of the resultant target product is shown below.

$^1$H-NMR (400 MHz, $CDCl_3$ ): δ6.72 (s, 1H), 7.52-7.60 (m, 3H), 7.82 (dd, 1H), 8.43 (s, 1H), 8.46 (s, 1H), 8.94 (s, 1H).

Some of the compounds according to the present invention produced in the same way as mentioned in the examples shown above are shown in Tables 1 and 2. In Table 1, substituents in the compounds of formula (II) are indicated. In the table, property, melting point (m.p.) or refractive index ($n_D$) is shown as a physical property of each compound. In Table 2, the wavy line indicating a bond between N (nitrogen atom) and O (oxygen atom) (N-O undefined stereo bond) represents an E body, a Z body, or a mixture thereof, due to the nitrogen carbon double bond.

In the table, Me indicates a methyl group, Et indicates an ethyl group, $^n$ Pr indicates a normal propyl group, $^i$Pr indicates an isopropyl group, $^n$ Bu indicates a normal butyl group, $^t$ Bu indicates a tert-butyl group, $^n$ Hex indicates a normal hexyl group, $^c$ Pr indicates a cyclopropyl group, and Boc indicates a tert-butoxycarbonyl group.

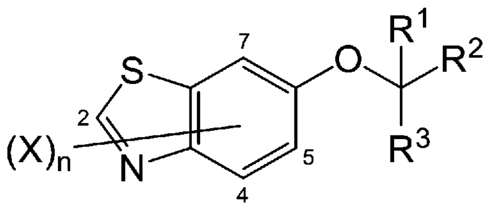

(II)

Table 1

| No. | $R^1$ | $R^2$ | $R^3$ | (X)n | Physical property |
|---|---|---|---|---|---|
| a-1 | H | 1,3,4-oxadiazol-2-yl | $^n$Bu | - | viscous oil |
| a-2 | H | 1,3,4-oxadiazol-2-yl | phenyl | 2-Me | amorphous |
| a-3 | H | 1,3,4-oxadiazol-2-yl | $^n$Bu | 2-Me | viscous oil |
| a-4 | H | 1,3,4-oxadiazol-2-yl | 4-F-phenyl | - | amorphous |
| a-5 | H | 1,3,4-oxadiazol-2-yl | pyridin-2-yl | - | amorphous |
| a-6 | H | 1,3,4-oxadiazol-2-yl | phenyl | 2-Cl | m.p.: 108-110°C |
| a-7 | H | 1,3,4-oxadiazol-2-yl | $^n$Bu | 2-Cl | viscous oil |
| a-8 | H | 1,3,4-oxadiazol-2-yl | 5-F-pyridin-2-yl | - | m.p.: 106-108°C |
| a-9 | H | 1,3,4-oxadiazol-2-yl | 5-Cl-pyridin-2-yl | | m,p,: 129-130°C |
| a-10 | H | 1,3,4-oxadiazot-2-yl | 5-Me-pyridin-2-yl | - | m.p.: 122-125°C |
| a-11 | H | 5-F-pyridm-2-yl | COOEt | - | m.p.: 105-106°C |
| a-12 | H | 1,3,4-oxadiazol-2-yl | SMe | - | m,p,: 100-102°C |
| a-13 | H | 1,3,4-oxadiazol-2-yl | Et | - | $n_D$(20.6°C) 1.5735 |
| a-14 | H | 1.3,4-oxadiazol-2-yl | phenyl | - | viscous oil |
| a-15 | H | 1.3,4-oxadiazol-2-yl | 5-Cl-pyrimidin-2-yl | - | amorphous |
| a-16 | H | 1.3.4-oxadiazo,1-2-yl | 5-F-pyrimidin-2-yl | - | m.p.: 155-161°C |
| a-17 | H | oxazol-2-yl | 5-F-pyridin-2-yl | | m.p.: 100-102°C |
| a-18 | H | 1,3,4-oxadiazol-2-yl | 5-F-pyridin-2-yl | 2-SMe | m.p.: 122-124°C |
| a-19 | H | 1,3,4-oxadiazol-2-yl | 5-F-pyridin-2-yl | 4-Me | viscous oil |
| a-20 | H | 1,3,4-oxadiazol-2-yl | 5-F-pyridin-2-yl | 4-Br | m.p.: 179-182°C |
| a-21 | H | 1,3,4-oxadiazol-2-yl | 5-F-pyridin-2-yl | 4-phenyl | amorphous |
| a-22 | H | 1,3,4-oxadiazol-2-yl | 5-F-pyridin-2-yl | 4-$^n$Pr | m.p.: 118-119°C |

(continued)

| No. | $R^1$ | $R^2$ | $R^3$ | (X)n | Physical property |
|---|---|---|---|---|---|
| a-23 | H | 1,3,4-oxadiazol-2-yl | 5-F-pyridin-2-yl | 4-(4-$CF_3$-phenyl) | m.p.: 172-176°C |
| a-24 | H | 1,3,4-oxadiazol-2-yl | 5-F-pyridin-2-yl | 4-(4-OMe-phenyl) | viscous oil |
| a-25 | H | 1,3,4-oxadiazol-2-yl | 5-F-pyridin-2-yl | 4-(3-$OCF_3$-phenyl) | viscous oil |
| a-26 | H | 1,3,4-oxadiazol-2-yl | 5-F-pyridin-2-yl | 4-$^n$Hex | m.p.: 87-89°C |
| a-27 | H | 1,3,4-oxadiazol-2-yl | 5-Cl- pyridin-2-yl | 4-phenyl | nD(20.4°C) 1.6142 |
| a-28 | H | 1,3,4-oxadiazol-2-yl | 5-Cl-pyridin-2-yl | 4-$^n$Pr | nD(20.4°C) 1.5945 |
| a-29 | H | 5-Cl-pyrimidin-2-yl | COOEt | 4-phenyl | m.p.: 96-100°C |
| a-30 | H | 1,3,4-oxadiazol-2-yl | 5-Cl-pyrimidin-2-yl | 4-phenyl | m.p,: 155-157°C |
| a-31 | H | 1,3,4-oxadiazol-2-yl | 5-F-pyrimidin-2-yl | 4-phenyl | amorphous |
| a-32 | H | 1,3,4-oxadiazol-2-yl | 5-F-pyridin-2-yl | 2-Me | m.p.: 110-113°C |
| a-33 | H | 1,3,4-oxadiazol-2-yl | 5-Cl-pyridin-2-yl | 2-Me | m.p.: 119-121°C |
| a-34 | H | 1,3,4-oxadiazol-2-yl | 4-Cl-phenyl | - | m.p,: 108-109°C |
| a-35 | H | 1,3,4-oxadiazol-2-yl | 5-Br-pyridin-2-yl | - | nD(20.3°C) 1.614 |
| a-36 | H | 1,3,4-oxadiazol-2-yl | 5-F-pyridin-2-yl | 2-$NH_2$ | m.p.: 188-192°C |
| a-37 | H | 1,3,4-oxadiazol-2-yl | 5-CN-pyridin-2-yl | - | m.p.: 174-180°C |
| a-38 | H | 1,3,4-oxadiazol-2-yl | 5-SMe-pyridin-2-yl | - | amorphous |
| a-39 | H | 1,3,4-oxadiazol-2-yl | 5-SOMe-pyridin-2-yl | - | viscous oil |
| a-40 | H | 1,3,4-oxadiazol-2-yl | 5-F-pyridin-2-yl | 2-Cl | m,p.: 140-143°C |
| a-41 | H | 1,3,4-oxadiazol-2-yl | 5-F-pyridin-2-yl | 2-F | m.p.: 165-168°C |
| a-42 | H | 1,3,4-oxadiazol-2-yl | 5-(1H-pyrazol-3-yl) pyridin-2-yl | - | amorphous |
| a-43 | H | 1,3,4-oxadiazol-2-yl | 5-F-pyridin-2-yl | 4-1 | m.p.: 196-201°C |
| a-44 | H | 1,3,4-oxadiazol-2-yl | 5-F-pyridin-2-yl | 5-F | m.p.: 128-131°C |
| a-45 | H | 1,3,4-oxadiazol-2-yl | 4-(prop-1-yn-1-yl) pyridin-2-yl | - | mp.: 121-123°C |

(continued)

| No. | $R^1$ | $R^2$ | $R^3$ | (X)n | Physical property |
|---|---|---|---|---|---|
| a-46 | H | 1,3,4-oxadiazol-2-yl | 5-F-pyridin-2-yl | 4-F | m.p.: 127-129°C |
| a-47 | H | 1,3,4-oxadiazol-2-yl | 5-Cl-pyridin-2-yl | 4-F | m,p,: 155-158°C |
| a-48 | H | 1,3,4-oxadiazol-2-yl | 5-F-pyridin-2-yl | 4-Cl | m.p,: 175-177°C |
| a-49 | H | 1,3,4-oxadiazol-2-yl | 5-F-pyridin-2-yl | 5-Cl | m.p.: 158-159°C |
| a-50 | H | 1,3,4-oxadiazol-2-yl | 5-Cl-pyridin-2-yl | 5-F | m.p.: 125-127°C |
| a-51 | H | 1,3,4-oxadiazol-2-yl | 5-Cl-pyridin-2-yl | 5-Cl | m,p,: 152-155°C |
| a-52 | H | 1,3,4-oxadiazol-2-yl | 5-Br-pyrazin-2-yl | 4-Cl | m.p.: 185-187°C |
| a-53 | H | 1,3,4-oxadiazol-2-yl | 2-Cl-thiazol-4-yl | - | viscous oil |
| a-54 | H | 1,3,4-oxadiazol-2-yl | 4-(prop-1-yn-1-yl) pyridin-2-yl | 4-F | m.p.: 162-164°C |
| a-55 | H | 1,3,4-oxadiazol-2-yl | 4-(prop-1-yn-1-yl) pyridin-2-yl | 4-Cl | m.p.: 149-152°C |
| a-56 | H | 1,3,4-oxadiazol-2-yl | 4-(prop-1-yn-1-yl) pyridin-2-yl | 5-F | m,p.: 151-153°C |
| a-57 | H | 1,3,4-oxadiazol-2-yl | 5-Br-pyrazin-2-yl | - | amorphous |
| a-58 | H | 1,3,4-oxadiazol-2-yl | 5-Me-pyrazin-2-yl | - | viscous oil |
| a-59 | H | 1,3,4-oxadiazol-2-yl | 4-{prop-1-yn-1-yl) pyridin-2-yl | 5-Cl | m,p,: 190-191°C |
| a-60 | H | 1,3,4-oxadiazol-2-yl | 5-Cl-4-(prop-l-yn-l-yl)pyridin-2-yl | - | m.p.: 183-185°C |
| a-61 | H | 1,3,4-oxadiazol-2-yl | 5-F-pyridin-2-yl | 4-vinyl | m.p.: 115-117°C |
| a-62 | H | oxadiazol-2-yl | 5-Cl-4-(3-OMe-prop-1-yn-1-yl)pyridin-2-yl | - | viscous oil |
| a-63 | H | 1,3,4-oxadiazol-2-yl | 5-Cl-4-ethynylpyridin-2-yl | - | m.p.: 167-170°C |
| a-64 | H | 1,3,4-oxadiazol-2-yl | 4-(3-OMe-prop-1-yn-1-yl)pyridin-2-yl | - | m.p.: 122-124°C |
| a-65 | H | 1,3,4-oxadiazol-2-yl | 5-F-pyridin-2-yl | 4-$CF_3$ | m.p.: 173-174°C |
| a-66 | H | 1,3,4-oxadiazal-2-yl | 5-F-pyndirin-2-yl | 2-CN | m,p,: 153-154°C |
| a-67 | H | 1,3,4-oxadiazol-2-yl | 5-F-pyridin-2-yl | 2-COOMe | m,p.: 205-207°C |
| a-68 | H | 1,3,4-oxadiazol-2-yl | 5-F-pyridin-2-yl | 4-Et | m.p.: 133-135°C |
| a-69 | H | 1,3,4-oxadiazol-2-yl | 4-Br-pyridin-2-yl | - | amorphous |

(continued)

| No. | $R^1$ | $R^2$ | $R^3$ | (X)n | Physical property |
|---|---|---|---|---|---|
| a-70 | H | 1,3,4-oxadiazol- 2-yl | 5-F-pyridin-2-yl | 4-$CH_2F$ | m.p.: 147-149°C |
| a-71 | H | 1,3,4-oxadiazol-2-yl | 4-$^n$Pr-pyridin-2-yl | - | viscous oil |
| a-72 | H | 1,3,4-oxadiazol-2-yl | 5-F-pyridin-2-yl | 4-$CHF_2$ | m.p.: 184-185°C |
| a-73 | H | 1,3,4-oxadiazol-2-yl | 4-OMe-pyridin-2-yl | - | viscous oil |
| a-74 | H | 1,3,4-oxadiazol-2-yl | 4-$CH_2$OMe-pyridin-2-yl | - | $n_D$(22.2°C) 1.5925 |
| a-75 | H | oxadiazol-2-yl | 4-$CH_2$OEt-pyridin-2-yl | - | $n_D$(19-5°C) 1.5881 |
| a-76 | H | 1,3,4-oxadiazol-2-yl | 4-$CH_2$SMe-pyridin-2-yl | - | viscous oil |
| a-77 | H | 1,3,4-oxadiazol-2-yl | 6-(prop-1-yn-1-yl) pyrimidin-4-yl | - | m.p.: 133-135°C |
| a-78 | H | 1,3,4-oxadiazol-2-yl | 4-ethynylpyridin-2-yl | - | m.p.: 121-123°C |
| a-79 | H | 1,3,4-oxadiazol-2-yl | 6-$^n$Pr-pyrimidin-4-yl | - | viscous oil |
| a-80 | H | 1,3,4-oxadiazol-2-yl | 4-phenyl-pyridin-2-yl | - | amorphous |
| a-81 | H | 1,3,4-oxadiazol-2-yl | 5-F-4-(prop-1-yn-1-yl)pyridin-2-yl | - | m.p.: 163-166°C |
| a-82 | H | 4-Br-pyridin-2-yl | COOMe | - | m.p.: 101-102°C |
| a-83 | H | 1,3,4-oxadiazol-2-yl | 4-(pyridin-2-yl) pyridin-2-yl | - | amorphous |
| a-84 | H | 1,3,4-oxadiazol-2-yl | 4-phenyl-pyridin-2-yl | 4-Br | viscous oil |
| a-85 | H | 1,3,4-oxadiazol-2-yl | 4-(thiophen-2-yl) pyridin-2-yl | - | viscous oil |
| a-86 | H | 1,3,4-oxadiazol-2-yl | 4-(pyridin-4-yl) pyridin-2-yl | - | amorphous |
| a-87 | H | 1,3,4-oxadiazol-2-yl | 4-phenyl-pyridin-2-yl | 4-$^n$Pr | m.p.: 127-129°C |
| a-88 | H | 1,3,4-oxadiazol-2-yl | 4-phenyl-pyridin-2-yl | 4-Et | viscous oil |
| a-89 | H | 1,3,4-oxadiazol-2-yl | 4-(CH($OMe)_2$)-5-F-pyridin-2-yl | - | m.p,: 127-129°C |
| a-90 | H | 1,3,4-oxadiazol-2-yl | 4-($COMe)_2$)pyridin-2-yl | - | amorphous |
| a-91 | H | 1,3,4-oxad lazo)-2-yl | 4-phenyl-pyridin-2-yl | 2-Me | amorphous |
| a-92 | H | 1,3,4-oxadiazol-2-yl | 4-phenyl-pyridin-2-yl | 2-OMe | amorphous |
| a-93 | H | 1,3,4-oxadiazol-2-yl | 5-F-4-((methoxymethoxy) methyl)pyridin-2-yl | - | m.p.: 67-69°C. |
| a-94 | H | 1,3,4-oxadiazol- 2-yl | 4-phenyl-5-F-pyridin-2-yl | - | nD(20.8°C) 1.6138 |
| a-95 | H | 1,3,4-oxadiazol-2-yl | 4-phenyl-5-F-pyridin-2-yl | 2-Me | nD(20.7°C) 1.6049 |
| a-96 | H | 1,3,4-oxadiazol-2-yl | 4-phenyl-5-F-pyridin-2-yl | 4-F | m.p,: 129-132°C |

(continued)

| No. | $R^1$ | $R^2$ | $R^3$ | (X)n | Physical property |
|---|---|---|---|---|---|
| a-97 | H | 1,3,4-oxadiazol-2-yl | 4-(pyridin-3-yl)pyridin-2-yl | - | nD(21,6°C) 1.6201 |
| a-98 | H | 1,3,4-oxadiazol-2-yl | 4-(4-F-phenyl)pyridiri-2-yl | - | m.p.: 148-150°C |
| a-99 | H | 1,3,4-oxadiazol-2-yl | 4-(3-F-phenyl)pyridin-2-yl | - | amorphous |
| a-100 | H | 1,3,4-oxadiazol-2-yl | 4-(2-F-phenyl)pyridin-2-yl | - | m.p.: 117-118°C |
| a-101 | H | 1,3,4-oxadiazol-2-yl | 4-(4-OMe-phenyl) pyridin-2-yl | - | nD(22.1°C) 1.6266 |
| a-102 | H | 1,3,4-oxadiazol-2-yl | 4-$O^nPr$-pyridin-2-yl | - | nD(20.7°C) 1.5937 |
| a-103 | H | 1,3,4-oxadiazol-2-yl | 4-phenyl pyrimidin-2-yl | - | viscous oil |
| a-104 | H | 1,3,4-oxadiazoi- 2-yl | 4-$CH_2$OTBS-5-F-pyridin-2-yl | - | nD(21.3°C) 1.5507 |
| a-105 | H | 1,3,4-oxadiazol-2-yl | 4-$CH_2$OH-5-F-pyridin-2-yl | - | amorphous |
| a-106 | Me | 1,3,4-oxadiazol-2-yl | 4-$CH_2$OH- 5-F-pyridin-2-yl | - | amorphous |
| a-107 | H | 1,3,4-oxadiazol-2-yl | 4-(1,3-dioxolan-2-yl) pyridin-2-yl | - | nD(21.4°C) 1.5924 |
| a-108 | H | 1,3,4-oxadiazol-2-yl | 4-(1,3-dioxan-2-yl) pyridin-2-yl | - | nD(20.7°C) 1.5720 |
| a-109 | H | 1,3,4-oxadiazol-2-yl | 3-Me-4-phenylpyridin-2-yl | - | m.p.: 180-183°C |
| a-110 | H | 1,3,4-oxadiazol-2-yl | 4-$CH_2$OMe-5-F-pyridin-2-yl | - | m.p.: 91-95°C |
| a-111 | H | 1,3,4-oxadiazol-2-yl | 4-$CH_2$OMe-5-F-pyridin-2-yl | 2-Me | m.p.: 142-146°C |
| a-112 | H | 1,3,4-oxadiazol-2-yl | 4-$CH_2$OMe-5-F-pyridin-2-yl | 2-Me-4-Et | m.p.: 96-101°C |
| a-113 | H | 1,3,4-oxadiazol-2-yl | 4-Cl-5-F-pyridin-2-yl | - | m.p.: 112-116°C |
| a-114 | H | 1.3.4-oxadiazol-2-yl | 5-fluoro-4-((methoxymethoxy) methyl) pyridin-2-yl | 2-Me | m.p.: 95-97°C |
| a-115 | H | 1,3,4-oxadiazol-2-yl | 5-F-4-(pyrimidin-5-yl) pyridin-2-yl | - | viscous oil |
| a-116 | H | 1,3,4-oxadiazol-2-yl | 5-F-4-(1-Me-1H-pyrazol-4-yl)pyridin-2-yl | - | viscous oil |
| a-117 | H | 1,3,4-oxadiazol-2-yl | 5-F-4-(1H-pyrazol-4-yl) pyridin-2-yl | - | amorphous |
| a-118 | H | 1,3,4-oxadiazol-2-yl | 4-COOMe-pyridin-2-yl | - | m.p.: 114-117°C |

(continued)

| No. | $R^1$ | $R^2$ | $R^3$ | (X)n | Physical property |
|---|---|---|---|---|---|
| a-119 | H | 1,3,4-oxadiazol-2-yl | 4-C(=O)NHMe-pyridin -2-yl | - | amorphous |
| a-120 | H | 1,3,4-oxadiazol-2-yl | 5-F-4-((1-isopropoxyethoxy) methyl)pyridin-2-yl | - | viscous oil |
| a-121 | H | 1,3,4-oxadiazol-2-yl | 5-F-4-(((2-methoxypropan-2-yl) oxy)methyl) pyridin-2-yl | - | viscous oil |
| a-122 | H | 1,3,4-oxadiazol-2-yl | 4-((1-ethoxyethoxy) methyl)-5-F-pyridin-2-yl | - | nD(20.7°C) 1.5531 |
| a-123 | H | 1,3,4-oxadiazol-2-yl | 5-F-4-((methoxymethoxy) methyl)pyridin-2-yl | 2-(prop-1-en-2-yl) | viscous oil |
| a-124 | H | 1,3,4-oxadiazol-2-yl | 5-F-4-((methoxymethoxy) methyl)pyridin-2-yl | 2-$^{i}$Pr | viscous oil |
| a-125 | H | 1,3,4-oxadiazol-2-yl | 5-F-4-((methoxymethoxy) methyl)pyridin-2-yl | 2-Me-4-Et | viscous oil |
| a-126 | H | 1,3,4-oxadiazol-2-yl | 5-F-4-((methoxymethoxy) methyl)pyridin-2-yl | 2-N(Boc)Me | viscous oil |
| a-127 | H | 1,3,4-oxadiazol-2-yl | 5-F-4-((methoxymethoxy) methyl)pyridin-2-yl | 2-NHMe | viscous oil |
| a-128 | H | 1,3,4-oxadiazol-2-yl | 4-(1,3-dioxolan-2-yl)-5-F-pyridin-2-yl | 2-Me | m.p.: 102-103°C |
| a-129 | H | 1,3,4-oxadiazol-2-yl | 4-(1,3-dioxolan-2-yl)-5-F-pyridin-2-yl | 2-Me-4-Et | m.p.: 99-100°C |
| a-130 | H | 1,3,4-oxadiazol-2-yl | 5-F-4-((methoxymethoxy) methyl)pyridin-2-yl | 2-$^{c}$Pr | viscous oil |
| a-131 | H | 1,3,4-oxadiazol-2-yl | 5-F-4-I-pyridin-2-yl | 2-Me | m.p.: 168-170°C |
| a-132 | H | 1,3,4-oxadiazol-2-yl | 5-F-4-((methoxymethoxy) methyl)pyridin-2-yl | 2-vinyl | viscous oil |
| a-133 | H | 1,3,4-oxadiazol-2-yl | 5-F-4-((methoxymethoxy) methyl)pyridin -2-yl | 2-Et | viscous oil |
| a-134 | H | 1,3,4-oxadiazol-2-yl | 5-F-4-(1-Me-1H-pyrazol-4-yl)pyridin-2-yl | 2-Me | amorphous |
| a-135 | H | 1,3,4-oxadiazol-2-yl | 5-F-4-(1-Me-1H-pyrazol-5-yl)pyridin-2-yl | 2-Me | m.p.: 188-189°C |
| a-136 | H | 1,3,4-oxadiazol-2-yl | 4-(6-OH-pyridin-3-yl)-pyridin-2-yl | 2-Me | m.p.: 216-217°C |
| a-137 | H | 1,3,4-oxadiazol-2-yl | 4-(1,3-dithiolan-2-yl)-5-F-pyridin-2-yl | - | nD(21.2°C) 1.6130 |
| a-138 | H | 1,3,4-oxadiazol-2-yl | 4-(1,3-dithiolan-2-yl)-5-F-pyridin-2-yl | 2-Me | nD(21.9°C) 1.6056 |
| a-139 | H | 1,3,4-oxadiazol-2-yl | 4-(1,3-dithiolan-2-yl)-5-F-pyridin-2-yl | 2-Me-4-Et | nD(21.2°C) 1.5991 |
| a-140 | H | 1,3,4-oxadiazol-2-yl | 4-(1,3-dioxolan-2-yl)-5-F-pyridin-2-yl | 2-N(Boc)Me | nD(24.3°C) 1.5587 |

(continued)

| No. | $R^1$ | $R^2$ | $R^3$ | (X)n | Physical property |
|---|---|---|---|---|---|
| a-141 | H | 1,3,4-oxadiazol-2-yl | 4-(1,3-dioxolan-2-yl)-5-F-pyridin-2-yl | 2-NHMe | amorphous |
| a-142 | H | 1,3,4-oxadiazol-2-yl | 4-(2-(1,3-dioxolan-2-yl) ethyl)-5-F-pyridin-2-yl | 2-Me | m.p.: 110-113°C |
| a-143 | H | 1,3,4-oxadiazol-2-yl | 5-F-4-(((methylthio)methoxy) methyl)pyridin -2-yl | 2-Me | viscous oil |
| a-144 | H | 1,3,4-oxadiazol-2-yl | 5-F-4-(((2-methoxyethoxy)methoxy) methyl) pyridin-2-yl | - | viscous oil |
| a-145 | H | 1,3,4-oxadiazol-2-yl | 5-F-4-(((2-methoxyethoxy)methoxy) methyl) pyridin-2-yl | 2-Me | viscous oil |
| a-146 | H | 1,3,4-oxadiazol-2-yl | 5-F-4-(((2-methoxyethoxy)methoxy) methyl) pyridin-2-yl | 4-OMe | viscous oil |
| a-147 | H | 1,3,4-oxadiazol-2-yl | 5-F-4-((methoxymethoxy) methyl)pyridin-2-yl | 4-OMe | viscous oil |
| a-148 | H | 1,3,4-oxad iazol-2-yl | 5-F-4-((methoxymethoxy) methyl)pyridin-2-yl | 4-NHMe | viscous oil |
| a-149 | H | 1,3,4-oxadiazol-2-yl | 5-F-4-((methoxymethoxy) methyl) pyridin -2-yl | 4-$NH_2$ | viscous oil |
| a-150 | H | 1,3,4-oxadiazol-2-yl | 4-((1-ethoxyethoxy) methyl)-5-F-pyridin-2-yl | 2-Me | $n_D$(23.5°C) 1,5600 |
| a-151 | H | 1,3,4-oxadiazol-2-yl | 5-F-4-((1-isopropoxyethoxy) methyl)pyridin-2-yl | 2-Me | $n_D$(23.7°C) 1.5467 |
| a-152 | H | 1,3,4-oxadiazol-2-yl | 5-F-4-(((2-methoxypropan-2-yl)oxy) methyl) pyridin-2-yl | 2-Me | $n_D$(24.1°C) 1.5531 |
| a-153 | H | 1,3,4-oxadiazol-2-yl | 5-F-4-(hydroxymethyl) pyridin-2-yl | 2-Me | m.p.: 177-178°C |
| a-154 | H | 1,3,4-oxadiazol-2-yl | 5-F-4-((methoxymethoxy) methyl) pyridin-2-yl | 2-COOMe | m.p.: 110-113°C |
| a-155 | H | 1,3,4-oxadiazol-2-yl | 5-F-4-((methoxymethoxy) methyl)pyridin-2-yl | 2-$CH_2$OH | viscous oil |
| a-156 | H | 1,3,4-oxadiazol-2-yl | 5-F-4-(hydroxymethyl) pyridin-2-yl | 2-$CH_2$OH | m.p.: 199-202°C |
| a-157 | H | 1,3,4-oxadiazol-2-yl | 4-(acetoxymethyl)-5 -F-pyridin-2-yl | 2-Me | $n_D$(24.8°C) 1.5790 |
| a-158 | H | 1,3,4-oxadiazol-2-yl | 5-F-4-((2-methoxyethoxy) methyl)pyridin-2-yl | 2-Me | $n_D$(20.0°C) 1.5606 |
| a-159 | H | 1,3,4-oxadiazol-2-yl | 5-F-4-((methoxymethoxy) methyl) pyridin-2-yl | 2-Me | viscous oil |
| a-160 | H | oxazol-5-yl | 5-F-4-((methoxymethoxy) methyl)pyridin-2-yl | 2-Me | viscous oil |
| a-161 | H | oxazol-2-yl | 5-F-4-((methoxymethoxy) methyl) pyridin-2-yl | 2-Me | viscous oil |
| a-162 | H | 1,3,4-oxadiazol-2-yl | 5-F-4-$^n$Pen-pyridin-2-yl | 2-Me | $n_D$(20.7°C) 1.5744 |

(continued)

| No. | $R^1$ | $R^2$ | $R^3$ | (X)n | Physical property |
|---|---|---|---|---|---|
| a-163 | H | 1,3,4-oxadiazol-2-yl | 5-F-4-(2-methoxyethoxy) pyridin-2-yl | 2-Me | m.p.: 107-109°C |
| a-164 | H | 5-Me-1,3,4-oxadiazol-2-yl | 5-F-4-(2-methoxyethoxy) pyridin-2-yl | 2-Me | viscous oil |
| a-165 | H | 1,3,4-oxadiazol-2-yl | 4-(1,3-dioxolan-2-yl)-5-F-pyridin-2-yl | - | $n_D$(20.8°C) 1.5771 |
| a-166 | H | 1,3,4-oxadiazol-2-yl | 4-(1,3-dioxolan-2-yl)-5-F-pyridin)-2-yl | 4-OMe | m.p.: 162-163°C |
| a-167 | H | 1,3,4-oxadiazol-2-yl | 5-F-4-((2-methoxyacetoxy)methyl) pyridin-2-yl | 2-Me | m.p.: 44-48°C |
| a-168 | H | 1,3,4-oxadiazol-2-yl | 5-F-4-(((methoxycarbonyl)oxy) methyl) pyridin-2-yl | 2-Me | viscous oil |
| a-169 | H | 1,3,4-oxadiazol-2-yl | 4-((methoxymethoxy) methyl)pyrimidin-2-yl | 2-Me | viscous oil |
| a-170 | H | 1,3,4-oxadiazol-2-yl | 4-(((dimethylcarbamoyl) oxy)methyl)-5-F-pyridin-2-yl | 2-Me | viscous oil |
| a-171 | H | 1,2,4-oxadiazol-2-yl | 5-F-4-((methoxymethoxy) methyl)pyridin-2-yl | 2-Me | viscous oil |
| a-172 | H | 1,3,4-oxadiazol-2-yl | 4-benzyl-5-F-pyridin-2-yl | 2-Me | $n_D$(20.1°C) 1.5955 |
| a-173 | H | 1,3,4-oxadiazol-2-yl | 4-$CH_2CH_2CH_2CN$-5 -F-pyridin-2-yl | 2-Me | $n_D$(20.0°C) 1.5741 |
| a-174 | H | 1,3,4-oxadiazol-2-yl | 5-F-4-(((3-methoxypropanoyl)oxy) methyl) pyridin-2-yl | 2-Me | viscous oil |
| a-175 | H | 1,3,4-oxadiazol-2-yl | 4-((methoxymethoxy) methyl)pyridin-2-yl | 2-Me | viscous oil |
| a-176 | H | 1,3,4-oxadiazol-2-yl | 5-F-4-CN-pyridin-2-yl | 2-Me | amorphous |
| a-177 | H | 1,3,4-oxadiazol-2-yl | 5-F-4-((methoxymethoxy) methyl)pyridin-2-yl | 2-$CH_2CH_2OMe$ | $n_D$(19.8°C) 1.5569 |
| a-178 | H | 1,3,4-oxadiazol-2-yl | 5-F-4-(furan-2-yl)pyridin-2-yl | 2-Me | m.p.: 146-147°C |
| a-179 | H | 1,3,4-oxadiazol-2-yl | 4-(3,6-dihydro-2H-pyran-4-yl)-5-F-pyridin-2-yl | 2-Me | m.p.: 133-134°C |
| a-180 | H | 1,3,4-oxadiazol-2-yl | 4-allyl-5-F-pyridin-2-yl | 2-Me | m.p.: 105-106°C |
| a-181 | H | 1,3,4-oxadiazol-2-yl | 5-F-4-(furan-3-yl)pyridin-2-yl | 2-Me | nD(20.5°C) 1.5895 |
| a-182 | H | 1,3,4-oxadiazol-2-yl | 4-(3,5-$Me_2$-isoxazol-4-yl)-5-F-pyridin-2-yl | 2-Me | m.p.: 156-157°C |
| a-183 | H | 1,3,4-oxadiazol-2-yl | 5-F-4-(6-OMe-pyridin-3-yl)pyridin-2-yl | 2-Me | amorphous |
| a-184 | H | 1,3,4-oxadiazol-2-yl | 5-F-4-(5-OMe-pyridin-3-yl)pyridin-2-yl | 2-Me | m.p.: 171-172°C |

(continued)

| No. | $R^1$ | $R^2$ | $R^3$ | (X)n | Physical property |
|---|---|---|---|---|---|
| a-185 | H | 1,3,4-oxadiazol-2-yl | 5-F-4-(2-OMe-pyrimidin-5-yl)pyridin-2-yl | 2-Me | m.p.: 157-158°C |
| a-186 | H | 1,3,4-oxadiazol-2-yl | 5-F-4-$^n$Pr-pyridin-2-yl | 2-Me | m.p.: 100-101°C |
| a-187 | H | 1,3,4-oxadiazol-2-yl | 5-F-4-(2-methoxyethyl)pyridin-2-yl | 2-Me | m.p.: 58-61°C |
| a-188 | H | 1,3,4-oxadiazol-2-yl | 5-F-4-(2-methoxyethyl)pyridin-2-yl | - | viscous oil |
| a-189 | H | 1,3,4-oxadiazol-2-yl | 5-F-4-(2-methoxyethyl)pyridin-2-yl | 2-$^c$Pr | viscous oil |
| a-190 | H | 1,3,4-oxadiazol-2-yl | 4-((methoxymethoxy) methyl)pyridin-2-yl | - | viscous oil |
| a-191 | H | 1,3,4-oxadiazol-2-yl | 4-((methoxymethoxy) methyl)pyridin- 2-yl | 2-$^c$Pr | m.p.: 109-111°C |
| a-192 | H | 1,3,4-oxadiazol-2-yl | 4-((methoxymethoxy) methyl)pyridin- 2-yl | 2-(prop-1-en-2-yl) | m.p.: 100-102°C |
| a-193 | H | 1,3,4-oxadiazol-2-yl | 4-((methoxymethoxy) methyl)pyridin-2-yl | 2-Me-4-Et | viscous oil |
| a-194 | H | 1,3,4-oxadiazol-2-yl | 4-((methoxymethoxy) methyl)pyridin-2-yl | 4-OMe | viscous oil |
| a-195 | H | 1,3,4-oxadiazol-2-yl | 4-$CH_2$OH-5-F-pyridin-2-yl | 2-$^c$Pr | m.p.: 127-128°C |

Table 2

| No. | Structure | Physical property |
|---|---|---|
| b-1 | 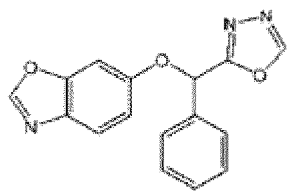 | m.p.: 113-115°C |
| b-2 | 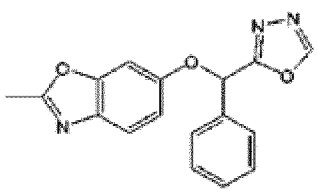 | m.p.: 94-96°C |
| b-3 | 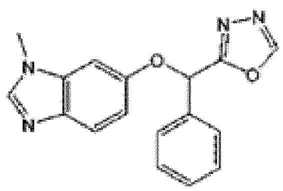 | m.p. : 114-116°C |

(continued)

| No. | Structure | Physical property |
|---|---|---|
| b-4 | 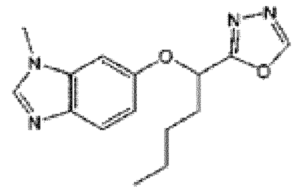 | amorphous |
| b-5 | 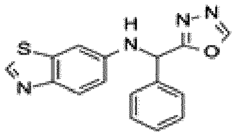 | amorphous |
| b-6 | 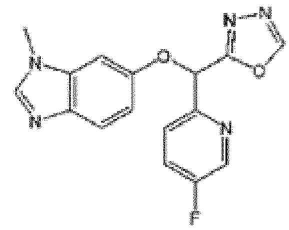 | viscous oil |
| b-7 | 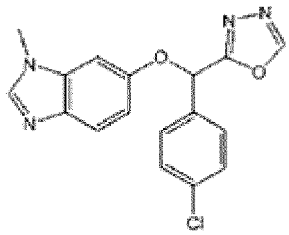 | viscous oil |
| b-8 | 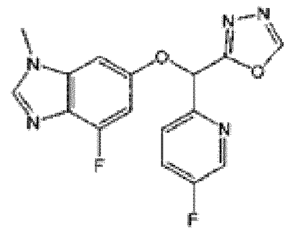 | viscous oil |
| b-9 | 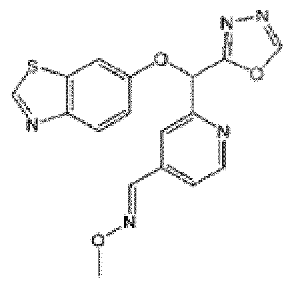 | viscous oil |

(continued)

| No. | Structure | Physical property |
|---|---|---|
| b-10 | 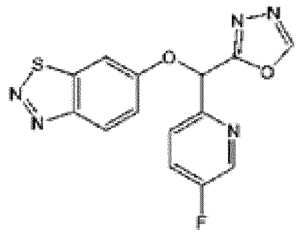 | m.p.: 141-142°C |
| b-11 | 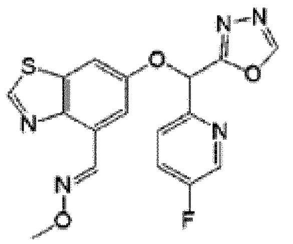 | m.p. : 165-167°C |
| b-12 | 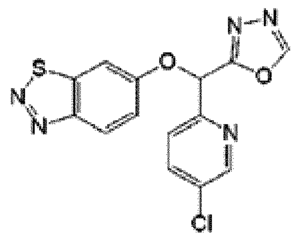 | m.p. : 133-134°C |
| b-13 | 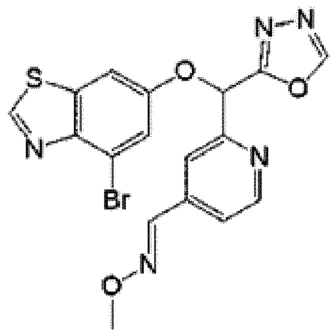 | amorphous |
| b-14 | 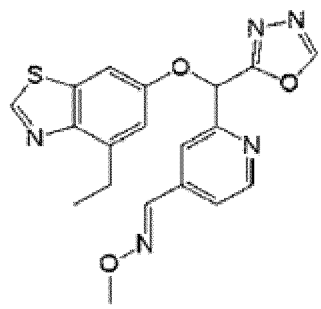 | viscous oil |

(continued)

| No. | Structure | Physical property |
|---|---|---|
| b-15 | 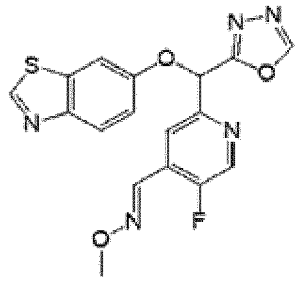 | m.p. : 136-138°C |
| b-16 | 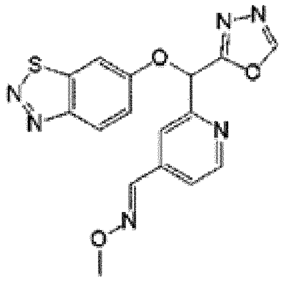 | m.p. : 172-173°C |
| b-17 | 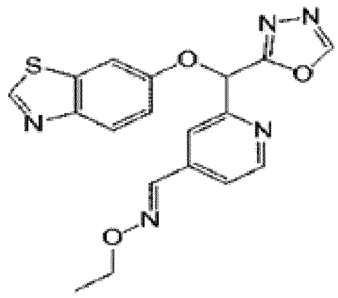 | m.p. : 113-114°C |
| b-18 | 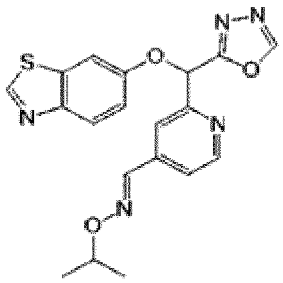 | viscous oil |
| b-19 | 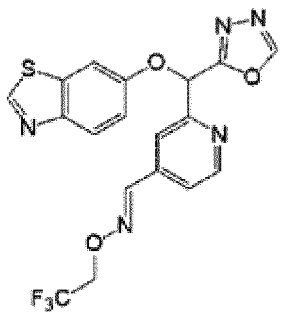 | m.p.: 128-130°C |

(continued)

| No. | Structure | Physical property |
|---|---|---|
| b-20 | 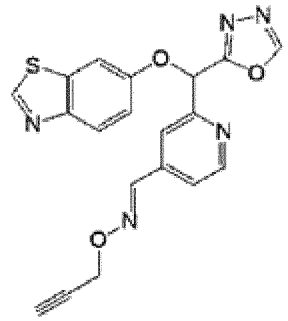 | viscous oil |
| b-21 | 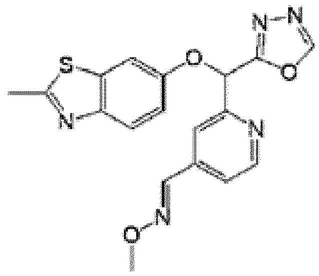 | m.p.: 107-109°C |
| b-22 | 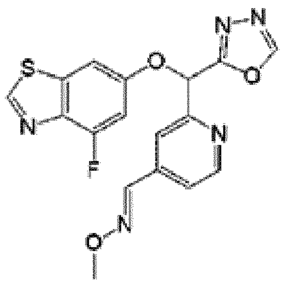 | m.p.: 146-148°C |
| b-23 | 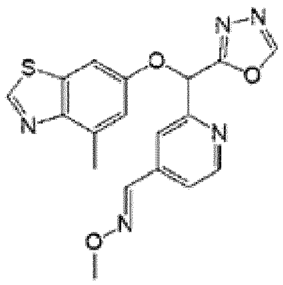 | m.p.: 146-148°C |
| b-24 | 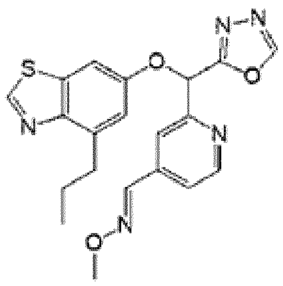 | m.p.: 80-82°C |

(continued)

| No. | Structure | Physical property |
| --- | --- | --- |
| b-25 | 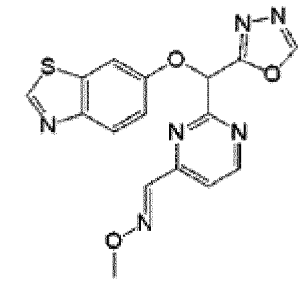 | viscous oil |
| b-26 | 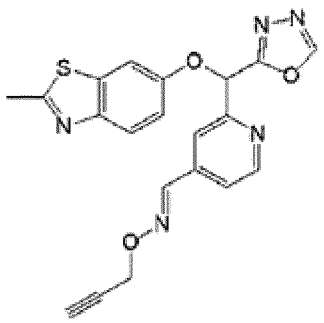 | m.p.: 118-121°C |
| b-27 | 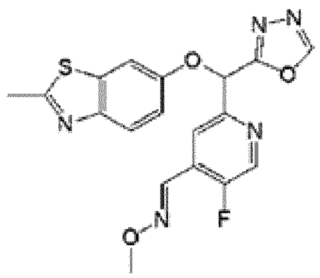 | m.p.: 129-131°C |
| b-28 | 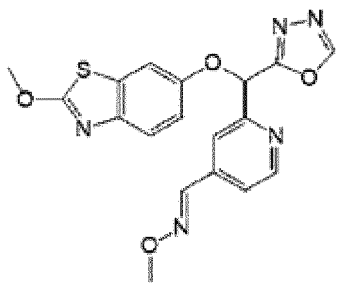 | amorphous |
| b-29 | 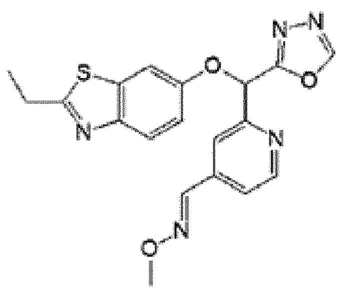 | viscous oil |

(continued)

| No. | Structure | Physical property |
|---|---|---|
| b-30 | 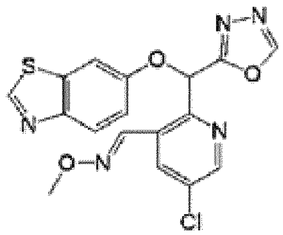 | m.p. : 216-220°C |
| b-31 | 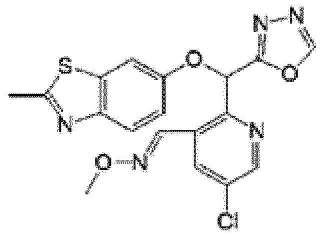 | m.p. : 198-201°C |
| b-32 | 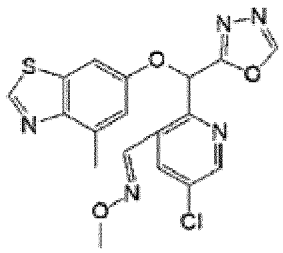 | m.p. : 122-124°C |
| b-33 | 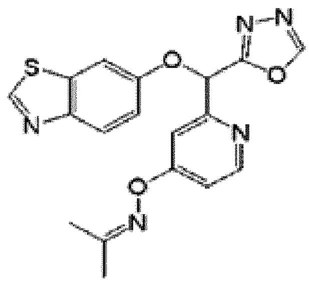 | amorphous |
| b-34 | 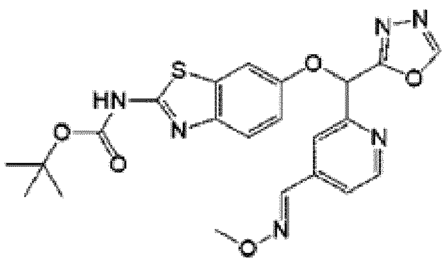 | viscous oil |
| b-35 | 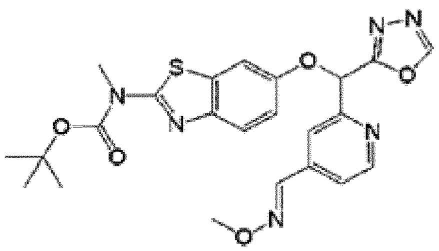 | viscous oil |

(continued)

| No. | Structure | Physical property |
|---|---|---|
| b-36 | 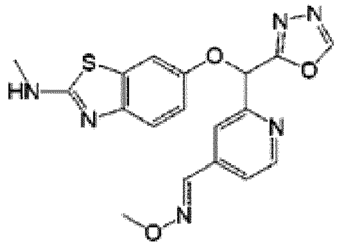 | viscous oil |
| b-37 | 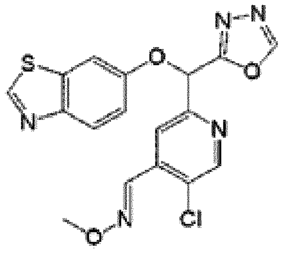 | amorphous |
| b-38 | 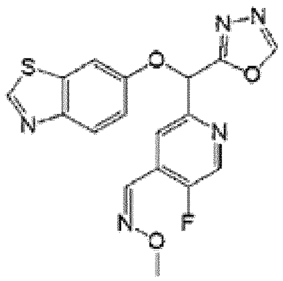 | amorphous |
| b-39 | 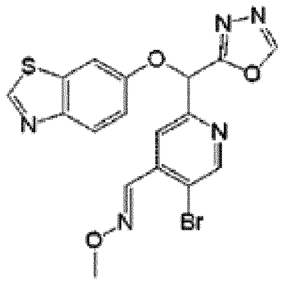 | $n_D$ (20.5°C) 1.6144 |
| b-40 | 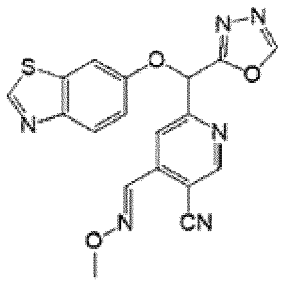 | m.p. : 129-131°C |

(continued)

| No. | Structure | Physical property |
|---|---|---|
| b-41 | 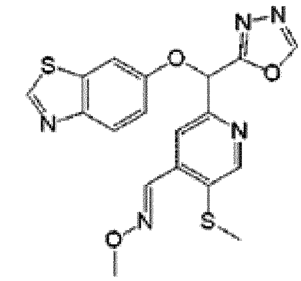 | viscous oil |
| b-42 | 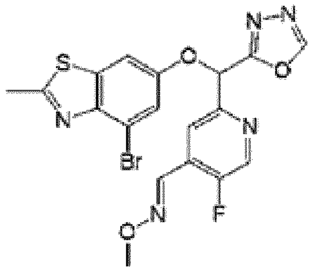 | m.p. : 70-75°C |
| b-43 | 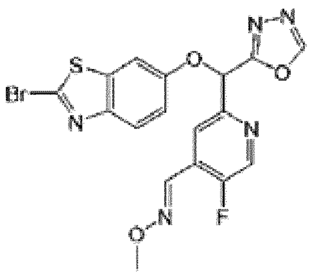 | m.p. : 151-154°C |
| b-44 | 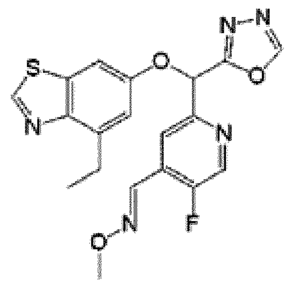 | m.p. : 114-117°C |
| b-45 | 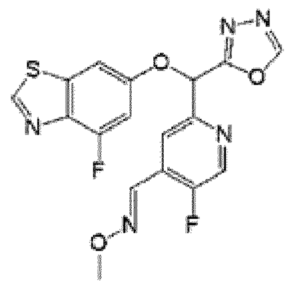 | m.p. : 137-139°C |

(continued)

| No. | Structure | Physical property |
|---|---|---|
| b-46 | 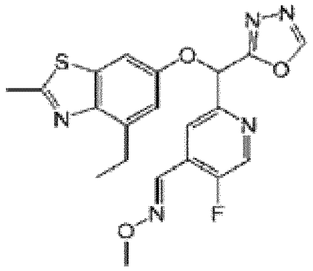 | m.p. : 145-147°C |
| b-47 | 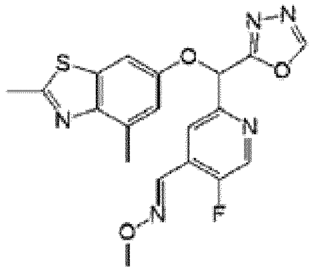 | m.p. : 151-154°C |
| b-48 | 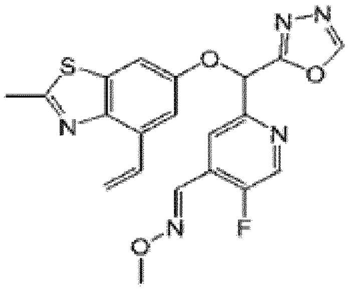 | m.p. : 116-119°C |
| b-49 | 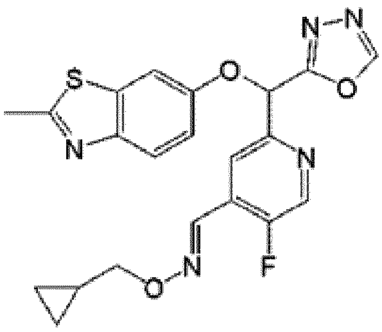 | viscous oil |
| b-50 | 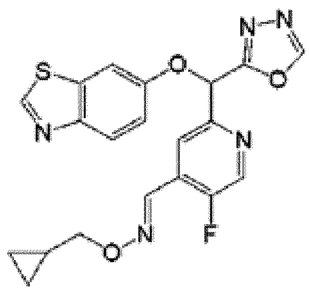 | m.p. : 129-131°C |

(continued)

| No. | Structure | Physical property |
|---|---|---|
| b-51 | 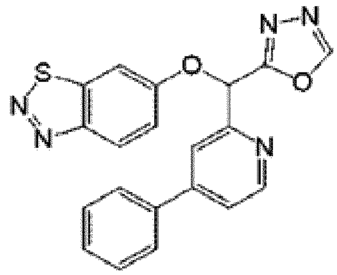 | amorphous |
| b-52 | 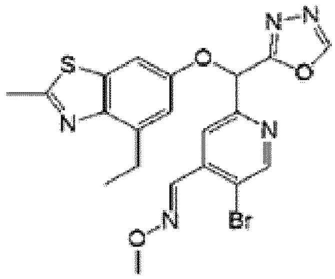 | m.p. : 160-161°C |
| b-53 | 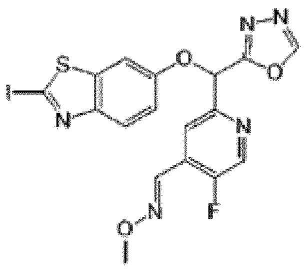 | m.p. : 166-168°C |
| b-54 | 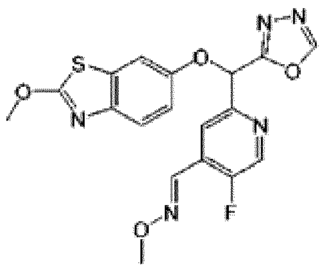 | m.p. : 134-136°C |
| b-55 | 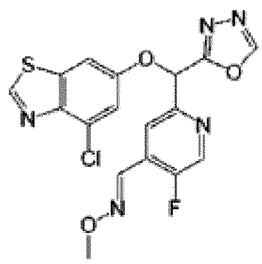 | m.p. : 153-154°C |

(continued)

| No. | Structure | Physical property |
| --- | --- | --- |
| b-56 | 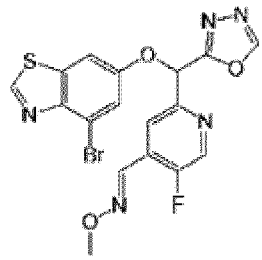 | m.p. : 170-172°C |
| b-57 | 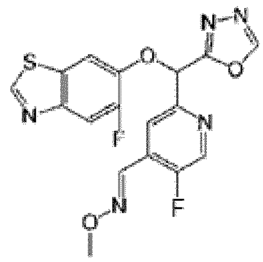 | m.p. : 150-151°C |
| b-58 | 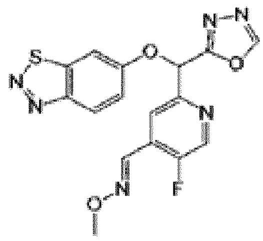 | m.p. : 134-135°C |
| b-59 | 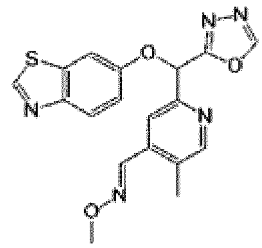 | viscous oil |
| b-60 | 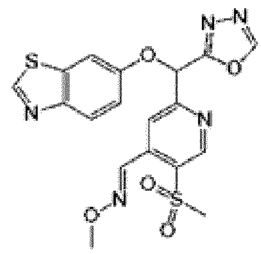 | m.p. : 90-93°C |
| b-61 | 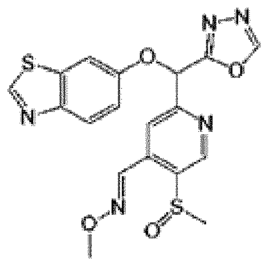 | m.p. : 85-88°C |

(continued)

| No. | Structure | Physical property |
|---|---|---|
| b-62 | 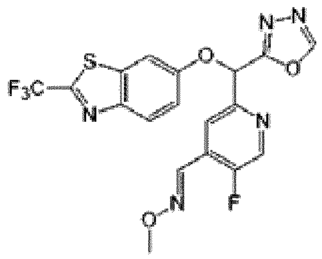 | m.p. : 113-114°C |
| b-63 | 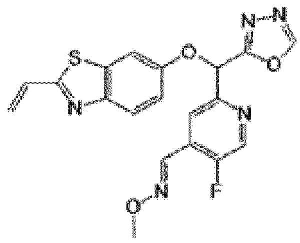 | m.p. : 88-89°C |
| b-64 | 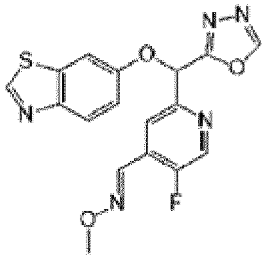 | m.p. : 176-178°C |
| b-65 | 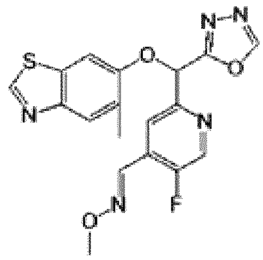 | m.p. : 179-180°C |
| b-66 | 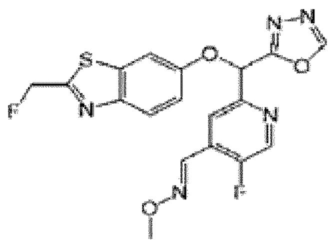 | m.p. : 133-134°C |
| b-67 | 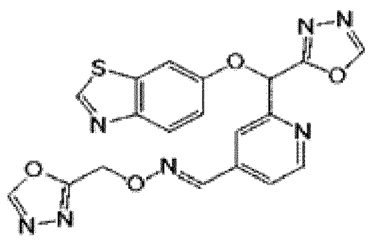 | amorphous |

(continued)

| No. | Structure | Physical property |
|---|---|---|
| b-68 | 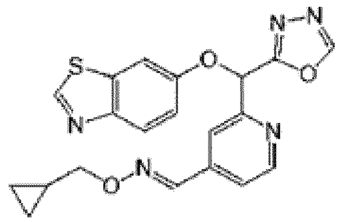 | $n_D$ (20.7°C) 1.6004 |
| b-69 | 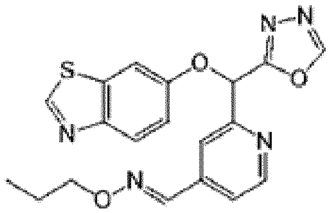 | $n_D$ (20.6°C) 1.6053 |
| b-70 | 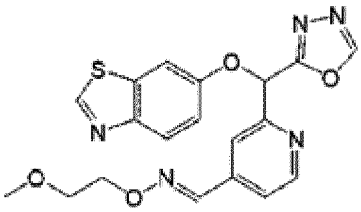 | amorphous |
| b-71 | 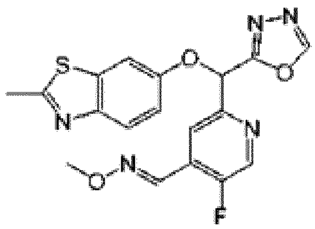 | m.p. : 167-168°C |
| b-72 | 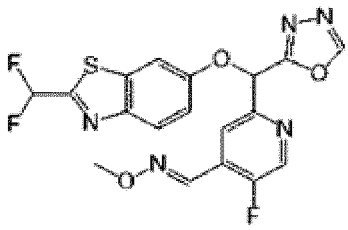 | m.p. : 143-144°C |
| b-73 | 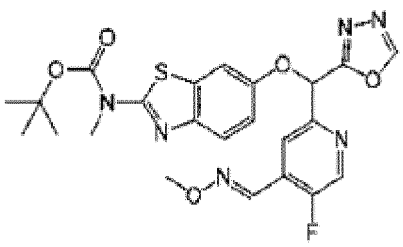 | m.p. : 157-158°C |

(continued)

| No. | Structure | Physical property |
|---|---|---|
| b-74 | 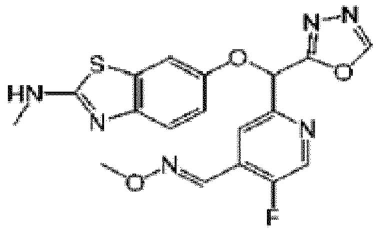 | m.p. : 181-182°C |
| b-75 | 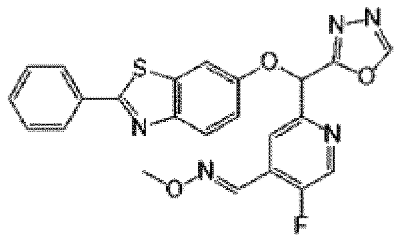 | m.p. : 182-183°C |
| b-76 | 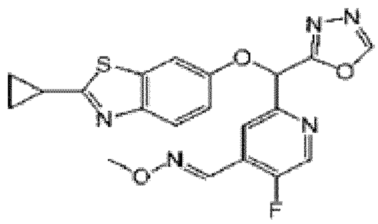 | m.p. : 103-104°C |
| b-77 | 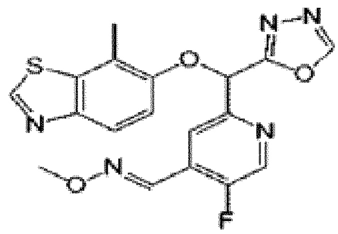 | m.p. : 108-110°C |
| b-78 | 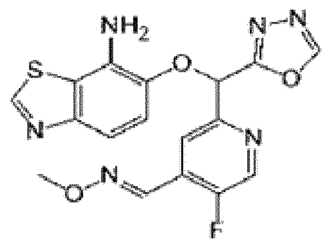 | $n_D$ (20.8°C) 1.6153 |
| b-79 | 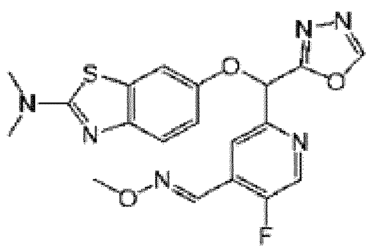 | m.p. : 103-104°C |

(continued)

| No. | Structure | Physical property |
|---|---|---|
| b-80 | 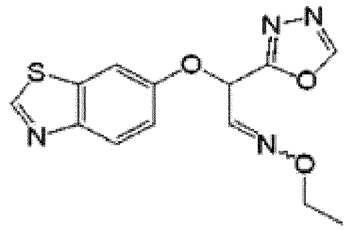 | $n_D$ (20.6°C) 1.5753 |
| b-81 | 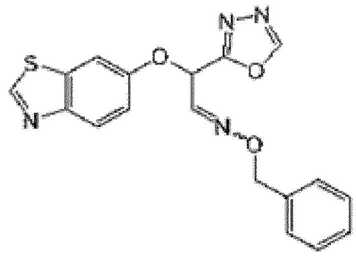 | $n_D$ (20.5°C) 1.6019 |
| b-82 | 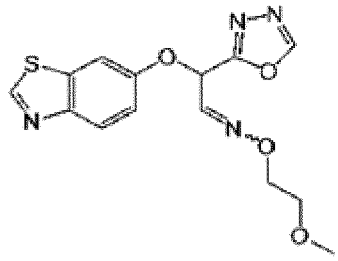 | $n_D$ (20.6°C) 1.5700 |
| b-83 | 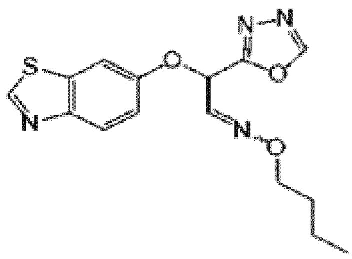 | $n_D$ (20.7°C) 1.5621 |
| b-84 | 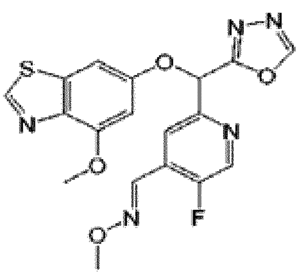 | m.p. : 103-104°C |
| b-85 | 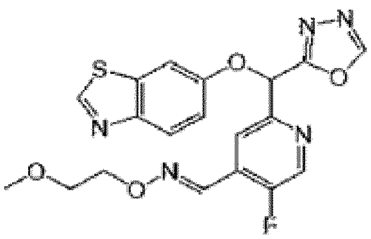 | $n_D$ (23.0°C) 1.5752 |

(continued)

| No. | Structure | Physical property |
|---|---|---|
| b-86 | 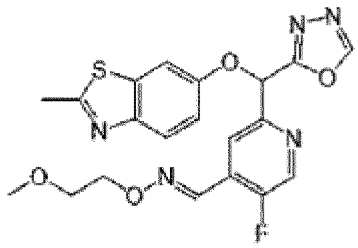 | $n_D$ (20.6°C) 1.5620 |
| b-87 | 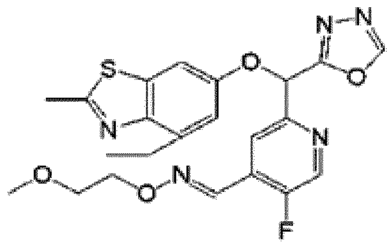 | m.p. : 138-139°C |
| b-88 | 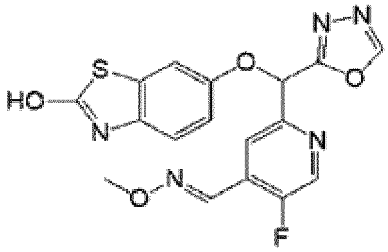 | m.p. : 197-198°C |
| b-89 | 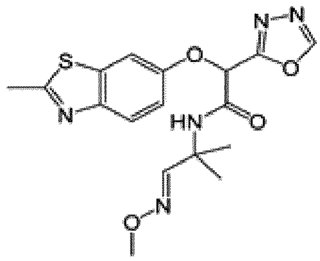 | m.p. : 143-145°C |
| b-90 | 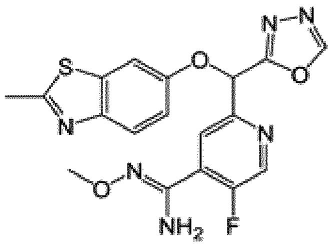 | m.p. : 116-121°C |
| b-91 | 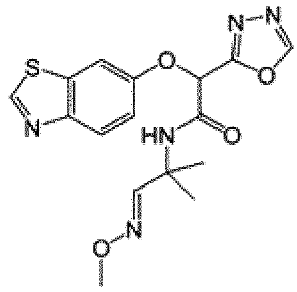 | m.p. : 129-130°C |

Among the compounds shown in Tables 1 and 2, the $^1$H-NMR data of the compounds having a viscous oil or amorphous physical property are shown below.

Compound No. (a-1): $^1$H-NMR (400MHz, $CDCl_3$): δ 8.84 (s, 1H), 8.38 (s, 1H), 7.99 (d, 1H), 7.52 (s, 1H), 7.18 (dd, 1H), 5.55 (dd, 1H), 2.20-2.31 (m, 1H), 2.01-2.13 (m, 1H), 1.50-1.61 (m, 1H), 1.31-1.46 (m, 3H), 0.92 (dd, 3H).
Compound No. (a-2): $^1$ H-NMR (400MHz, $CDCl_3$): δ 8.37 (s, 1H), 7.80 (d, 1H), 7.60 (dd, 2H), 7.33-7.47 (m, 4H), 7.15 (dd, 1H), 6.60 (s, 1H), 2.77 (s, 3H).
Compound No. (a-3): $^1$ H-NMR (400MHz, $CDCl_3$): δ 8.37 (s, 1H), 7.79 (s, 1H), 7.39 (d, 1H), 7.08 (dd, 1H), 5.51 (dd, 1H), 2.76 (s, 3H), 2.18-2.30 (m, 1H), 2.00-2.11 (m, 1H), 1.43-1.60 (m, 1H), 1.33-1.43 (m, 3H), 0.91 (dd, 3H).
Compound No. (a-4): $^1$ H-NMR (400MHz, $CDCl_3$): δ 8.87 (s, 1H), 8.41 (s, 1H), 8.02 (d, 1H), 7.62 (dd, 2H), 7.54 (d, 1H), 7.25 (dd, 1H), 7.08-7.19 (m, 2H), 6.63 (s, 1H).
Compound No. (a-5): $^1$ H-NMR (400MHz, $CDCl_3$): δ 8.87 (s, 1H), 8.62 (dd, 1H), 8.43 (s, 1H), 8.03 (d, 1H), 7.82 (dd, 2H), 7.60 (s, 1H), 7.21-7.38 (m, 2H), 6.76 (s, 1H).
Compound No. (a-7): $^1$H-NMR (400MHz, $CDCl_3$): δ 8.38 (s, 1H), 7.80 (d, 1H), 7.35 (d, 1H), 7.13 (d, 1H), 5.51 (dd, 1H), 2.19-2.30 (m, 1H), 2.00-2.12 (m, 1H), 1.45-1.60 (m, 1H), 1.32-1.43 (m, 3H), 0.91 (dd, 3H).
Compound No. (a-14): $^1$ H-NMR (400MHz, $CDCl_3$ ): δ 8.84 (s, 1H), 8.37 (s, 1H), 8.00 (d, 1H), 7.60 (dd, 1H), 7.54 (d, 1H), 7.33-7.45 (m, 3H), 7.19-7.25 (m, 2H), 6.64 (s, 1H).
Compound No. (a-15): $^1$ H-NMR (400MHz, $CDCl_3$): δ 8.88 (s, 1H), 8.80 (s, 2H), 8.50 (s, 1H), 8.03 (d, 1H), 7.61 (d, 1H), 7.29 (dd, 1H), 6.87 (s, 1H).
Compound No. (a-19): $^1$ H-NMR (400MHz, $CDCl_3$): δ 8.97 (s, 1H), 8.45 (d, 1H), 8.41 (s, 1H), 7.82 (dd, 1H), 7.48-7.54 (m, 1H), 7.38 (d, 1H), 7.08 (d, 1H), 6.72 (s, 1H), 2.72 (s, 3H).
Compound No. (a-21): $^1$ H-NMR (400MHz, $CDCl_3$ ): δ 8.89 (s, 1H), 8.45 (d, 2H), 7.85 (dd, 1H), 7.76 (dd, 2H), 7.32-7.58 (m, 6H), 6.98 (s, 1H).
Compound No. (a-24): $^1$ H-NMR (400MHz, $CDCl_3$ ): δ 8.87 (s, 1H), 8.43 (d, 2H), 7.85 (dd, 1H), 7.73 (dd, 2H), 7.48-7.59 (m, 2H), 7.31 (d, 1H), 7.01 (d, 2H), 6.78 (s, 1H), 3.85 (s, 3H).
Compound No. (a-25): $^1$ H-NMR (400MHz, $CDCl_3$ ): δ 8.90 (s, 1H), 8.45 (d, 2H), 7.85 (dd, 1H), 7.73 (dd, 1H), 7.46-7.68 (m, 4H), 7.35 (s, 1H), 7.26 (d, 1H), 6.80 (s, 1H).
Compound No. (a-31): $^1$ H-NMR (400MHz, $CDCl_3$): δ 6.95 (s, 1H), 7.38-7.45 (m, 2H), 7.46-7.53 (m, 2H), 7.60 (d, 1H), 7.74-7.81 (m, 2H), 8.50 (s, 1H), 8.71 (s, 2H), 8.91 (s, 1H).
Compound No. (a-38): $^1$ H-NMR (400MHz, $CDCl_3$): δ 8.85 (s, 1H), 8.45 (d, 2H), 8.01 (d, 1H), 7.69 (d, 1H), 7.61 (dd, 1H), 7.25 (dd, 2H), 6.69 (s, 1H), 2.49 (s, 3H).
Compound No. (a-39): $^1$ H-NMR (400MHz, $CDCl_3$): δ 8.85 (s, 1H), 8.73 (d, 2H), 8.42 (s, 1H), 8.16 (dd, 1H), 7.97-8.05 (m, 1H), 7.57 (s, 1H), 7.20-7.29 (m, 1H), 6.80 (s, 1H), 2.79 (s, 3H).
Compound No. (a-42): $^1$ H-NMR (400MHz, $CDCl_3$): δ 10.58 (s, 1H), 9.03 (d, 1H), 8.85 (s, 1H), 8.42 (s, 1H), 8.20 (dd, 1H), 8.02 (d, 1H), 7.83 (d, 1H), 7.60 (dd, 2H), 7.29 (dd, 1H), 6.78 (s, 1H), 6.68 (s, 1H).
Compound No. (a-53): $^1$ H-NMR (400MHz, $CDCl_3$): δ 8.87 (s, 1H), 8.44 (s, 1H), 8.02 (d, 1H), 7.57 (dd, 2H), 7.24 (dd, 1H), 6.75 (s, 1H).
Compound No. (a-57): $^1$ H-NMR (400MHz, $CDCl_3$): δ 6.79 (s, 1H), 7.28 (dd, 1H), 7.64 (d, 1H), 8.06 (d, 1H), 8.46 (s, 1H), 8.66 (dd, 1H), 8.90 (d, 1H), 8.91 (s, 1H).
Compound No. (a-58): $^1$ H-NMR (400MHz, $CDCl_3$): δ 2.62 (s, 3H), 6.80 (s, 1H), 7.29 (dd, 1H), 7.67 (d, 1H), 8.04 (d, 1H), 8.45 (d, 1H), 8.47 (s, 1H), 8.89 (d, 1H), 8.97 (d, 1H).
Compound No. (a-62): $^1$ H-NMR (400MHz, $CDCl_3$): δ 8.89 (s, 1H), 8.60 (s, 1H), 8.43 (s, 1H), 8.05 (d, 1H), 7.88 (s, 1H), 7.60 (d, 1H), 7.28 (dd, 1H), 6.71 (s, 1H), 4.40 (s, 2H), 3.84 (s, 3H).
Compound No. (a-69): $^1$ H-NMR (400MHz, $CDCl_3$): δ 6.74 (s, 1H), 7.29 (dd, 1H), 7.51 (dd, 1H), 7.62 (d, 1H), 8.03 (d, 1H), 8.07 (s, 1H), 8.42 (s, 1H), 8.43 (s, 1H), 8.89 (s, 1H).
Compound No. (a-71): $^1$H-NMR (400MHz, $CDCl_3$): δ 0.92 (t, 3H), 1.63-1.72 (m, 2H), 2.62 (t, 2H), 6.74 (s, 1H), 7.14 (dd, 1H), 7.28 (dd, 1H), 7.61 (d, 2H), 8.02 (d, 1H), 8.43 (s, 1H), 8.48 (d, 1H), 8.87 (s, 1H).
Compound No. (a-73): $^1$ H-NMR (400MHz, $CDCl_3$): δ 3.89 (s, 3H), 6.70 (s, 1H), 6.82 (dd, 1H), 7.28 (dd, 1H), 7.33 (d, 1H), 7.60 (d, 1H), 8.03 (d, 1H), 8.43 (s, 1H), 8.44 (s, 1H), 8.87 (s, 1H).
Compound No. (a-76): $^1$ H-NMR (400MHz, $CDCl_3$ ):δ 1.99 (s, 3H), 3.66 (s, 2H), 6.74 (s, 1H), 7.24-7.30 (m, 2H), 7.59 (s, 1H), 7.73 (s, 1H), 8.00 (d, 1H), 8.41 (s, 1H), 8.53 (d, 1H), 8.85 (s, 1H).
Compound No. (a-79): $^1$ H-NMR (400MHz, $CDCl_3$): δ 0.99 (t, 3H), 1.76-1.86 (m, 2H), 2.83 (t, 2H), 6.68 (s, 1H), 7.29-7.31 (m, 1H), 7.63 (m, 1H), 7.71 (s, 1H), 8.06 (d, 1H), 8.44 (s, 1H), 8.90 (s, 1H), 9.11 (s, 1H).
Compound No. (a-80): $^1$ H-NMR (400MHz, $CDCl_3$): δ 6.82 (s, 1H), 7.33 (dd, 1H), 7.46-7.52 (m, 3H), 7.55 (dd, 1H), 7.65-7.66 (m, 2H), 7.67 (m, 1H), 8.04 (d, 2H), 8.44 (s, 1H), 8.66-8.67 (m, 1H), 8.88 (s, 1H).
Compound No. (a-83): $^1$ H-NMR (400MHz, $CDCl_3$ ):δ 6.85 (s, 1H), 7.34-7.39 (m, 2H), 7.66 (d, 1H), 7.82-7.86 (m, 2H), 7.95-7.97 (m, 1H), 8.04 (d, 1H), 8.44 (s, 2H), 8.73 (d, 1H), 8.74-8.77 (m, 1H), 8.87 (s, 1H).
Compound No. (a-84): $^1$ H-NMR (400MHz, $CDCl_3$): δ 6.78 (s, 1H), 7.40-7.68 (m, 8H), 7.99 (s, 1H), 8.44 (s, 1H),

8.65 (d, 1H), 8.92 (s, 1H).

Compound No. (a-85): $^1$ H-NMR (400MHz, $CDCl_3$): δ 6.78 (s, 1H), 7.14-7.16 (m, 1H), 7.32-7.35 (m, 1H), 7.44-7.46 (m, 1H), 7.50-7.52 (m, 1H), 7.56-7.57 (m, 1H), 7.65 (d, 1H), 7.99 (s, 1H), 8.05 (d, 1H), 8.45 (s, 1H), 8.57-8.59 (m, 1H), 8.88 (s, 1H).

Compound No. (a-86): $^1$ H-NMR (400MHz, $CDCl_3$): δ 6.84 (s, 1H), 7.33 (dd, 1H), 7.46-7.49 (m, 1H), 7.54-7.59 (m, 2H), 7.65-7.70 (m, 2H), 8.05-8.08 (m, 2H), 8.45 (s, 1H), 8.74-8.78 (m, 2H), 8.89 (s, 1H).

Compound No. (a-88): $^1$ H-NMR (400MHz, $CDCl_3$): δ 1.35 (t, 3H), 3.15 (q, 2H), 6.80 (s, 1H), 7.15 (s, 1H), 7.38-7.57 (m, 6H), 7.64 (d, 1H), 8.02 (s, 1H), 8.43 (s, 1H), 8.65 (d, 1H), 8.83 (s, 1H).

Compound No. (a-90): $^1$ H-NMR (400MHz, $CDCl_3$): δ 8.87 (s, 1H), 8.62 (d, 1H), 8.43 (s, 1H), 8.03 (d, 1H), 7.90 (s, 1H), 7.62 (d, 1H), 7.44-7.40 (m, 1H), 7.33-7.29 (m, 1H), 6.78 (s, 1H), 5.44 (s, 1H), 3.33 (s, 3H), 3.31 (s, 3H).

Compound No. (a-183): $^1$ H-NMR (400MHz, $CDCl_3$): δ 8.51 (1H, d), 8.47-8.47 (1H, m), 8.44 (1H, s), 7.91 (1H, d), 7.87-7.84 (2H, m), 7.50 (1H, d), 7.20 (1H, dd), 6.87 (1H, d), 6.75 (1H, s), 4.00 (3H, s), 2.79 (3H, s).

Compound No. (a-188): $^1$ H-NMR (400MHz, $CDCl_3$): δ 8.88 (1H, s), 8.44 (1H, s), 8.37 (1H, d), 8.04 (1H, d), 7.75 (1H, d), 7.61 (1H, d), 7.30-7.27 (1H, m), 6.73 (1H, s), 3.64 (2H, td), 3.30 (3H, s), 2.98 (2H, t).

Compound No. (a-189): $^1$H-NMR (400MHz, $CDCl_3$): δ 8.42 (1H, s), 8.36 (1H, s), 7.77 (1H, d), 7.73 (1H, d), 7.45 (1H, d), 7.17 (1H, dd), 6.67 (1H, s), 3.64 (2H, td), 3.31 (3H, s), 2.97 (2H, t), 2.35-2.33 (1H, m), 1.22-1.18 (4H, m).

Compound No. (a-190): $^1$H-NMR (400MHz, $CDCl_3$): δ 8.88 (1H, s), 8.58 (1H, d), 8.43 (1H, s), 8.04 (1H, d), 7.80 (1H, s), 7.62 (1H, d), 7.33 (1H, d), 7.30 (1H, dd), 6.76 (1H, s), 4.74 (2H, s), 4.67 (2H, s), 4.03 (0H, d), 3.39 (3H, s).

Compound No. (a-193): $^1$H-NMR (400MHz, $CDCl_3$): δ 8.57 (1H, d), 8.41 (1H, s), 7.79 (1H, s), 7.32 (1H, d), 7.30 (1H, d), 7.05 (1H, d), 6.72 (1H, s), 4.74 (2H, s), 4.67 (2H, s), 3.39 (3H, s), 3.09 (2H, q), 2.79 (3H, s), 1.33 (3H, t).

Compound No. (a-194): $^1$H-NMR (400MHz, $CDCl_3$): δ 8.77 (1H, s), 8.59 (1H, d), 8.43 (1H, s), 7.78 (1H, s), 7.33 (1H, d), 7.13 (1H, d), 6.77 (1H, d), 6.76 (1H, s), 4.74 (2H, s), 4.67 (2H, s), 4.03 (3H, s), 3.39 (3H, s).

Compound No. (b-5): $^1$ H-NMR (400MHz, $CDCl_3$): δ 8.68 (d, 1H), 8.35 (s, 1H), 7.31-7.55 (m, 5H), 7.08 (d, 1H), 6.91 (dd, 1H), 5.95 (d, 1H), 5.05 (d, 1H).

Compound No. (b-6): $^1$ H-NMR (400MHz, $CDCl_3$ ):δ 3.76 (s, 3H), 6.75 (s, 1H), 7.01 (d, 1H), 7.04-7.05 (m, 1H), 7.50-7.57 (m, 1H), 7.66 (d, 1H), 7.78 (s, 1H), 7.85 (dd, 1H), 8.41 (s, 1H), 8.45 (d, 1H).

Compound No. (b-7): $^1$ H-NMR (400MHz, $CDCl_3$ ): δ 3.75 (s, 3H), 6.61 (s, 1H), 6.99 (s, 1H), 7.00 (d, 1H), 7.36-7.45 (m, 2H), 7.55 (s, 1H), 7.57 (s, 1H), 7.65 (d, 1H), 7.78 (s, 1H), 8.38 (s, 1H).

Compound No. (b-8): $^1$ H-NMR (400MHz, $CDCl_3$): δ 3.76 (s, 3H), 6.72 (s, 1H), 6.76 (dd, 1H), 6.85 (d, 1H), 7.50-7.57 (m, 1H), 7.76 (s, 1H), 7.82 (dd, 1H), 8.42 (s, 1H), 8.45 (d, 1H).

Compound No. (b-13): $^1$ H-NMR (400MHz, $CDCl_3$): δ 4.04 (s, 3H), 6.74 (s, 1H), 7.50-7.52 (m, 1H), 7.57 (dd, 2H), 7.95 (s, 1H), 8.05 (s, 1H), 8.44 (s, 1H), 8.61 (d, 1H), 8.95 (s, 1H).

Compound No. (b-14): $^1$ H-NMR (400MHz, $CDCl_3$): δ 1.24 (t, 3H), 3.14 (q, 2H), 4.03 (s, 3H), 6.76 (s, 1H), 7.15 (d, 1H), 7.43 (d, 1H), 7.49 (dd, 1H), 7.97 (s, 1H), 8.04 (s, 1H), 8.43 (s, 1H), 8.61 (d, 1H), 8.85 (s, 1H).

Compound No. (b-18): $^1$ H-NMR (400MHz, $CDCl_3$): δ 1.25-1.35 (m, 6H), 4.45-4.53 (m, 1H), 6.75 (s, 1H), 7.29 (dd, 1H), 7.48 (d, 1H), 7.60 (s, 1H), 7.93 (dd, 1H), 8.01 (s, 1H), 8.05 (s, 1H), 8.42 (s, 1H), 8.58 (d, 1H), 8.86 (s, 1H).

Compound No. (b-20): $^1$ H-NMR (400MHz, $CDCl_3$): δ 2.51 (s, 1H), 4.81 (s, 2H), 6.76 (s, 1H), 7.28 (dd, 1H), 7.51 (d, 1H), 7.61 (s, 1H), 7.98 (s, 1H), 8.03 (d, 1H), 8.10 (s, 1H), 8.42 (s, 1H), 8.62 (d, 1H), 8.87 (s, 1H).

Compound No. (b-25): $^1$ H-NMR (400MHz, $CDCl_3$): δ 4.03 (s, 3H), 6.83 (s, 1H), 7.30 (d, 1H), 7.60 (s, 1H), 7.78 (d, 1H), 7.98 (d, 1H), 8.03 (s, 1H), 8.47 (s, 1H), 8.77 (d, 1H), 8.85 (s, 1H).

Compound No. (b-28): $^1$ H-NMR (400MHz, $CDCl_3$): δ 4.01 (s, 3H), 4.14 (s, 3H), 6.66 (s, 1H), 7.10 (d, 1H), 7.33 (s, 1H), 7.45 (d, 1H), 7.56 (d, 1H), 7.94 (s, 1H), 8.02 (s, 1H), 8.41 (s, 1H), 8.58 (d, 1H).

Compound No. (b-29): $^1$ H-NMR (400MHz, $CDCl_3$): δ 1.42 (t, 3H), 3.07 (q, 2H), 4.01 (s, 3H), 6.71 (s, 1H), 7.19 (d, 1H), 7.46 (d, 1H), 7.49 (s, 1H), 7.83 (d, 1H), 7.93 (s, 1H), 8.02 (s, 1H), 8.41 (s, 1H), 8.58 (d, 1H).

Compound No. (b-33): $^1$ H-NMR (400MHz, $CDCl_3$): δ 2.05 (s, 3H), 2.08 (s, 3H), 6.71 (s, 1H), 7.14 (dd, 1H), 7.31 (dd, 1H), 7.59-7.62 (m, 2H), 8.03 (d, 1H), 8.43-8.44 (m, 2H), 8.87 (s, 1H).

Compound No. (b-34): $^1$ H-NMR (400MHz, $CDCl_3$): δ 1.55 (s, 9H), 4.01 (s, 3H), 6.71 (s, 1H), 7.13 (d, 1H), 7.44 (s, 1H), 7.45 (d, 1H), 7.73 (d, 1H), 7.95 (s, 1H), 8.02 (s, 1H), 8.41 (s, 1H), 8.59 (d, 1H).

Compound No. (b-35): $^1$ H-NMR (400MHz, $CDCl_3$ ):δ 1.57 (s, 9H), 3.58 (s, 3H), 4.00 (s, 3H), 6.69 (s, 1H), 7.13 (d, 1H), 7.40 (s, 1H), 7.42 (d, 1H), 7.65 (d, 1H), 7.94 (s, 1H), 8.01 (s, 1H), 8.41 (s, 1H), 8.56 (d, 1H).

Compound No. (b-36): $^1$ H-NMR (400MHz, $CDCl_3$): δ 3.06 (s, 3H), 4.01 (s, 3H), 5.31 (br, 1H), 6.64 (s, 1H), 7.04 (d, 1H), 7.30 (s, 1H), 7.42 (d, 1H), 7.46 (d, 1H), 7.95 (s, 1H), 8.02 (s, 1H), 8.40 (s, 1H), 8.57 (d, 1H).

Compound No. (b-37): $^1$ H-NMR (400MHz, $CDCl_3$): δ 4.06 (s, 3H), 6.72 (s, 1H), 7.29 (d, 1H), 7.61 (s, 1H), 8.03 (d, 1H), 8.25 (s, 1H), 8.38 (s, 1H), 8.43 (s, 1H), 8.57 (s, 1H), 8.87 (s, 1H).

Compound No. (b-38): $^1$ H-NMR (400MHz, $CDCl_3$): δ 8.89 (1H, s), 8.59-8.58 (1H, m), 8.54 (1H, m), 8.45 (1H, s), 8.05 (1H, d), 7.62-7.61 (2H, m), 7.30-7.28 (1H, m), 6.76 (1H, s), 4.06 (3H, s).

Compound No. (b-41): $^1$ H-NMR (400MHz, $CDCl_3$): δ 8.88 (1H, s), 8.54 (1H, s), 8.44 (1H, s), 8.43 (1H, s), 8.08 (1H, s), 8.04 (1H, d), 7.62 (1H, d), 7.31 (1H, dd), 6.73 (1H, s), 4.05 (3H, s), 2.53 (3H, s).

Compound No. (b-49): $^1$ H-NMR (400MHz, $CDCl_3$): δ 8.89 (1H, s), 8.49-8.48 (1H, m), 8.44 (1H, s), 8.30 (1H, s), 8.22-8.21 (1H, m), 8.05 (1H, d), 7.63-7.62 (1H, m), 7.32-7.29 (1H, m), 6.75 (1H, s), 4.11-4.07 (2H, m), 1.26-1.19 (1H, m), 0.62-0.59 (2H, m), 0.36-0.32 (2H, m).

Compound No. (b-51): $^1$ H-NMR (400MHz, $CDCl_3$): δ 8.69-8.66 (m, 1H), 8.53 (d, 1H), 8.46 (s, 1H), 8.01-7.99 (m, 1H), 7.71 (d, 1H), 7.68-7.62 (m, 2H), 7.58-7.55 (m, 1H), 7.53-7.42 (m, 4H), 6.88 (s, 1H).

Compound No. (b-59): $^1$ H-NMR (400MHz, $CDCl_3$ ): δ 8.87 (1H, s), 8.45 (1H, s), 8.44 (1H, s), 8.25 (1H, d), 8.05 (1H, s), 8.02 (1H, d), 7.62 (1H, d), 7.32 (1H, dd), 6.73 (1H, s), 4.04 (3H, s), 2.41 (3H, s).

Compound No. (b-67): $^1$ H-NMR (400MHz, $CDCl_3$): δ 8.89 (1H, s), 8.64 (1H, d), 8.46 (1H, s),8.45 (1H, s), 8.17 (1H, s), 8.05 (1H, d), 7.95 (1H, s), 7.61 (1H, d), 7.51 (1H, dd), 7.31 (1H, dd), 6.77 (1H, s), 5.46 (2H, s).

Compound No. (b-70): $^1$ H-NMR (400MHz, $CDCl_3$): δ 8.88 (1H, s), 8.61 (1H, d), 8.45 (1H, s), 8.12 (1H, s), 8.04 (1H, d), 7.97 (1H, s), 7.63 (1H, d), 7.49 (1H, dd), 7.31 (1H, dd), 6.77 (1H, s), 4.42-4.36 (2H, m), 3.72-3.67 (2H, m), 3.41 (3H, s).

(Biological test)

The following test examples show that the compound according to the present invention is useful as an active ingredient of an agricultural or horticultural fungicide.

(Preparation of test emulsion)

5 parts by mass of the compound according to the present invention, 93.5 parts by mass of dimethylformamide, and 1.5 parts by mass of polyoxyethylene alkylaryl ether were mixed and dissolved to prepare an emulsion (I) containing 5% by mass of an active ingredient.

The control value was calculated according to the following formula.

$$\text{Control value (\%)} = 100-\{\text{blotch area ratio in treated area/ blotch area ratio in untreated area}\} \times 100$$

(Test example 1) Tomato late blight control test

Water was added to the emulsion (I) such that the concentration of the heterocyclic compound became 125 ppm by mass, and then the heterocyclic compound was dissolved therein to obtain a pharmaceutical solution. Then, the pharmaceutical solution was sprayed on young tomato seedlings (variety "Rejina" at the $4^{th}$ to $5^{th}$ leaf stage) grown in nursery pots. After air-drying, the young tomato seedlings on which the pharmaceutical solution was sprayed were inoculated with zoosporangia of tomato late blight pathogen (*Phytophthora infestans)* in a suspension by spraying the suspension thereon (treated area). As a control, young tomato seedlings on which no pharmaceutical solution was sprayed were inoculated in the same way as mentioned above (untreated area). The resultant young tomato seedlings were left in a moist chamber at 20°C. Four days after inoculation, leaves of the young tomato seedlings were visually observed to measure the blotch area ratio to calculate the control value.

The compounds shown in Table 3 were subjected to the tomato late blight control test. The control value of all of the compounds was 75% or more.

Table 3

| | | | | | | | | |
|---|---|---|---|---|---|---|---|---|
| a-1 | a-35 | a-61 | a-88 | a-115 | a-145 | a-172 | b-20 | b-55 |
| a-2 | a-36 | a-62 | a-89 | a-116 | a-146 | a-173 | b-21 | b-56 |
| a-3 | a-37 | a-63 | a-90 | a-117 | a-147 | a-174 | b-22 | b-57 |
| a-4 | a-38 | a-64 | a-91 | a-120 | a-148 | a-175 | b-23 | b-58 |
| a-5 | a-39 | a-65 | a-93 | a-121 | a-149 | a-177 | b-24 | b-63 |
| a-8 | a-40 | a-66 | a-94 | a-122 | a-150 | a-178 | b-25 | b-66 |
| a-9 | a-43 | a-67 | a-95 | a-123 | a-151 | a-179 | b-26 | b-67 |
| a-10 | a-44 | a-68 | a-96 | a-124 | a-152 | a-180 | b-27 | b-68 |
| a-13 | a-45 | a-69 | a-97 | a-125 | a-153 | a-181 | b-29 | b-69 |
| a-14 | a-46 | a-70 | a-98 | a-127 | a-154 | a-182 | b-33 | b-70 |
| a-15 | a-47 | a-71 | a-99 | a-128 | a-155 | b-3 | b-36 | b-71 |

(continued)

| Table 3 | | | | | | | | |
|---|---|---|---|---|---|---|---|---|
| a-16 | a-48 | a-73 | a-100 | a-129 | a-157 | b-4 | b-37 | b-74 |
| a-17 | a-49 | a-74 | a-101 | a-130 | a-158 | b-5 | b-38 | b-76 |
| a-18 | a-50 | a-75 | a-102 | a-132 | a-159 | b-6 | b-39 | b-78 |
| a-19 | a-51 | a-76 | a-103 | a-133 | a-160 | b-7 | b-40 | b-79 |
| a-20 | a-52 | a-78 | a-104 | a-134 | a-161 | b-8 | b-42 | b-84 |
| a-21 | a-53 | a-79 | a-105 | a-135 | a-162 | b-9 | b-44 | b-85 |
| a-22 | a-54 | a-80 | a-107 | a-137 | a-163 | b-10 | b-45 | b-86 |
| a-23 | a-55 | a-81 | a-108 | a-138 | a-165 | b-12 | b-46 | b-87 |
| a-28 | a-56 | a-83 | a-110 | a-139 | a-166 | b-13 | b-47 | |
| a-31 | a-57 | a-84 | a-111 | a-141 | a-167 | b-14 | b-48 | |
| a-32 | a-58 | a-85 | a-112 | a-142 | a-168 | b-15 | b-49 | |
| a-33 | a-59 | a-86 | a-113 | a-143 | a-170 | b-17 | b-50 | |
| a-34 | a-60 | a-87 | a-114 | a-144 | a-171 | b-18 | b-51 | |

(Test example 2) Grape downy mildew control test

Water was added to the emulsion (I) such that the concentration of the heterocyclic compound became 100 ppm by mass, and then the heterocyclic compound was dissolved therein to obtain a pharmaceutical solution. Then, the pharmaceutical solution was sprayed on a leaf disc of grape (variety "Chardonnay") placed on agar in a 6-well plate. After air-drying, the leaf disc on which the pharmaceutical solution was sprayed was inoculated with zoosporangia of grape downy mildew pathogen (*Plasmopara viticola)* in a suspension by spraying the suspension thereon (treated area). As a control, a leaf disc on which no pharmaceutical solution was sprayed was inoculated in the same way as mentioned above (untreated area). The resultant leaf discs were left in a moist chamber at 20°C. Seven days after inoculation, the grape leaf discs were visually observed to measure the blotch area ratio to calculate the control value.

The compounds shown in Table 4 were subjected to the grape downy mildew control test. The control value of all of the compounds was 75% or more.

| Table 4 | | | | | | | | |
|---|---|---|---|---|---|---|---|---|
| a-4 | a-48 | a-75 | a-103 | a-133 | a-160 | b-14 | b-44 | b-74 |
| a-5 | a-49 | a-76 | a-105 | a-134 | a-161 | b-15 | b-45 | b-76 |
| a-8 | a-50 | a-78 | a-107 | a-135 | a-162 | b-16 | b-46 | b-77 |
| a-9 | a-51 | a-80 | a-108 | a-136 | a-163 | b-17 | b-47 | b-81 |
| a-15 | a-52 | a-81 | a-110 | a-137 | a-165 | b-18 | b-48 | b-83 |
| a-16 | a-53 | a-84 | a-111 | a-138 | a-166 | b-19 | b-49 | b-84 |
| a-17 | a-54 | a-85 | a-112 | a-139 | a-168 | b-20 | b-50 | b-85 |
| a-19 | a-55 | a-86 | a-114 | a-140 | a-169 | b-21 | b-51 | b-86 |
| a-20 | a-56 | a-87 | a-115 | a-141 | a-170 | b-22 | b-52 | b-87 |
| a-21 | a-57 | a-88 | a-116 | a-142 | a-171 | b-23 | b-53 | |
| a-22 | a-58 | a-89 | a-117 | a-143 | a-172 | b-24 | b-54 | |
| a-23 | a-60 | a-90 | a-118 | a-144 | a-173 | b-26 | b-55 | |
| a-27 | a-61 | a-91 | a-120 | a-145 | a-175 | b-27 | b-56 | |
| a-28 | a-62 | a-92 | a-121 | a-146 | a-177 | b-28 | b-57 | |
| a-31 | a-63 | a-93 | a-122 | a-147 | a-178 | b-29 | b-58 | |
| a-32 | a-64 | a-94 | a-123 | a-148 | a-179 | b-33 | b-59 | |
| a-33 | a-65 | a-95 | a-124 | a-150 | a-180 | b-36 | b-63 | |
| a-35 | a-66 | a-96 | a-125 | a-151 | a-181 | b-37 | b-66 | |
| a-38 | a-68 | a-97 | a-127 | a-152 | a-182 | b-38 | b-67 | |
| a-41 | a-69 | a-98 | a-128 | a-154 | a-183 | b-39 | b-68 | |
| a-44 | a-70 | a-99 | a-129 | a-155 | b-9 | b-40 | b-69 | |
| a-45 | a-71 | a-100 | a-130 | a-157 | b-10 | b-41 | b-70 | |

(continued)

| Table 4 | | | | | | | | |
|---|---|---|---|---|---|---|---|---|
| a-46 | a-72 | a-101 | a-131 | a-158 | b-12 | b-42 | b-71 | |
| a-47 | a-74 | a-102 | a-132 | a-159 | b-13 | b-43 | b-72 | |

Since the compounds randomly selected from the compounds according to the present invention exhibited the above-shown effects, it is understood that the compound according to the present invention, involving aspects of compounds that are not mentioned above, is a compound that exhibits fungicidal activities without causing harmful effects on plants and that provides less toxicity in humans, animals, or fish, and fewer detrimental effects on the environment.

INDUSTRIAL APPLICABILITY

The heterocyclic compound and the salts thereof, according to the present invention, has an excellent fungicidal activity, exhibits effect surely, is excellent in safety, and can be synthesized industrially advantageously. The agricultural or horticultural fungicide according to the present invention exhibits an excellent control effect without causing harmful effects on plants and provides less toxicity in humans, animals, or fish, and fewer detrimental effects on the environment.

## Claims

1. A compound of formula (I) or a salt thereof:

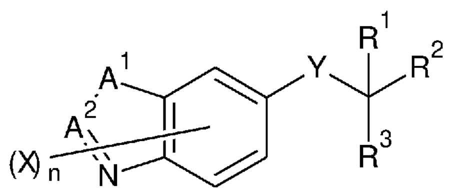

(I)

wherein, in the formula (I),

$R^1$ represents a hydrogen atom, a substituted or unsubstituted C1-6 alkyl group, or a halogeno group,
$R^2$ represents a substituted or unsubstituted 5- or 6-membered heteroaryl group,
$R^3$ represents a substituted or unsubstituted C1-6 alkyl group, a substituted or unsubstituted C1-6 alkoxycarbonyl group, a substituted or unsubstituted C1-6 alkylthio group, a substituted or unsubstituted C6-10 aryl group, a substituted or unsubstituted 5- or 6-membered heteroaryl group, a group of formula: "$-C(R^i)=NOR^j$", or a group of formula: "$-C(=O)NHR^s$",
$R^i$ represents a hydrogen atom or a C1-6 alkyl group,
$R^j$ represents a substituted or unsubstituted C1-6 alkyl group,
$R^s$ represents a substituted or unsubstituted C1-6 alkyl group,
Y represents an oxygen atom or NH,
$A^1$ represents a sulfur atom, an oxygen atom, or $NR^4$,
$R^4$ represents a C1-6 alkyl group,
$A^2$ represents a carbon atom or a nitrogen atom,
X represents a substituent,
n represents a chemically acceptable number of X, and is an integer of 0 to 3, and
when n is 2 or more, X is identical to or different from each other.

2. The compound or the salt thereof according to claim 1, wherein, in the formula (I), X represents a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a substituted or unsubstituted C1-6 alkylthio group, a substituted or unsubstituted C3-8 cycloalkyl group, a substituted or unsubstituted C6-10 aryl group, a halogeno group, a cyano group, a group of formula: "$-NR^aR^b$" (wherein $R^a$ and $R^b$ each independently represents a hydrogen atom, a C1-6 alkyl group, or a C1-6 alkoxycarbonyl group), or a group of formula: "$-C(R^e)=NOR^f$" (wherein $R^e$ and $R^f$ each independently represents

a hydrogen atom or a C1-6 alkyl group).

**3.** The compound or the salt thereof according to claim 1, wherein the formula (I) is formula (II):

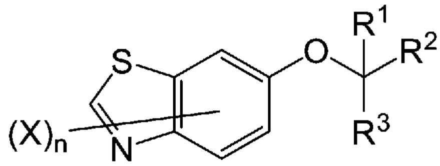

(II)

wherein, in the formula (II), $R^1$, $R^2$, $R^3$, X and n represent the same as defined in the formula (I).

**4.** The compound or the salt thereof according to claim 1, wherein the formula (I) is formula (III):

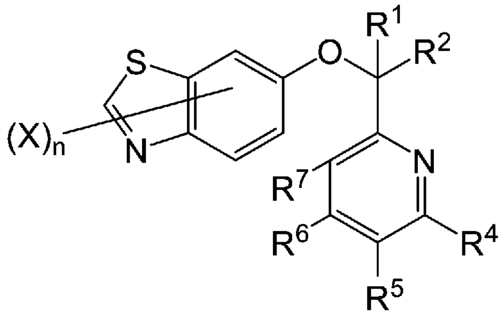

(III)

wherein, in the formula (III), $R^1$, $R^2$, X and n represent the same as defined in the formula (I), and

$R^4$, $R^5$, $R^6$ and $R^7$ each independently represents a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C6-10 aryl group, a substituted or unsubstituted 5- or 6-membered heterocyclyl group, a halogeno group, a cyano group, a nitro group, a group of formula "-C($R^g$)=NOR$^h$" (wherein $R^g$ represents a hydrogen atom, a C1-6 alkyl group or an amino group, and $R^h$ represents a hydrogen atom, a substituted or unsubstituted C1-6 alkyl group, or a C2-6 alkynyl group), a group of formula: "-O-N=CR$^c$R$^d$" (wherein $R^c$ and $R^d$ each independently represents a hydrogen atom or a C1-6 alkyl group), or a group of formula: "-C(=O)NR$^p$R$^q$" (wherein $R^p$ and $R^q$ each independently represents a hydrogen atom or a C1-6 alkyl group).

**5.** An agricultural or horticultural fungicide comprising at least one selected from the group consisting of compounds and salts thereof of claims 1 to 4, as an active ingredient thereof.

## INTERNATIONAL SEARCH REPORT

International application No. PCT/JP2020/010905

A. CLASSIFICATION OF SUBJECT MATTER
Int.Cl. C07D413/12(2006.01)i, C07D413/14(2006.01)i, C07D417/12(2006.01)i, C07D417/14(2006.01)i, A01N47/18(2006.01)i, A01P3/00(2006.01)i, A01N43/78(2006.01)i, A01N43/824(2006.01)i
FI: C07D413/12CSP, A01P3/00, A01N43/824A, A01N47/18101Z, A01N43/78101, C07D413/14, C07D417/14, C07D417/12
According to International Patent Classification (IPC) or to both national classification and IPC

B. FIELDS SEARCHED
Minimum documentation searched (classification system followed by classification symbols)
Int.Cl. C07D413/12, C07D413/14, C07D417/12, C07D417/14, A01N47/18, A01P3/00, A01N43/78, A01N43/824

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2016/171755 A1 (FORMA THERAPEUTICS, INC.) 27.10.2016 (2016-10-27), claim 18, (6-chloro-3-[(1S)-1-[(2-oxo-2, 3-dihydro-1, 3-benzoxazol-6-yl)amino]ethyl]-1, 2-dihydroquinolin-2-one) | 1, 2<br>3-5 |
| X<br>A | WO 2016/016421 A1 (INSTITUT PASTEUR KOREA) 04.02.2016 (2016-02-04), no. 150, compound | 1, 2<br>3-5 |
| X<br>A | US 2012/0135977 A1 (BESHORE, D. C.) 31.05.2012 (2012-05-31), table 3, example 3-18, compound, table 4, examples 4-3, 4-4, 4-11, 4-37, 4-52, compounds, table 10, example 10-155, compound | 1, 2<br>3-5 |
| X<br>A | WO 2008/055950 A1 (PROBIODRUG AG) 15.05.2008 (2008-05-15), compounds 76-212 | 1, 2<br>3-5 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07.05.2020 | 26.05.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/010905

C (Continuation). DOCUMENTS CONSIDERED TO BE RELEVANT

| Category* | Citation of document, with indication, where appropriate, of the relevant passages | Relevant to claim No. |
|---|---|---|
| A | MOORMANN, A. E., Potential antisecretory antidiarrheals. 2. Alpha2-adrenergic 2-[(aryloxy)alkyl]imidazolines, Journal of Medicinal Chemistry, 1990, 33, 614-626, compound 37 | 1-5 |
| A | SIDHU, P. S., Development of novel Vitamin D receptor-coactivator inhibitors, ACS Medicinal Chemistry Letters, 2014, 5, 199-204, compound 25 | 1-5 |

Form PCT/ISA/210 (second sheet) (January 2015)

**INTERNATIONAL SEARCH REPORT**
Information on patent family members

International application No.
PCT/JP2020/010905

| | | |
|---|---|---|
| WO 2016/171755 A1 | 27.10.2016 | US 2016/0311774 A1<br>US 2018/0141910 A1<br>US 2019/0202790 A1 |
| WO 2016/016421 A1 | 04.02.2016 | CA 2955565 A<br>AU 2015295248 A<br>KR 10-2017-0045234 A<br>EP 3177597 A1<br>US 2017/0166563 A1<br>CN 106879256 A<br>JP 2017-522339 A<br>BR 112017001918 A<br>RU 2017103148 A |
| US 2012/0135977 A1 | 31.05.2012 | (Family: none) |
| WO 2008/055950 A1 | 15.05.2008 | US 2008/0207715 A1<br>EP 2086960 A1<br>JP 2010-509286 A<br>JP 5599614 B |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019048196 A
- WO 2016108282 A
- WO 2004108964 A


### Non-patent literature cited in the description


- *Bioorganic & Medicinal Chemistry,* vol. 19 (1), 359-373
- *CHEMICAL ABSTRACTS,* 943137-49-3